**Europäisches Patentamt**

**European Patent Office**

**Office européen des brevets**

(19)

(11) Publication number: **0 122 961**
**A2**

(12) **EUROPEAN PATENT APPLICATION**

(21) Application number: **83110137.3**

(22) Date of filing: **11.10.83**

(51) Int. Cl.³: **A 61 B 3/12**

(30) Priority: **12.10.82 US 433813**

(43) Date of publication of application:
**31.10.84 Bulletin 84/44**

(84) Designated Contracting States:
**DE FR GB**

(71) Applicant: **Optische Werke G. Rodenstock**
**Isartalstrasse 43**
**D-8000 München 5(DE)**

(72) Inventor: **Cornsweet, Tom N.**
**23652 Calle Hogar**
**Mission Viejo, California 92691(US)**

(72) Inventor: **Hersh, Samuel**
**28521 Casenal**
**Mission Viejo, California 92692(US)**

(74) Representative: **Schiller, Walter, Dr.**
**Willibaldstrasse 36**
**D-8000 München 21(DE)**

(54) Ocular fundus analyzer.

(57) This invention relates to apparatus for examining the ocular fundus of the eye and, more particulary, to an optoelectronic ocular fundus analyzer.

The apparatus for examining the ocular fundus of the eye, according to the invention includes means for illuminating the ocular fundus, means, responsive to the light reflected by the ocular fundus, for forming an image of the ocular fundus at a detecting plane, means for sensing and scanning the image of the ocular fundus at the detecting plane, and data processing means, coupled to said sensing and scanning means, for storing digitally and displaying data about the ocular fundus.

FIG _ 1

EP 0 122 961 A2

## Description

## Ocular Fundus Analyzer

### Technical Field

This invention relates to apparatus for examining the ocular fundus of the eye and, more particularly, to an optoelectronic ocular fundus analyzer.

### Background Art

Many disorders of the eye, a common one of which is glaucoma, cause changes in the color and/or shape of regions of the surface lining the inside of the eyeball, which is the ocular fundus. Other disorders of the entire body, such as diabetes and atherosclerosis, also produce such changes in the ocular fundus. These changes usually occur at the early stages of the disorders, and often the disorders are first detected during an examination of the ocular fundus known as a fundoscopic examination.

The detection of disorders by the fundoscopic examination may be divided into three aspects. These are (1) detection of abnormalities or changes in the color of regions of the ocular fundus, (2) detection of abnormalities or changes in the two dimensional shape of regions of the ocular fundus, e.g., changes in the tortuosity of fundus blood vessels that occur in atherosclerosis or changes in the size of a discolored region, and (3) detection of abnormalities or changes in the third dimension of regions of the ocular fundus, i.e., along the optic axis or visual axis of the eye.

Abnormal color of a region of the ocular fundus is a consequence of physical abnormalities in the fundus that cause the region to reflect light abnormally. For example, the leakage of certain chemicals from the blood into the tissue of the fundus can change the chemical composition of the region. Therefore, the way in which the region interacts with light may be changed so that, for example, a greater fraction of long wavelength light and a smaller fraction of short wavelength light are reflected. If so, the region will appear abnormally more red.

As another example, there are regions of the ocular fundus that should normally appear reddish. These regions constitute a white surface, i.e., a surface that reflects equally most wavelengths of the visible spectrum, overlaid by a fine mesh of tiny blood vessels which circulate blood, i.e., a substance that reflects long wavelength light much more strongly than short wavelength light. Abnormal color of these regions may be a consequence of impaired blood circulation. If so, if illuminated with white light, these regions will appear abnormally more white and exhibit pallor.

Various ophthalmological instruments have been developed to examine the ocular fundus to detect abnormal color, as well as changes in the two-dimensional and three-dimensional shape of the fundus. One instrument, called an ophthalmoscope, illuminates the inside of a patient's eye and provides an optical path for the physician to see the fundus. The physician relies on his own or subjective color vision to detect subtle abnormalities in the color of the fundus. This is neither highly accurate nor objective since the physician cannot visually determine the amount or intensity of light being reflected at each wavelength, which is data that provide more subtle color information. Furthermore, because many disorders are detected or their treatments monitored by evaluating changes in the fundus over time, the physician using the ophthalmoscope may have to depend either on her memory or her sketches of the appearance of the fundus. The same problems occur when evaluating changes in the two-dimensional and three-dimensional shapes of the fundus.

Another instrument is a special camera called a fundus camera that is used to photograph the ocular fundus. A series of black and white photographs can be taken with the fundus camera using light at various wavelengths. In essence, the series of photographs taken at different wavelengths represent a set of "reflectance spectra", that is, they represent the amount of light reflected from each point in the picture at each wavelength. Then, the photographs are compared visually or with a densitometer. While the reflectance spectrum is more objective data than can be obtained using the ophthalmoscope, this photographic procedure is awkward and suffers seriously from the fact that slight variations in the conditions under which the black and white film is developed can cause significant changes in the resulting photographic densities.

Also, the fundus camera can be used to take photographs over time. While these photographs provide better and more objective data than the above-mentioned sketches for detecting changes in color, as well as shape, the variability of the conditions of illumination, and especially of the chemistry of the photographic film processing, makes accurate measures of change difficult.

Furthermore, and with respect to abnormalities in the three-dimensional shape of the ocular fundus, during the course of, for example, undetected or uncontrolled glaucoma, a region of the ocular fundus called the optic disk develops cupping or excavation. That is, the surface of the optic disk recedes from the front of the eye. On the other hand, brain tumors, for example, can cause the optic disk to bulge toward the front of the eye, as can fundus tumors.

The three-dimensional shape and changes in this shape of the fundus are very difficult to evaluate with the two-dimensional photographs taken with the standard fundus camera. A modified fundus camera has been used that produces stereo pairs of fundus photographs. When viewed with a stereo viewer, these stereo pictures provide better data on which to evaluate the three-dimensional shape of the optic disk. However, the evaluation is essentially visual and subjective and, therefore, not highly accurate.

Summary of the Invention

It is an object of the present invention to provide a novel apparatus for examining the ocular fundus of the eye.

It is another object of the present invention to provide an ocular fundus examination apparatus for objectively determining eye disorders.

Yet another object of the present invention is to provide objective, highly accurate and quantitative data having information about the ocular fundus.

Still another object of the present invention is to utilize state-of-the-art optoelectronics for the novel ocular fundus examining apparatus.

These and other objects of the present invention are obtained through the use of apparatus for examining the ocular fundus of the eye, including means for illuminating the ocular fundus, means, responsive to the light reflected by the ocular fundus, for forming an image of the ocular fundus at a detecting plane, means for sensing and scanning the image of the ocular fundus at the detecting plane, and data processing means, coupled to said sensing and scanning means, for storing digitally and displaying data about the ocular fundus.

The light that illuminates and is reflected by the ocular fundus forms an image of the fundus at the detecting plane. The sensing and scanning means can be, for example, a TV camera receiving the image as a picture which is then scanned. The output data of the camera are then digitized, stored and processed by the data processing means. The optical and digital data that are obtained constitute objective, highly accurate and quantitative information about the ocular fundus, including information about the color, two-dimensional shape and three-dimensional shape of the fundus, as well as changes in these parameters.

Brief Description of the Drawings

Fig. 1 is a schematic side view illustration of an optical system of the present invention.

Fig. 2 is a pictorial view of an optical component of the optical system of Fig. 1.

Fig. 3A and Fig. 3B are stereo images of a region of the ocular fundus providing data about the three-dimensional shape of the fundus.

Fig. 3C is a depth profile of a region of the ocular fundus obtained from the optical data of Fig. 3B.

Fig. 4 shows schematically an optical system used in conjunction with the optical system of Fig. 1.

Fig. 5 is a block diagram of the hardware of an electronic data processor of the present invention.

Fig. 6 illustrates an image of the pupil of the eye for explaining an automatic alignment feature of the present invention.

Fig. 7 is a view used to explain a specific manner of obtaining the depth profile of Fig. 3C.

Detailed Description of the Invention

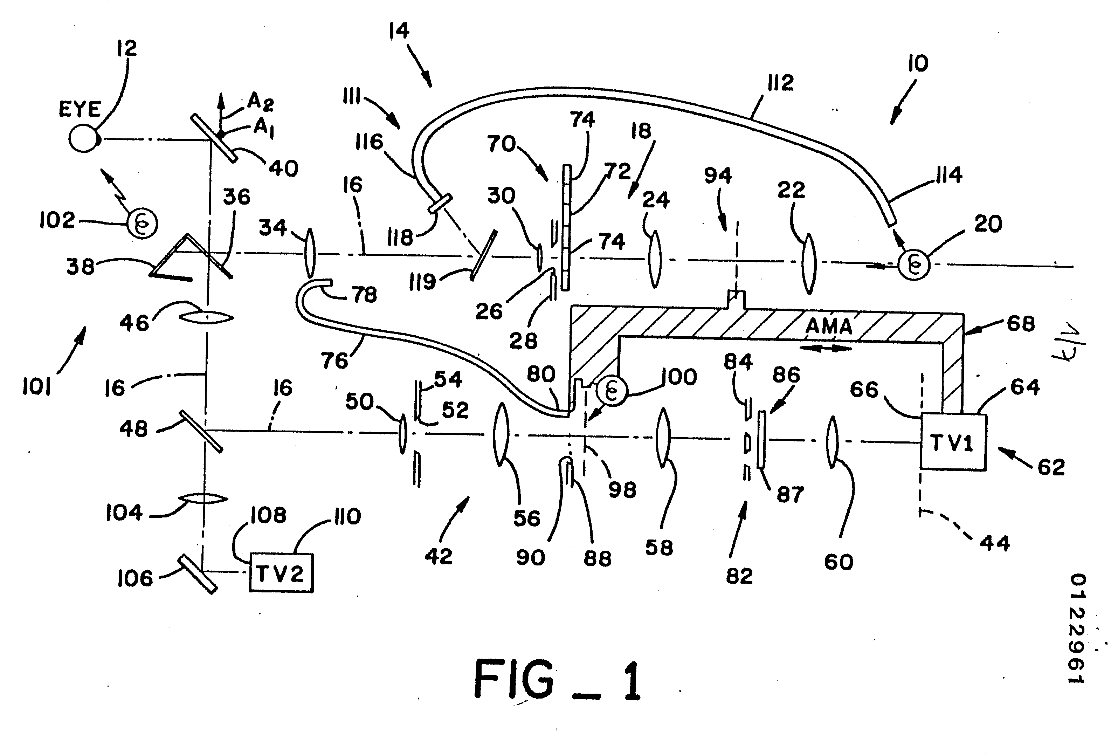

Fig. 1 illustrates a portion of an apparatus or instrument 10 used to examine the ocular fundus of an eye 12. In particular, the portion shown in Fig. 1 is an optical system 14 having an optical axis 16 and used to acquire optical data for examining the fundus. While the optical components are shown in full lines in Fig. 1, it will become appreciated that several of these are used or introduced into the optical system 14 to carry out particular modes of operation.

The optical system 14 includes a subsystem 18 for illuminating the ocular fundus of the eye 12. Subsystem 18 has a light source 20 whose light passes through a lens 22 and a lens 24. Lenses 22 and 24 form an image of the light source 20 at a circular aperture 26 provided by a mask 28.

Light from aperture 26 then propagates through a lens 30 and a lens 34 to a beam splitter 36. Half of the light then passes through beam splitter 36 and is absorbed by a light trap 38 and the other half of the light is reflected by the beam splitter 36 onto a movable mirror 40 which then reflects the light into the eye 12. Lenses 30 and 34 form an image of aperture 26 in the plane of the pupil of the eye 12, and the light then passes through the pupil and falls on the ocular fundus, illuminating it. Mirror 40 is rotatable about a horizontal axis A1 and a vertical axis A2 for optical alignment reasons to be described.

Optical system 14 has another subsystem 42 for forming an image of the ocular fundus at a detecting plane 44. Subsystem 42 includes the mirror 40 which receives some of the light reflected by the ocular fundus outside the eye 12 and reflects the light towards the beam splitter 36. Half the light is then reflected by the beam splitter 36 towards the source 20 and is lost, but the other half passes through the beam splitter 36 and a lens 46 towards a cold mirror 48. The light is then reflected by cold mirror 48 through a lens 50 towards a circular aperture 52 provided by a mask 54. Lens 46 and lens 50 form an image of the pupil of the eye 12 in the plane of aperture 52. The diameter of aperture 52 is smaller than the image of the typical pupil to prevent any light that might be reflected from the iris of the eye 12 from propagating along the remainder of subsystem 42.

The light passing through aperture 52 is then propagated by a lens 56, a lens 58 and a lens 60 onto the detecting plane 44. Lenses 46, 50, 56, 58 and 60, in combination with the optics of the eye 12, form an image of the ocular fundus at the detecting plane 44.

Also shown in Fig. 1 is an apparatus 62 for sensing and scanning the image of the fundus. For example, apparatus 62 can be a TV camera 64 whose screen 66 is at the detecting plane 44 to receive the image. Thus, the image or a picture of the fundus is on the screen 66 which senses the light falling on it. TV camera 64 is supported on an axial motion assembly 68 in such a way that screen 66 can be moved toward or away from lens 60 to bring the image of the fundus in sharp focus should the eye 12 exhibit a refractive error. For example, if the patient is nearsighted, the camera 64 can be moved toward the lens 60 to focus the image on screen 66.

In summary, thus far, light from source 20 illuminates the fundus, and light reflected from the fundus forms an image or picture of the fundus on screen 66. As will be described below, the picture can then be scanned, digitized and stored for processing. Any number of scans or pictures can be taken, either at one examination or over a period of time, the latter to aid in detecting changes in the fundus.

To obtain objective optical data about the color of the fundus, optical system 14 has a subsystem 70 for filtering the light from source 20. Subsystem 70 can be a rotatable wheel 72 having a plurality of different light filters 74, any one of which can be rotated into the path of the light along the optical axis 16. As will be further described, two or more pictures of the fundus can be taken under different illuminating wavelengths, i.e., with two or more different filters 74, to provide data from which a reflectance spectrum of the fundus can be determined. The wheel 72 can support an aperture in lieu of one of the filters 74 to transmit unfiltered light should color information not be required.

To provide highly accurate reflectance spectrum data about the fundus, differences in the sensitivity of the TV camera 64 to different wavelengths preferably should be taken into account. Therefore, subsystem 70 can include a fiber optic bundle 76 having one end 78 located near lens 34 to intercept a small amount of light that is filtered by filters 74 and that is illuminating the fundus. The other end 80 of fiber optic bundle 76 is positioned as shown so that light entering end 78 and exiting end 80 forms a small bright spot on a corner of screen 66 after being propagated by lens 58 and lens 60. Consequently, the level or intensity of the video signal corresponding to this bright spot on screen 66, for each fundus picture taken with a different filter 74, provides data about the sensitivity of camera 64 to different wavelengths.

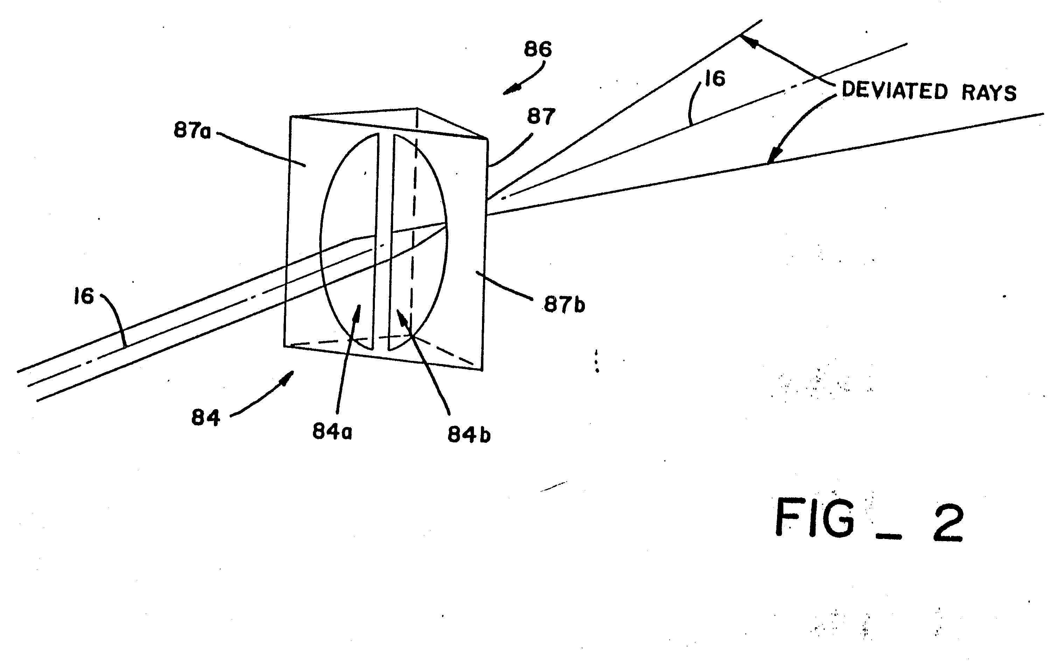

The optical system 14 can include another subsystem 82 to obtain data at the detecting plane 44 having three-dimensional or depth information about the ocular fundus. This subsystem 82 includes an aperture mask 84 and a prism 86 shown in more detail in Fig. 2 to which reference is now made. Mask 84 has two semicircular apertures 84a, 84b positioned side-by-side, as shown. Prism 86 is, for example, a bi-prism 87, constituting two wedge prisms 87a, 87b connected base-to-base. Light in one path passing through aperture 84a is deviated horizontally by wedge prism 87a in one direction from the optical axis 16 and light in the other path passing through the other aperture 84b is deviated horizontally by the other prism 87b in the other direction from the optical axis 16, i.e., the light is spatially separated by prism 86.

Lenses 46, 50, 56 and 58 combine to form an image of the patient's pupil in the plane 84. Therefore, the light in one of the paths forms an image of the region of the fundus under examination as if it were viewed through one side of the pupil and the light in the other path forms an image of the same region of the fundus as if it were viewed through the other side of the pupil. These two images form a stereo pair or picture of this region of the fundus on screen 66 as shown, for example, and as will be further described, in Fig. 3A-1 and Fig. 3A-2. Also shown in Fig. 1 as part of subsystem 82 is a mask 88 which has a rectangular aperture 90 whose horizontal width is designed to block those portions of the stereo pair that would overlap on screen 66.

More particularly, as previously mentioned, lenses 46 and 50 form an image of the pupil at aperture 52. Lens 56 then collimates the light from aperture 52 and lens 58 reimages the pupil in the plane of apertures 84a, 84b. Lenses 46, 50 and 56, in combination with the optics of the eye 12, form an image of the fundus in the plane of the rectangular aperture 90. If bi-prism 87 were removed from the axis 16, lenses 58 and 60 would simply reimage the region of the fundus on screen 66. However, when bi-prism 87 is in place, as illustrated, two laterally displaced images of this region of the fundus are formed on screen 66, as shown in Fig. 3A-1 and Fig. 3A-2. Because all the light in one of the images passes through aperture 84a and all the light in the other of the images passes through aperture 84b, and are deviated by bi-prism 87, these two images form a stereo pair of the same region on screen 66.

With reference to Fig. 3A-1 and Fig. 3A-2, the stereo pair of fundus images that are formed on screen 66 have the same blood vessels 92, e.g., a blood vessel 92A-1 in the left stereo picture and the identical blood vessel 92A-2 in the right stereo picture. As will be described below, three-dimensional or depth data can be obtained from the distance X along a horizontal scan line 1 between pairs of corresponding areas or features in the stereo pair, e.g., the distances between corresponding areas on the blood vessels 92A-1, 92A-2.

More generally, three-dimensional optical data are obtained whenever there are features of the fundus imaged on screen 66 that have a directional component perpendicular to a horizontal line joining the centers of the two apertures 84a, 84b through which the stereo pair were taken. However, if the region of the fundus being examined or illuminated were featureless, (e.g., no blood vessels), as some regions are, no features would be imaged on the screen 66 and, therefore, no three-dimensional data would be available. Some regions of the ocular fundus may even have features but would appear featureless to TV camera 64 if illuminated with certain wavelengths. Therefore, to obtain three-dimensional data of these featureless or apparently featureless regions, subsystem 82 also has the following.

A mask 94 illustrated in Fig. 1 and having a narrow vertical slit (not shown) can be selectively interposed between lenses 22 and 24. The combination of lenses 24, 30 and 34, together with the optics of the eye 12, form an image of the slit on the ocular fundus. Then, when the stereo pair of images of a region is formed on screen 66, the images are of the fundus region having a pair of vertical lines 96A-1, 96A-2 shown in Figs. 3A-1, 3A-2. As will be further described, the distance X' along horizontal scan line 1 between line 96A-1 and line 96A-2 provides the three-dimensional data used to examine the region of the fundus. Mask 94, as shown in Fig. 1, is mounted on the axial motion assembly 68 so that the mask 94 can be moved towards and away from lens 24 to bring the vertical slit in sharp focus on the fundus. Of course, as will become apparent, more than one such slit of mask 94 can be used to acquire more three-dimensional data.

The acquisition of the most accurate output data from TV camera 64 is dependent on the amount of any geometric distortion introduced by the camera 64 in scanning the image on screen 66. The TV camera 64 may be a silicon intensified target vidicon camera, in which the image is scanned by a deflected electron beam. The electro-optics of the camera 64 permit small distortions of the scan and, therefore, of the apparent spatial positions of the analog signals proportional to the intensity of the light of the fundus features and outputted from the camera 64. These distortions may change with, for example, temperature or component age. Because the extraction of depth or three-dimensional information from the stereo pair of images requires precise spatial localization of the images on screen 66 for high accuracy purposes, such distortion would compromise this accuracy if it were not corrected.

Consequently, the subsystem 82 can also include optical components for obtaining optical data to calibrate the distortion in the camera 64. Specifically, a mask 98 having a narrow vertical slit (not shown), identical to mask 94, can be moved into the position shown near the plane of aperture 90. A light source 100 then can be actuated to illuminate the slits of mask 98. Because aperture 90 is in the plane where the image of the fundus is formed if illuminated with source 20, lens 58, bi-prism 87 and lens 60 form two adjacent images of the slit of mask 98 on screen 66. These slit images are identical with the slit images 96A-1, 96A-2 that would appear on screen 66 if mask 98 were removed from the optical path and a perfectly flat region of the fundus were illuminated through mask 94.

Then, if there were no distortion in the TV camera 64, the calibration information read out of the camera 64 would comprise data identifying two adjacent slits of perfectly straight and parallel vertical lines. To provide the calibration data, mask 98 is moved into the position shown and source 100 is energized to illuminate the mask 98. The resulting image or calibration picture on screen 66 is then scanned and data are read out by camera 64. As will be further described, irregularities in this calibration picture are measured and corrections are calculated that, if applied to the calibration picture, would yield perfectly straight, vertical slit images. These same corrections can then be applied to an actual stereo pair of images of the type shown in Fig. 3A-1, 3A-2 to correct for distortion. If more than one slit is in mask 94 then the same number of slits is used in mask 98.

An alternative to the above-described calibration technique is to use a distortion-free TV camera for camera 64, such as a microchannel plate intensifier coupled to a solid-state array sensor. However, presently this device is new and expensive.

Optical system 14 should be accurately aligned with respect to the eye 12 so that the light properly enters the pupil and falls on the fundus. Therefore, optical system 14 can include optical components to provide manual coarse alignment and automatic fine alignment of the eye 12 relative to the optical axis 16.

To achieve manual coarse alignment, optical system 14 can include a subsystem 101 that has a near infrared light source 102 which casts diffuse illumination onto the area where the eye 12 is expected to be. Light from the source 102 that is reflected by the front of the eye 12 is reflected by movable mirror 40 through beam splitter 36, lens 46, and cold mirror 48. A lens 104 then propagates the near infrared light onto a mirror 106 which reflects the light onto a screen 108 of a TV camera 110 which is sensitive to the near infrared light. Lenses 46 and 104 combine to form an image on screen 108 of the facial area about the eye 12, e.g., an area of about 25 mm. in diameter including the pupil. As will be further described, the entire instrument 10, including optical system 14, can be driven toward and away from the eye 12 to focus the pupil image on screen 108 and can be driven sideways horizontally (in and out of the plane of the Fig. 1) with respect to the eye 12 until the pupil image is roughly horizontally centered on the screen 108. Then, the mirror 40 can be rotated around its horizontal axis A1, which results in the image of the pupil on screen 108 moving up and down, until the image is roughly vertically centered on screen 108.

To achieve automatic fine alignment, optical system 14 includes a subsystem 111 that has a fiber optic bundle 112 having a light input end 114 receiving light from source 20 and a light output end 116 having an infrared filter 118. The infrared light outputted through filter 118 is reflected by a hot mirror 119 through lens 34 and is then reflected by beam splitter 36 and rotatable mirror 40 to the eye 12, where it passes through the pupil and illuminates the fundus. The output end 116 of fiber optic bundle 112 is at a distance optically from lens 34 such that lens 34 forms an image of the end 116 in the plane of the pupil. This image is smaller than the usual pupil, e.g., it is about 1.5 mm. in diameter, and when alignment is correct, is centered on the pupil.

Thus, the infrared light from filter 118 at output end 116 passes into the eye 12 and illuminates the fundus. Then, the infrared light is reflected diffusely by the fundus and emerges from the pupil, in effect backlighting the latter. This light then is reflected by mirror 40 through beam splitter 36, lens 46, cold mirror 48, lens 104 and mirror 106 onto screen 108. The image now falling on screen 108 is of a bright pupil on a dark background, and this image is used for automatic fine alignment purposes. As will be further described, mirror 40 will be automatically rotated about its horizontal axis A1 and vertical axis A2 until this image is precisely centered on screen 108. When this occurs, the instrument 10 and eye 12 are accurately aligned.

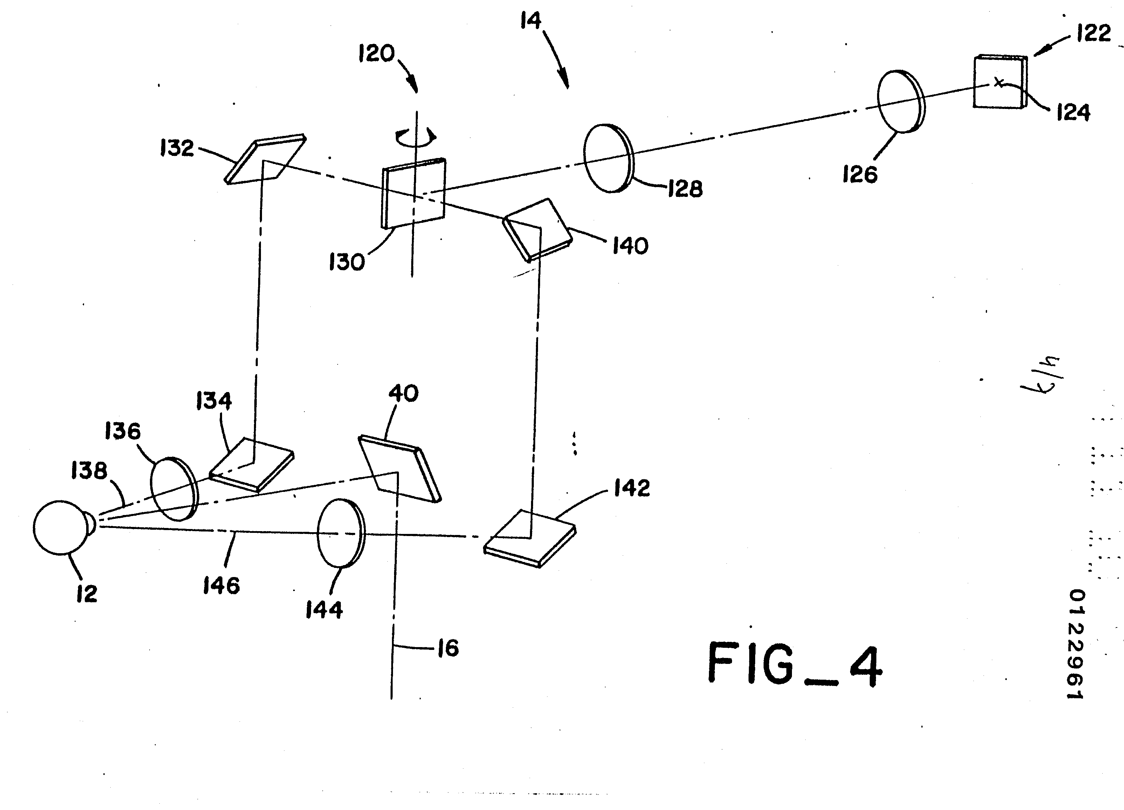

Another feature of the optical system 14 is an optical subsystem 120, shown in Fig. 4, which is used to obtain images or pictures of different regions of the fundus, such as the optic disk, which is a region about 5 mm. toward the temple from the fovea. To obtain these images, the visual axis or optic axis of the eye 12 should be moved with respect to the optical axis 16. This is accomplished with the optical subsystem 120 by maintaining the optical axis 16 fixed and controlling the orientation of the visual axis or optic axis of the eye 12.

More particularly, subsystem 120 includes the rotatable mirror 40 through which the optical axis 16 passes exactly as shown in Fig. 1 and indicated in Fig. 4. A patient's viewing screen 122, which may be a display device such as a TV screen, displays a movable target 124, such as an "X". Light from the X-shaped target 124 passes through a lens 126 and a lens 128. A rotatable mirror 130, which can rotate about its vertical axis as shown by the double-headed arrow, reflects the light from lens 128 onto a mirror 132 which then reflects the light onto a mirror 134. The reflected light from mirror 134 then passes through a lens 136 into the eye 12. Lenses 126, 128 and 136, together with the optics of the eye 12, combine to form an image of the target 124 on the fundus along an axis 138.

Assume that the target 124 is positioned, for example, on screen 124 corresponding to an angle of about 17 degrees relative to optical axis 16. Therefore, the axis 138 is at an angle of about 17 degrees from the optical axis 16, so that when the patient moves eye 12 to look at the target 124, the region of the fundus illuminated by subsystem 18 in Fig. 1 and imaged by subsystem 42 in Fig. 1 onto screen 66 is about 17 degrees toward the temple, which is the optic disk. The movements of the target 124 across screen 122 make for fine adjustment in viewing the optic disk. Lens 128 can be manually controlled to be moved or positioned towards or away from lens 126 to correct for a patient's refractive error. For example, if the patient is nearsighted, lens 128 can be moved away from lens 126 to bring target 124 into focus for the patient. The focal lengths and positions of lenses 126, 128 and 136 are chosen so that the magnification of the image of the target 124 on the fundus is the same regardless of the position of lens 128.

The preceding discussion of subsystem 120 actually referred to the imaging of the optic disk of the right eye 12 on screen 66. To image the optic disk of the left eye 12, the entire instrument 10 can be moved horizontally to align the optical axis 16 with the pupil of the left eye 12. Then, mirror 130 is rotated 90 degrees about its vertical axis. Now, light from target 124 passes through lens 126 and lens 128, and is reflected by mirror 130 onto a mirror 140. From mirror 140, the light is reflected from a mirror 142 through a lens 144 into the eye 12. The combination of lenses 126, 128 and 144, together with the optics of the eye 12, forms an image of the target 124 on the fundus along an axis 146, which is at the angle about 17 degrees from the optical axis 16. Therefore, when the patient views the target 124, the region of the fundus illuminated by subsystem 18 in Fig. 1 and imaged by subsystem 42 onto screen 66 in Fig. 1 is about 17 degrees toward the temple, i.e., the region of the optic disk for the left eye 12. Proper focusing and magnification are obtained with lens 126, lens 128 and lens 144 for imaging the target 124 onto the fundus of the left eye 12 as described above for the right eye 12, and fine positioning is achieved by moving target 124 across screen 122.

To position the visual axis or optic axis of either eye 12 so that the fovea is imaged, the patient simply looks along the optical axis 16 to receive light from the source 20. The target 124 is not displayed at this time, so that no light enters eye 12 along axis 138 on axis 146. Other regions of the fundus than the optic disk can be imaged by providing another suitably positioned light source or target (not shown) on which the patient's vision can be fixed.

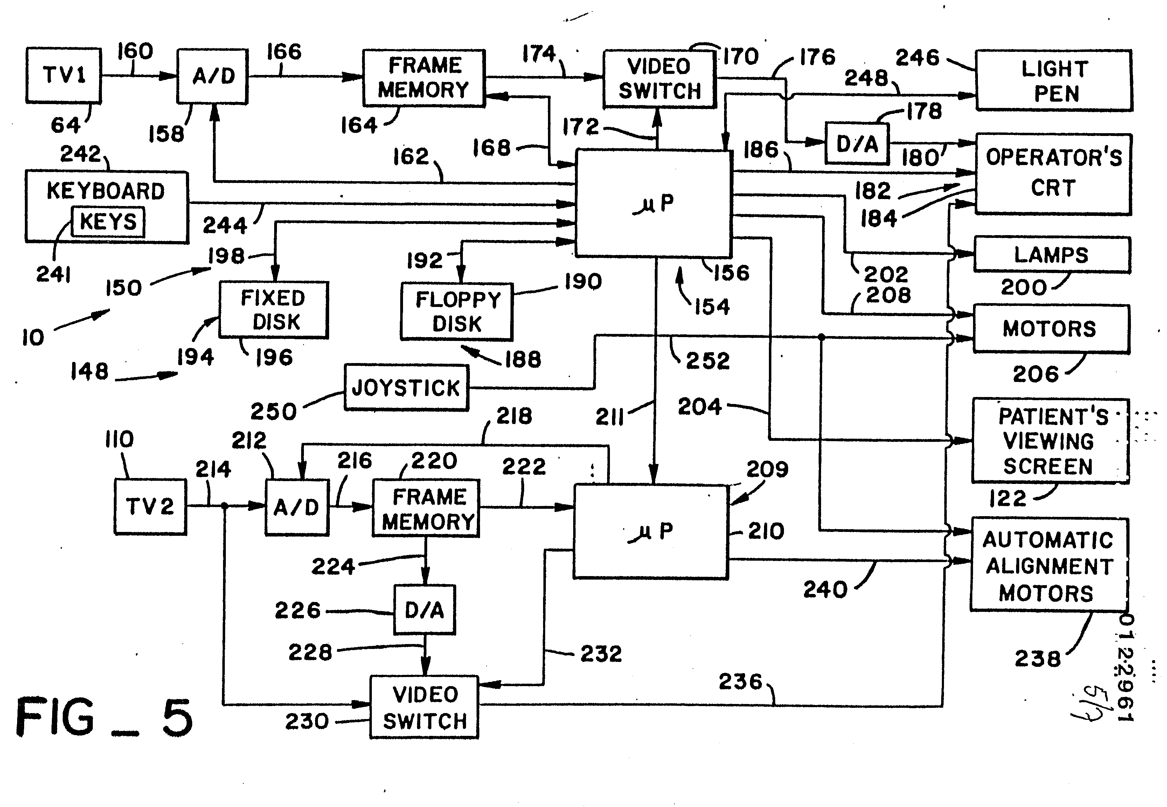

Fig. 5 shows a block diagram of an electronic system 148 of the instrument 10 for storing digitally and displaying data about the ocular fundus and for performing other functions to be described. Electronic system 148 constitutes a data processor 150 having a microcomputer 154, such as a Model 8086 microprocessor 156 manufactured by the Intel Corporation, Santa Clara, California. Microprocessor 156, under software control, primarily performs the data storage and display functions.

An analog-to-digital (A/D) converter 158 receives, on a line 160, output video analog signals from the screen 66 in response to the scanning by TV camera 64 and converts the analog signals to a series of digital signals of, for example, 8-bit length. Thus, the intensity of the light falling on each small region of the screen is converted to analog data by camera 64 and then digitized by A/D converter 158. Microprocessor 156 controls the range of the A/D converter 158 over a line 162 to perform the equivalent of automatic gain control, ensuring that the full 8-bit range of the digitized levels is used regardless of the output level or amplitude of the analog signals on line 160. A frame memory 164 receives the digital signals from A/D converter 158 on a line 166 and temporarily stores digitally a frame at a time or one entire scanned picture of the image of the region of the ocular fundus on screen 66. Frame memory 164 is under read/write control of, and transfers an entire frame of data to, microprocessor 156 over a bi-directional line 168.

An on-off video switch 170, when turned on in response to a control signal from microprocessor 156 on a line 172, couples each frame of digital data stored in frame memory 164 from a line 174 to a line 176. A digital-to-analog (D/A) converter 178 converts the digital data on line 176 to video analog signals which are outputted on a line 180. An operator's monitor or display 182, such as a CRT display 184, displays the information received on line 180, i.e., the image of the ocular fundus falling on screen 66 is displayed on CRT display 184. Microprocessor 156 controls the CRT display 184 to display this image information via a control line 186 carrying the usual display control signals.

A digital data storage device 188, such as a floppy disk 190 used with a floppy disk drive (not shown), communicates with microprocessor 156 over a bi-directional line 192 to store a plurality of frames of digital data, each acquired from frame memory 164. Thus, floppy disk 190 can store digitally fundus images acquired over time from a patient during two or more eye examinations, and can store digitally a plurality of fundus images acquired during a single eye examination.

Another digital data storage device 194, such as a hard disk 196 used with a Winchester disk drive (not shown), is in communication with microprocessor 156 via a bi-directional line 198. Hard disk 196 can store the system and applications software to be described for performing the various functions carried on by instrument 10. Hard disk 196 can also be back-up storage for floppy disk 190.

Microprocessor 156 controls the turning on and off of various lamps 200 over a line 202. The lamps 200 include light source 20, light source 100 and light source 102 shown in Fig. 1. Microprocessor 156 controls the patient's viewing screen 122 (see also Fig. 4) via a control line 204 by generating and positioning the target 124 to examine various regions of the optical fundus, as previously described. Furthermore, microprocessor 156 controls the energization and de-energization of a plurality of motors 206 over a line 208 which move various optical components, as will be described.

Data processor 148 also includes a microcomputer 209 such as a Model 8088 microprocessor 210 manufactured by the Intel Corporation. Microprocessor 210 is controlled or instructed by microprocessor 156 via a line 211 primarily to initiate and perform the optical alignment functions. An analog-to-digital (A/D) converter 212 converts the video analog signals being outputted by TV camera 110 over a line 214 to digital data on an output line 216. A/D converter 212 is a one-bit digitizer or comparator which compares each analog signal identifying the intensity of light falling on a corresponding position on screen 108 with a threshold level and produces a logic 1 or 0 on a line 216 for each such comparison. Microprocessor 210 adjusts the threshold level of A/D converter 212 via a control line 218 for reasons to be described.

A frame memory 220 temporarily stores a frame of digital data, i.e., stores all the bits on line 216 constituting one scan by TV camera 110, and transfers this frame of digital data over a line 222 to microprocessor 210. The digital data in frame memory 220 also are fed over a line 224 to a digital-to-analog (D/A) converter 226 that converts the data back to video analog signals which are outputted on a line 228. An on-off video switch 230 is under control by microprocessor 210 over a line 232 to couple, when turned on, the analog signals on line 228 and the analog signals on line 214 onto a line 236. The CRT display 184 receives the signals on line 236 to display the images falling on screen 108 and scanned by TV camera 110.

Microprocessor 210, in response to the digital data on line 222, and under control of the software stored on hard disk 196, controls the energization and de-energization of various automatic alignment motors 238 over a line 240.

Microprocessor 156 and, hence, microprocessor 210, are commanded to perform their functions by means of input keys 241 on a keyboard 242, coupled over a line 244 to microprocessor 156, and a light pen 246 which is coupled to and activated by microprocessor 156 via a bi-directional line 248. Light pen 246 can be applied to the screen of CRT display 184 for reasons to be described. Also shown in Fig. 5 is a manual control lever or joystick 250 which is used to actuate one or more of the motors 206 and one of the motors 238 over a line 252 for manual alignment and other purposes to be described.

In the overall operation of instrument 10, including optical system 14 and data processor 150, for examining the eye 12, assume that the instrument 10 is powered-up and is otherwise ready to perform its functions. Also assume that a patient has been initially positioned with her eye 12 approximately in the correct position relative to optical axis 16 and that the region of the ocular fundus to be examined is the fovea, so that the patient is looking directly along optical axis 16.

Manual and Automatic Alignment

To assure the highest accuracy, the

0122961

manual and automatic alignment procedures should be sequentially activated. The operator first depresses a manual alignment key 241 on the keyboard 242, causing microprocessor 156 to energize light source 102 and to activate CRT display 184. Microprocessor 156 also instructs microprocessor 210 to close video switch 230 and, thereby, couple TV camera 110 to CRT display 184. An image of the facial area of about 25 mm. in diameter, including the pupil of the eye 12, is then formed on screen 108, scanned and read out by TV camera 110 as analog signals on line 214. Video switch 230 then couples the analog signals onto line 236, ultimately resulting in the pupil image being seen by the operator on CRT display 184.

To coarsely center the pupil image on the optical axis 16, the operator then manipulates joystick 250 to energize one of the motors 206 to move the entire instrument 10 toward or away from the eye 12 until the pupil image appears sharply focused on CRT display 184. Then, the operator manipulates the joystick 250 to energize another of the motors 206 which moves the entire instrument 10 sideways horizontally relative to the eye 12 until the pupil image is centered roughly horizontally on the CRT display 184. Next, the operator manipulates the joystick 250 to energize one of the automatic alignment motors 238 which causes rotatable mirror 40 to rotate about its horizontal axis A1. This causes the image of the pupil seen on CRT display 184 to move vertically and when it is roughly centered the operator ceases this movement. At this time, then, the image of the pupil of the eye 12 is roughly centered on CRT display 184, as well as on screen 108 of TV camera 110 which is fixedly centered on the optical axis 16 of the instrument 10. Therefore, the eye 12 is roughly aligned with optical axis 16.

Having manually and roughly centered the eye 12 relative to the optical axis 16, the operator then depresses an automatic alignment key 241 on the keyboard 242. Microprocessor 156 responds by turning off light source 102 and turning on light source 20, and by instructing microprocessor 210 to switch to the automatic alignment mode. As a result of the infrared light emerging from the output end 116 of fiber optic bundle 112, an image of the pupil on a dark background is now formed on the screen 108 of TV camera 110.

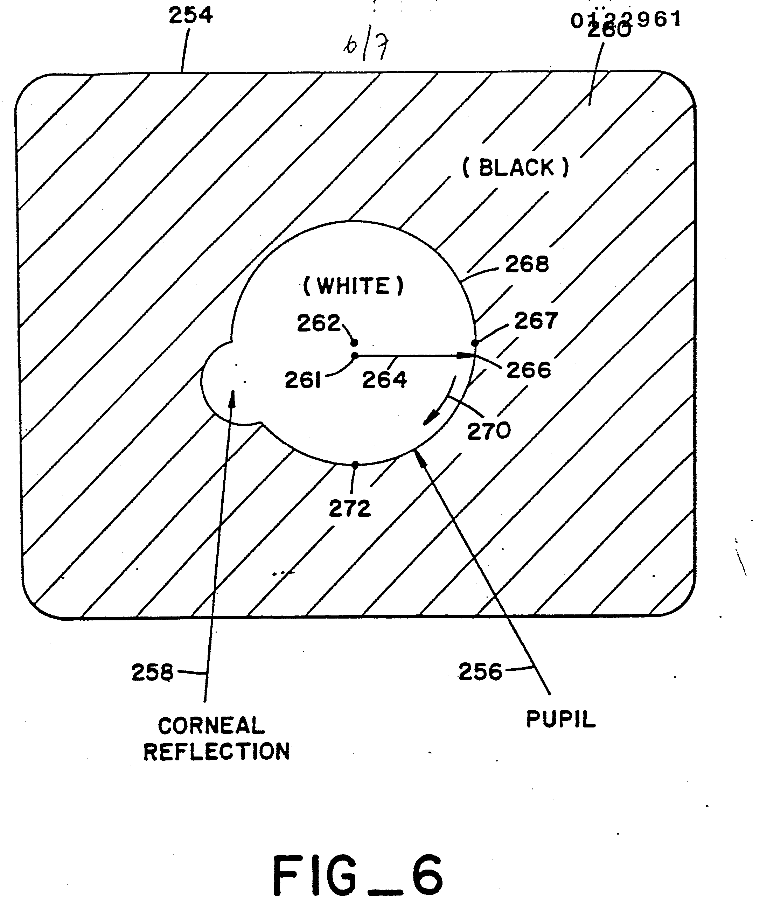

As one example, Fig. 6 illustrates a picture 254 that is at the screen 108 during a threshold setting procedure that is described below. To show a worst case eye reflection situation, the picture 254 includes the white image 256 of the pupil in which a small white area 258 also appears, the area 258 resulting from, for example, some corneal reflection that obscures part of the pupil image. Also shown is the dark background 260.

Next, the threshold of A/D converter 212 is initially set by microprocessor 210 to the highest level. Thereafter, one frame or picture of the pupil image on screen 108 is scanned by TV camera 110, digitized by A/D converter 212 and stored in frame memory 220, with each stored bit representing a single pixel of a white or dark level. Microprocessor 210 then accesses the frame of bits stored in frame memory 220 and counts the total number of bits representing white pixels, which should be few due to the high threshold level while the total number of bits representing the black pixels should be high. Microprocessor 210 then sets the threshold level of A/D converter 212 one step lower, and the above procedure is repeated, i.e., again the total number of bits representing white pixels is counted, which should be several more than previously counted due to the slightly lower threshold level. This procedure of lowering the threshold level and counting the "white" bits is followed until there is an abrupt change in the total number of "white" bits counted. At this point, the appropriate threshold level is reached for continuing with the automatic alignment procedure.

To further adjust the threshold level, if necessary, microprocessor 210 then closes video switch 230 which allows the present digitized picture in frame memory 220, after being converted by D/A converter 226, to be coupled to and displayed on CRT display 184. Thus, the picture falling on screen 108 is displayed on CRT display 184. The operator, by means of a control knob (not shown) can then view the display 184 and fine tune the threshold level of A/D converter 212 until a very clear picture of the white pupil image is seen contrasted against a dark background.

With the picture appearing on screen 108 as shown in Fig. 6, and being scanned by TV camera 110, digitized by A/D converter 212 and stored in frame memory 220, the automatic alignment procedure continues as follows in order to determine the center of the pupil image 256 relative to the center of screen 108. Microprocessor 210 accesses a frame of bits stored in frame memory 220 and examines the bits by starting with the bit corresponding to a pixel 261 near the center 262 of image 256, which bit represents a white pixel since the pupil image 256 has been roughly centered on screen 108 during manual alignment. Then, microprocessor 210 examines sequentially bits corresponding to pixels on a horizontal line to the right of the pixel 261, as shown by the arrow 264 in Fig. 6. When a bit corresponding to a black pixel is encountered, as shown by numeral 266, microprocessor 210 then examines the bits corresponding to the border 268 by following the border 268 vertically upward from pixel 266, so long as the direction of border 268 is toward the right. Otherwise, microprocessor 210 turns around and begins to trace the border 268 vertically downward from pixel 266, so long as the direction of the border 266 is to the right. In this way, the bit corresponding to the rightmost position of border 268 is identified, e.g., the bit corresponding to a pixel 267, thereby defining the vertical position of the center 262 of the pupil.

Thereafter, microprocessor 210 examines the bits identifying pixels around the pixel 267 by following the border 268 downwardly along the arrow 270 until the edge of the border 268 begins to turn upwardly at pixel 272. At this pixel 272, the horizontal position of the center 262 of the pupil is identified. Thus, the bit corresponding to pixel 267 and the bit corresponding to pixel 272 define the X-Y coordinate data of the center 262 of the pupil image 256.

Next, using this X-Y coordinate data of the center 262, and having available the known X-Y coordinate data of the center of the screen 108, microprocessor 210 calculates the difference between the pupil center 262 and the center of screen 108 and generates respective X and Y error signals. In response to these error signals, microprocessor 210 then drives respective X and Y alignment motors 238 to rotate mirror 40 about its vertical axis A2 and its horizontal axis A1 until the pupil image 256 is centered and no error signals are generated. The generation of the error signals and rotation of mirror 40 continuously occur during the examination to follow small eye movements, thereby constantly automatically aligning the eye 12 relative to optical axis 16. The above procedure is for the right eye; for the left eye the corneal reflection 258 lies on the opposite side from that shown and the search commences towards the left rather than towards the right along line 264.

Acquisition of Digital Data, Generally

With the automatic alignment procedure described above being in effect, digital data can be acquired by first depressing a select key 241 on keyboard 242 corresponding to, for example, white light. Microprocessor 156 responds by energizing a motor 206 to rotate filter wheel 70 to position the appropriate filter 74 or aperture

0122961

on wheel 70 along the optical axis 16.  Thus, the ocular fundus is illuminated with white light from source 20.

Then, a read key 241 is depressed, causing microprocessor 156 to read a frame of digital data stored in frame memory 164 and to store this data in floppy disk 190.  Other such readings can be taken merely by depressing the read key 241.

Acquisition of Reflectance Spectrum Data

With the automatic alignment procedure being in effect, a select key 241 corresponding to one wavelength of light is depressed.  Microprocessor 156 responds by energizing a motor 206 to rotate filter wheel 70 to position the corresponding filter 74 along the optical axis 16.  Thus, the ocular fundus is illuminated at the selected wavelength.  Then, the read key 241 is depressed, causing microprocessor 156 to read the frame of data in frame memory 164 and to store these data in floppy disk 190.  This first frame of data stored in floppy disk 190 may be considered to be a "master" frame for registration purposes to be described.

Then, additional readings at different wavelengths can be taken.  For each reading, a different select key 241 is depressed, causing microprocessor 156 to operate a motor 206 to rotate filter wheel 70 until another selected filter 74 is along the optical axis 16.  Next, the read key 241 is depressed, resulting in a frame of data being stored in floppy disk 190 corresponding to the other selected wavelength.  Thus, frames of data are stored in floppy disk 190, each

0122961

corresponding to the amplitude of light at a different wavelength falling on the ocular fundus and all the frames taken together thereby containing information from which to derive a reflectance spectrum.

Between readings taken at the different wavelengths, slight movements of the eye 12 will occur, resulting in readings being taken of slightly different regions of the ocular fundus. Consequently, for reasons which will become apparent, all frames of data stored in floppy disk 190 should be registered with one another and this is accomplished in the following manner.

A key 241 calling up the "master" frame is depressed, and microprocessor 156 responds by reading the "master" frame in floppy disk 190 and displaying the corresponding fundus image on CRT display 184 as a positive picture. Then, another key 241 is depressed, causing microprocessor 156 to read another frame of data stored in floppy disk 190 and taken at a different wavelength and to display the corresponding fundus image on display 184 as a negative picture simultaneously with the positive "master". The operator will then manipulate a control knob (not shown) on the CRT display 184 to move or shift the negative image in registration with the positive image until they are precisely aligned or at least well matched. This visual registration is accomplished by aligning features on CRT display 184, such as blood vessels of the ocular fundus. During this movement, microprocessor 156 receives data from CRT display 184 identifying the magnitude of the amount and the direction of the shift. When the shift or registration is completed, the operator depresses a key 241 which instructs the microprocessor 156 to store this magnitude and direction data in floppy disk 190. These data are later used to compute the correct spatial relationships among these two images or pictures so that pixels at the same location in the two pictures can be assumed to represent the same point on the fundus.

Thereafter, with the "master" frame still being displayed on CRT display 184, each of the other frames of data taken at different wavelengths can be sequentially fetched and displayed as a negative on display 184 and then registered with the "master" to acquire the shift magnitude and direction data for the corresponding picture. Alternatively, both the "master" and all other frames of data can appear as positives on display 184. The positive master and a given other positive frame can appear alternately on the display 184 at, for example, two alterations per second. The operator can then move one of these frames until they are superimposed on one another, i.e., registered. Again, the shift magnitude and direction data are acquired.

Once the pictures taken at different wavelengths have been digitally stored in floppy disk 190 and registered as described above, various information about the ocular fundus can be displayed on CRT display 184, as will now be described.

### Spot Reflectance Spectrum

The operator depresses a key 241 causing microprocessor 156 to read a selected stored

0122961

picture in floppy disk 190 and to display this image of the ocular fundus on CRT display 184. Then, the operator uses the light pen 246 to point to any desired spot or pixel on the CRT display 184. In response to this pointing of the light pen 246, microprocessor 156 reads the stored data in each of the stored pictures in floppy disk 190 taken at different wavelengths and corresponding to the spot or pixel at which light pen 246 is pointing. Since each picture was taken at a different wavelength, these read data identify the energy or intensity of the spot reflectance at different wavelengths and are processed by microprocessor 156 to display on CRT display 184 a graph of the light intensity vs. wavelength for that spot, which is the reflectance spectrum. In a similar manner, the light pen 246 can be used to point to any other pixel on the picture called up on CRT display 184 to provide the reflectance spectrum for that spot of the ocular fundus. In producing this reflectance spectrum, microprocessor 156 will correct for the light sensitivity of the camera 64, using the sensitivity data previously acquired, as mentioned above.

### Feature Enhancement

Other information that can be obtained relates to various features of the ocular fundus. For example, it may be important to see the distribution of oxygenated blood in the region of the ocular fundus being examined. The operator can then depress a key 241 calling for information about this distribution. Microprocessor 156 responds by reading two particular stored pictures in floppy disk 190 taken at different wavelengths. Microprocessor 156 then takes the ratio of the intensities of each of the corresponding pixels on the two pictures and produces new data that are proportional to each such ratio. These new data constitute a "ratio picture" which is then displayed on CRT display 184. In performing this operation, microprocessor 156 again corrects for the light sensitivity of camera 64.

More specifically, since information about the oxygenated blood is desired, one of the two stored pictures selected by microprocessor 156 is the one taken at a wavelength for which oxygenated and reduced blood have equal reflectances and the other at a wavelength where those two reflectances are as different as possible. Under these conditions, it can be shown that the ratio of the intensities of the two pictures at any pixel is proportional to the concentration of oxygenated blood at that pixel. Thus, the displayed ratio picture is information about the distribution of oxygenated blood.

To further enhance the ratio picture or render this information more visible, the ratio picture can be split by the microprocessor 156 and pseudocolored, e.g., all pixels with ratios between 0.0 and 0.1 can be colored blue, all pixels with ratios between 0.1 and 0.2 can be colored green, etc. Alternatively, iso-ratio contour lines can be calculated and superimposed on the displayed ratio picture. Quantitative measures are also available, such as the total area of the ratio picture in which the ratio is between, for example, 0.4 and 0.5.

### Change Enhancement

In this mode, a change in the ocular fundus is the information that is acquired. For this mode, it is assumed that, for example, two pictures of a given region of the ocular fundus have been taken and digitally stored at substantially different times, e.g., weeks apart. The two pictures are first registered with one another in the manner described above. Then, a "change enhancement" key 241 is depressed calling for this feature. In response, microprocessor 156 reads the data stored in floppy disk 190 for these two pictures, calculates the ratio of corresponding pixels and displays on CRT display 184 a ratio picture in the manner described above for the Feature Enhancement mode. This ratio picture will be a uniform grey, except where changes have occurred. The quantitative aspects of the change can be visually enhanced by using level splitting, pseudocolor and contour lines as described above.

### Alternatives to Feature Enhancement and Change Enhancement

As an alternative to calculating and displaying a ratio picture for either the Feature Enhancement or the Change Enhancement mode, similar information can be obtained with a "difference picture". Microprocessor 156, instead of calculating a ratio, determines the difference in intensity of the corresponding pixels of the relevant pictures after correcting for the sensitivity of camera 64, and displays the resulting difference picture on CRT display 184.

## Abnormalities in the Two-Dimensional Shape

Other important information that can be acquired with the instrument 10 is any abnormality or change in the two-dimensional shape of the region of the ocular fundus under examination. For example, such a change can be in the size of a discolored area or in the tortuosity of blood vessels. The change can be analyzed by using the same procedure described above for the Change Enhancement mode, i.e., by computing a ratio or difference picture and displaying it on CRT display 184.

## Abnormalities in the Three-Dimensional Shape

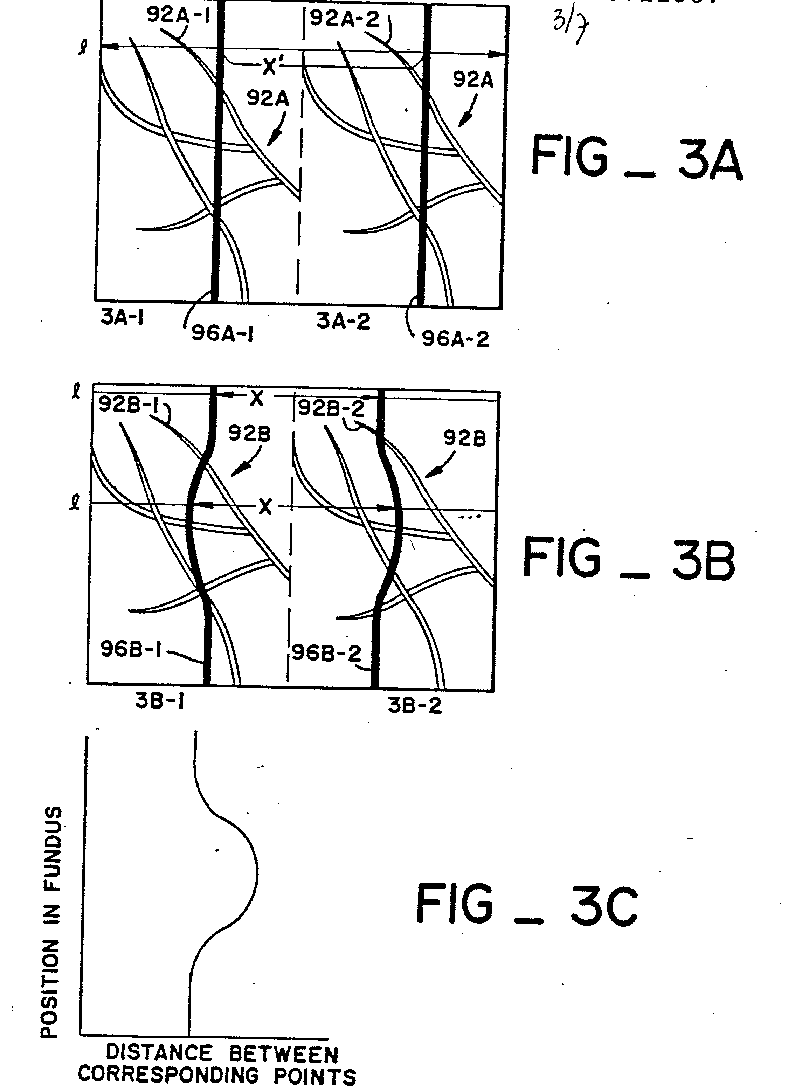

The operator commences this mode by depressing a "three-dimensional shape" key 241 on keyboard 242. Then, microprocessor 156 actuates one of the motors 206 to move mask 94 into position along the optical axis 16 and actuates another of the motors 206 to move system 82 into the position along the optical axis 16. Consequently, a stereo pair of images 92A of a region of the fundus, such as shown in Fig. 3A, may be obtained at detecting plane 66. However, rather than the stereo pair of images shown in Fig. 3A, assume that the stereo pair is as shown in Fig. 3B, i.e., stereo pair 92B. As will become apparent, whereas the straight lines 96A-1, 96A-2 of Fig. 3A indicate a region of the ocular fundus that is flat, the corresponding curved lines 96B-1, 96B-2 indicate that this region is not flat, but is subject to, e.g., cupping.

Thereafter, the stereo pair of images 92B is scanned by TV camera 64, digitized by A/D converter 158 and stored in floppy disk 190 by microprocessor 156. Then, as will be further described, microprocessor 156 measures along horizontal scan lines 1 the distances X shown in Fig. 3B between corresponding features on the stereo pair 92B, such as one of the blood vessels 92B-1, 92B-2 or, preferably, the lines 96B-1, 96B-2. The distances X are each proportional to the distance in the depth dimension between an optical reference point near the center of the pupil of the eye 12 and the region being examined on the fundus. Therefore, if the region being examined were not cupped (and neglecting camera distortions), all the distances X between lines 96B-1, 96B-2 would be identical, as shown for lines 96A-1, 96A-2 in Fig. 3A. However, since the region is cupped, these distances X are not all equal, as can be clearly seen in Fig. 3B.

Having measured the distances X along all the horizontal scan lines, microprocessor 156 can now control CRT display 184 to display a depth profile of the fundus region coincident with lines 96B-1, 96B-2. This depth profile is illustrated in Fig. 3C, which is a graph of the region of the fundus illuminated along the lines 96B-1, 96B-2 vs. distances X. This depth profile of Fig. 3C represents what would be seen if the fundus were cut along the region coincident with lines 96B-1, 96B-2 and then viewed edge-on.

To generate a highly accurate depth profile, the distances X should be measured with great precision. In general the accuracy of the depth profile shown in Fig. 3C depends on the ability to identify corresponding areas along a horizontal scan line 1 of line 92B-1 and line 92B-2.

This identification can be difficult because even if the fundus is illuminated with a very fine and sharply focused vertical line as a result of imaging the slit of mask 94 shown in Fig. 1, the resulting images of this line shown in Fig. 3B as lines 96B-1, 96B-2 may appear defocused because of light scatter by the fundus, as well as from optical aberrations in the eye 12 and in the instrument 10.

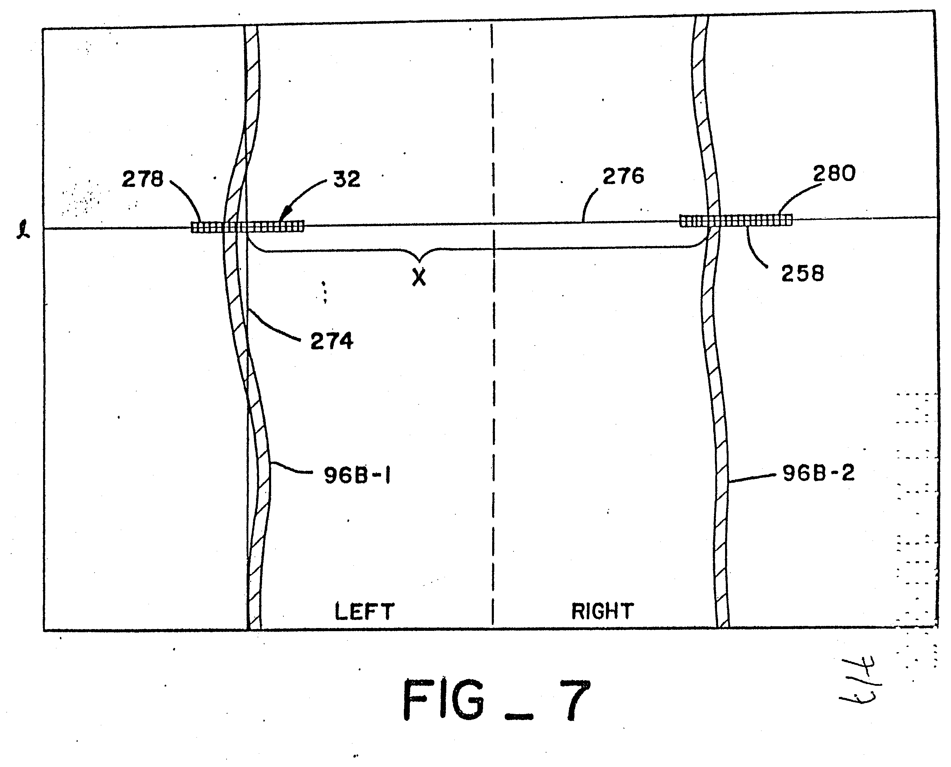

Preferably, therefore, to acquire a highly accurate depth profile, a technique is used that will be described with reference to Fig. 7 which shows an expanded view of the lines 96B-1, 96B-2, but not the other fundus features, such as the blood vessels, to simplify the illustration. Microprocessor 156 generates a fine vertical line 274 on screen 66 that is approximately centered on line 96B-1 of the left image of the stereo pair 92B. Because the exact location of line 274 relative to line 96B-1 is not critical, and because the location of line 96B-1 on screen 66 will be approximately in the same place for all eyes 12, the line 274 can be generated at the same location for all eyes.

Then, microprocessor 156 evaluates each horizontal scan line, such as a scan line 276, in turn in the following way. Assume there are 256 pixels along a scan line 276, with 128 pixels in each image of the stereo pair 92B numbered 0-127 and 128-255, respectively. As a scan along a horizontal line 276 occurs, A/D converter 158 generates the digital data identifying the intensity of the light at each of the 256 pixels and microprocessor 156 temporarily stores these data in system memory. Then, microprocessor 156 evaluates a pixel group 278 containing, for example, 32 pixels, and centered on the line 274, by reading the digital data stored in system memory 190 and corresponding to this group 278. Next, microprocessor 156 evaluates the first 32 pixels of the scan line 276 for the right side of the stereo pair 92B, e.g., pixels numbered 128-159, by reading the corresponding stored digital data. Microprocessor 156 then correlates the pixel group 278 with the group of pixels numbered 128-159. Then, microprocessor 156 reads the stored data corresponding to the next group of pixels 129-160 and correlates this group with the pixel group 278, and so on.

This correlation procedure continues until the pixel group 278 centered on line 274 has been correlated with every group of 32 pixels numbered 128-159, 129-160, 130-161, etc., of the right picture of the stereo pair 92B. The position of the center of the group of 32 pixels in the right picture, for example group 280 shown in Fig. 7, for which the correlation is the highest is determined to be the position in the right picture that corresponds to the center of the pixel group 278. The distance between these two centers is the distance X for that scan line 276. When this procedure is completed for each horizontal scan line, and the distance X determined for each scan line, microprocessor 156 then generates the depth profile shown in Fig. 3C on CRT display 194.

As an example, the pixel group 278 has been given as containing 32 pixels, but the group can contain a different number, e.g., 16. Further, because for even the largest cupping in the fundus, the actual displacement in the third dimension is small, the region of the right picture of the stereo pair 92B having the highest correlation with the pixel group 278 along a scan line 276 can usually be found within a restricted pixel group. Therefore, it is not necessary to correlate every pixel group in the right picture with the group 278. Typically, as few as 16 groups centered around the middle of the right picture need be examined.

As previously mentioned, the only reason for using the mask 94 is to illuminate those areas of the fundus under examination that are essentially featureless, e.g., that have no blood vessels, or any features that cannot be adequately distinguished by optical system 14 and TV camera 64, to acquire the necessary stereo pair of images on screen 66. For those regions that contain features such as blood vessels that are distinguishable, mask 94 is not required. Then, a procedure similar to that described above could be used to display a depth profile of a given region by evaluating the distances X between corresponding blood vessels in the stereo pair.

In summary, therefore, the present invention solves the problems previously mentioned in connection with prior fundoscopic detection techniques for acquiring data for detecting disorders of the eye as well as the entire body. The data that are acquired and processed with the present invention are objective data and highly accurate, unlike the data acquired by a physician using an ophthalmoscope. Also, more accurate reflectance spectra data are acquired using the present invention than are acquired using the prior fundus camera. This is because the present invention does not have the problems associated with developing film that has a plurality of photographs taken at different wavelengths. Moreover, none of the prior techniques provide an accurate and objective technique for evaluating the three dimensional shape or changes in the shape or color of the fundus over time, as does the present invention.

Included as a part of this disclosure at the end of the specification and prior to the claims is a Topograph (Ocular Fundus Analyzer) Procedure Glossary followed by software that is used to perform the previously mentioned functions of the present invention and other functions that will become apparent. The software is divided into a Listing No. 1, a Listing No. 2 and a Listing No. 3. The Topograph Procedure Glossary includes a Word column, a Vocabulary column and a Source column. The Word column gives a particular word followed beneath by a sentence indicating the function of a portion or routine of the software, e.g., "Hit-Detects illuminated pixels beneath lightpen. The Vocabulary column identifies the component which is associated with the word in the Word column, e.g. Video, which relates to the Operators CRT 184. The Source column gives the location, in hexadecimal, of the corresponding portion of the software in the following software listing, e.g. location 4584 corresponding to Hit.

The software is written in a language known as Forth and a Forth implemented 8086 Assembler, which is sold by Forth, Inc. The software is a modified version of Forth, Inc.'s commercially available program 86/12-202 PROM polyForth, dated March 27, 1982. This software was developed specifically for the 86/12A single board microcomputer, manufactured by the Intel Corporation and an SBC202 floppy disk controller; however, as described below, modifications have been made to support a floppy disk controller model 1403D, manufactured by Shugart Associates, Sunnyvale, California. As the name PROM polyForth implies, the software is implemented in EPROM (i.e., four 2716's). The 86/12A single board microcomputer includes the microprocessor referenced herein as microprocessor 156.

A polyForth Target Compiler was used to change the above-mentioned EPROMs to support the Shugart 1403D floppy disk controller. The only changes were to redefine the disk support words FDC, FLOPPY, (BUFFER) and (BLOCK). This is indicated in Listing No. 1 of the software, which listing contains the code for these words.

Listing No. 2 of the software contains the object code for the 86/12A single board microcomputer. This software can be stored in precompiled form on a disk, read into RAM and then started using FORTH commands.

The various motors, mirrors, lamps and pupil alignment camera 110 previously described are controlled directly by the peripheral microprocessor 210, which can be the 8088 manufactured by the Intel Corporation. The software for the 8088 can be target compiled by the 86/12A as a closed headless FORTH system. The 86/12A and the 8088 can communicate using a shared memory region which they can jointly access. This region also can be used to exchange messages and hold common variables. Listing No. 3 contains the object code for the 8088 peripheral processor.

Other aspects, objects and advantages of the invention can be obtained from a study of the drawings, the disclosure and the appended claims.

Topograph Procedure Glossary

WORD.........VOCABULARY..........SOURCE...........

AHIT          VIDEO                   4584
   Detects illuminated pixels beneath lightpen.
ARED          8088                    7AE
   Tests whether the 8086 is requesting 8088 action.
ATIP          VIDEO                   4564
   Tests for lightpen tip depression.
ACQ           8088                    811
   Using the adress stored in 86/88 communication area,
   fetches a byte and leaves in the comm area.
ACQ           86 ->88                 518F
   Request the alignment processor to fetch a byte of
   its memory.
ACKREQ        8088                    7C5
   Acknowledges an 8086 request.
AGCPROF       VIDEO                   4COC
   Real time frame grabbing with automatic gain control
   of the SIT camera ADC.
AUTOREF       8088                    A15
   Computes the ADC reference value that gives the
   spharpest pupil edge.
AUTOREF       86 - 88                 5181
   Requests the alignment processor to compute an op-
   timal alignment camera ADC reference value for di-
   gitizing a 1 bit image of the pupil.
AVID          VIDEO                   464A
   Selects alignment video signal as RGB DAC reference.
CENTER        8088                    D8A
   Computes the center of the 1 bit pupil in grab RAM.
CPRO          CORRELATE               5FEF
   Computes the depth profile for a given pair of ver-
   ticales lines.
DAVE          OPERATOR                .5809
   Displays 1 bit digitezed alignment so that operator
   can center the pupil to requeting eyetracking.
DAVID         VIDEO                   4660
   Select digitized alignment video as RGB DAC reference.
DEPTH         CORRELATE               5C09
   Computes the peak variance and depth of the fundus
   for a single raster line.
EXAM          OPERATOR                5899
   Enters the standard patient examination routine for
   aquiring and storing fundus data.
EYETRACK      8088                    DED
   Tracks the pupil at 30 Hz, servoing if enabled, and
   monitoring requests from 8086.

Topograph Procedure Glossary

WORD.........VOCABULARY.........SOURCE

EYETRACK       86 ->88              51A9
   Requests the alignment processor to begin tracking the
   pupil.
EYETRACK/      8088                 E18
   Stops tracking.
EYETRACK/      86 ->88              51C3
   Request the alignment processor to stop tracking the
   pupil.
FILTER         CORRELATE           5BCF
   A nonrecursive variable low pass digital filter rou-
   tine used to smooth the crosscorrelation function
   prior to computing its peak.
FINDSLITS      CORRELATE           5F1B
  Locates the vertical lines projected on the fundus in
  VRAM.
FROMDISC       VIDEO               48A2
   Reads a frame from disk to VRAM.
GRAB           VIDEO               45EB
   Writes the next frame from the SIT camera to video RAM.
HIST           8088                998
   Computes a histogram of light pixel tallies as a func-
   tion of alignment camera a ADC reference value.
HOME           OPERATOR            530F
   Initializes video switch, CRAM, cmaeras, motors & bi-
   stables.
HSCROLL        VIDEO               45F9
   Updates the horizontal scroll register.
INIT88         8088                80
   Initializes the 8088, target FORTH systems pointers
   and the 8255 I/O port.
IOREQ          86 ->88             4F39
   Interprocessor communication primitive used to select
   alignment processor functions.
LIVE           VIDEO               46A4
   Select either SIT camera ADC oder VRAM with which to
   enter the color RAM lookup tables.
MAN            OPERATOR            5794
   Displays live alignment video so that operator can center
   the pupil manually before requesting automatic eyetracking
MONITOR        8088.               A37
   Monitors 8086 request & performs them by vectored execution
MONKEYS        OPERATOR            5764
   Monitors the operator input and executes primary control
   routines by vectored execution.
PALLOR         ANALYSIS            64F4
   Computes the pallor of two registered pixels and stores the
   value in VRAM.

Topograph Procedure Glossary
WORD.......... VOCABULARY... SOURCE. ......................,,...................

FALLORKEYS    ANALYSIS       68EA
    Displays the pseudocolored contents of VRAM and enables
    interactive control of the assignment of colors to,the values
    in VRAM.  Used primarily for enhancing small differences in
    ratio pictures.              .

PEAK          CORRELATE      5AF7
    Finds the peak of the crosscorrelation function.

RATIO         ANALYSIS       652E
    Computes in place the ratio of two registered frames.

REALREG       VIDEO          4708
    Select the display mode for registering a stored frame with
    a live frame.

REG           VIDEO          46A4
    Select the display mode for registering two stored frames.

REGISTER      ANALYSIS       6440
    Displays a positive fundus frame overlaid by a negative fundus
    for manual registration by horizontal & vertical scrolling of
    one image with respect to the other.

START88       8088           E23
    Initializes interrupt service and monitors 8086 requests.

TALLYHI       8088           96E
    Counts the number of light pixels in pupil grab RAM.

TODISK        VIDEO          48AE
    Writes a frame from VRAM to disk.

VBLANK        VIDEO          4634
    Selects ground as the RGB reference.

VRAM          VIDEO          4676
    Selects video RAM as RGB reference.

SCROLL        VIDEO          460E
    Updates the vertical scroll register.

WIPE          VIDEO          4623
    Clears video RAM to FF.

XCORRELATE    CORRELATE      5A9C
    Computes the crosscorrelation function of two vertical lines
    illuminated on the fundus.

[BLOCK]       +DISK          2D3F
    Error trapping version of BLOCK.
    The system variable 'BLOCK vectors to this routine.        ~

[BUFFER]      +DISK          2CED
    Version of BUFFER which tests for disk errors and retries
    10 times issuing an error message if necessary.
    The system variable 'BUFFER vectors to this routine.

## LISTING NO. 1

```
1A33   58 BF 76 20 AA    59 81 F9 67  2 7E  5 B8    20  0 EB
1A4J    2 2B C0 AA D1    E1 D1 E1 86 C0 B8 C1 AB    BU  4  8
1A53   AB B8 76 20 E6    F4 BA C1 E6 F5 2B C0 E6    F7 A2 60
1A63   20 5B 50 E6 F2    8A C1 E6 F3 2B C0 E6 F6    B0  1 E6
1A73   F0 E9  E EB 50    1E 8C C8 8E D8 E4 F0 BA    E0 E4 F1
1A83   A3 60 20 57 8B    3E 5E 20 C7  5 FF D7 5F    1F 5B CF
1A93    8 28 42 55 89    19 3A  0 3D  5  D 19 17     A 17  0
1AA3    A 31 1A 3D  5    19 19 4D  0  7 28 42 4C    93 1A 3A
1AB3    0 81  4 B7  1    88  2 7F  5 DF  9 BF  A    3D  5 2E
1AC3   19 56  2 17  0     8 31 1A 46  2 7B  A 3D     5 19 19
1AD3   4D  0  5 44 52    49 B8 18 3A  0  F  0 68     2 F8  2
1AE3   81  4 C6  1 4D     0
```

Code for the Redefinition of:

    FDC
    FLOPPY
    (BUFFER)
    (BLOCK)

to support Shugart 14Ø3D disk controller for the
main computer (86/12A)

Code in EPROM Ø-1FFF is otherwise identical to
FORTH, Inc.'s software product called
86/12-2Ø2 PROM polyFORTH.

| | | | | | | | | | | | | | | |
|---|---|---|---|---|---|---|---|---|---|---|---|---|---|---|
| 2000 | C8 | 20 | 0 | FE | CE | 19 | 0 | FE | CE | 19 | 0 | FE | CE | 19 | 0 | FE |
| 2010 | CE | 19 | 0 | FE | CE | 19 | 0 | FE | CE | 19 | 0 | FE | CE | 19 | 0 | FE |
| 2020 | CE | 19 | 0 | FE | 1 | 4A | 0 | FE | FF | 27 | 0 | FE | FB | 69 | FA | F8 |
| 2030 | 37 | 6A | FA | F8 | A0 | 2D | 0 | FE | 28 | 4A | 0 | FE | 1C | DD | C1 | F2 |
| 2040 | 51 | 0 | 7F | 36 | 5D | 36 | 6E | 36 | E8 | 35 | DE | 35 | 82 | 26 | B5 | 36 |
| 2050 | DA | 34 | 51 | 0 | C8 | 2F | AB | 2F | 0 | 0 | 9F | 25 | 87 | 36 | 0 | 0 |
| 2060 | 0 | C1 | 4 | 0 | 4 | 0 | A8 | 1 | A4 | 1 | 64 | 0 | A9 | 1 | 0 | 0 |
| 2070 | 0 | 0 | 0 | 0 | 0 | 0 | B | 0 | 5 | 4C | 4 | 0 | 0 | 0 | 0 | 0 |
| 2080 | 33 | 2E | 6D | 2 | 2A | 7B | 0 | 0 | 3 | 4B | 45 | 59 | CF | 19 | 3A | 0 |
| 2090 | BA | 4 | B7 | 1 | 8B | 3 | C0 | 3 | 95 | 3 | C7 | 5 | 46 | 2 | BA | 4 |
| 20A0 | C6 | 1 | 4D | 0 | 4 | 45 | 4D | 49 | E | 18 | 3A | 0 | C0 | 3 | 95 | 3 |
| 20B0 | AB | 5 | 38 | 2 | 4D | 0 | F6 | 7 | 7 | 54 | 52 | 41 | 50 | 50 | 45 | 44 |
| 20C0 | BE | B6 | 20 | FB | AD | 97 | FF | 25 | 2B | C0 | 99 | CF | 9 | 49 | 4D | 4D |
| 20D0 | 7A | 18 | 3A | 0 | F | 0 | 80 | 0 | F0 | 4 | B7 | 1 | B7 | 1 | D5 | 1 |
| 20E0 | 4D | 0 | B | 44 | 45 | 46 | D5 | 1A | 3A | 0 | DE | 4 | B7 | 1 | E7 | 4 |
| 20F0 | C6 | 1 | 4D | 0 | 6 | 46 | 4F | 52 | A4 | 20 | 3A | 0 | 7F | 9 | 17 | 0 |
| 2100 | 8 | 99 | 2 | 28 | 2 | A8 | 4 | C6 | 1 | B9 | 2 | E7 | 4 | DE | 4 | C8 |
| 2110 | 2 | E2 | 0 | 79 | 2 | B7 | 1 | 35 | 4 | 9B | 1 | D6 | 0 | 9 | C8 | 2 |
| 2120 | C8 | 2 | 87 | 1 | CC | 0 | F0 | 79 | 2 | C6 | 1 | 17 | 0 | 2 | 20 | 1 |
| 2130 | EZ | 38 | 2 | 4D | 0 | 6 | 41 | 54 | 54 | 37 | 19 | 3A | 0 | DE | 4 | C8 |
| 2140 | 2 | 17 | 0 | 10 | 1D | 3 | 4D | 0 | 7 | 44 | 49 | 53 | E2 | 20 | 66 | 0 |
| 2150 | 27 | 0 | 8 | 50 | 52 | 49 | E5 | 18 | 66 | 0 | 2A | 0 | 8 | 43 | 4F | 4D |
| 2160 | 48 | 21 | 66 | 0 | 5A | 0 | 6 | 2B | 46 | 4F | 88 | 20 | 66 | 0 | 15 | 0 |
| 2170 | 5 | 54 | 41 | 53 | 5C | 21 | 66 | 0 | 1C | 0 | 9 | 32 | 43 | 4F | 35 | 21 |
| 2180 | 3A | 0 | 5E | 0 | 24 | B | D6 | B | FF | 75 | 4 | FF | 75 | 2 | AD | 97 |
| 2190 | FF | 25 | 2 | 30 | 2E | 20 | 52 | 21 | 88 | 21 | 0 | 0 | 0 | 0 | 5 | 32 |
| 21A0 | 53 | 57 | 7A | 21 | A6 | 21 | 5A | 5F | 58 | 59 | 57 | 52 | 51 | 50 | AD | 97 |
| 21B0 | FF | 25 | 5 | 32 | 4F | 56 | 9E | 21 | BA | 21 | 8B | FC | FF | 75 | 6 | FF |
| 21C0 | 75 | 4 | AD | 97 | FF | 25 | 2 | 44 | 2B | 20 | 70 | 21 | CE | 21 | 5A | 58 |
| 21D0 | 88 | FC | 1 | 45 | 2 | 11 | 15 | AD | 97 | FF | 25 | 6 | 44 | 4D | 49 | C6 |
| 21E0 | 21 | E3 | 21 | 5A | 58 | F7 | D8 | 83 | D2 | 0 | F7 | DA | 50 | 52 | AD | 97 |
| 21F0 | FF | 25 | 2 | 44 | 2D | 20 | DB | 21 | 3A | 0 | E1 | 21 | CC | 21 | 4D | 0 |
| 2200 | 3 | 44 | 30 | 3D | F2 | 21 | 3A | 0 | 61 | 1 | 46 | 2 | 61 | 1 | 2E | 1 |
| 2210 | 4D | 0 | 2 | 44 | 3C | 20 | 0 | 22 | 3A | 0 | F8 | 21 | 85 | 1 | 46 | 2 |
| 2220 | 38 | 2 | 4D | 0 | 4 | 44 | 41 | 42 | 12 | 22 | 3A | 0 | 28 | 2 | 85 | 1 |
| 2230 | D6 | 0 | 2 | E1 | 21 | 4D | 0 | 4 | 44 | 4D | 49 | 24 | 22 | 3A | 0 | B8 |
| 2240 | 21 | F8 | 21 | 28 | 2 | 85 | 1 | D6 | 0 | 5 | CC | 21 | CC | 0 | 2 | 48 |
| 2250 | 4 | 4D | 0 | 4 | 44 | 4D | 41 | 37 | 22 | 3A | 0 | B8 | 21 | B8 | 21 | 3D |
| 2260 | 22 | F8 | 21 | CC | 21 | 4D | 0 | 3 | 4D | 4F | 44 | 50 | 18 | 6F | 22 | 5F |
| 2270 | 2B | D2 | 58 | F7 | F7 | 52 | AD | 97 | FF | 25 | 5 | 2A | 2F | 4D | CC | 20 |
| 2280 | 82 | 22 | 5F | 5A | 58 | F7 | E2 | F7 | F7 | 52 | 50 | AD | 97 | FF | 25 | 2 |
| 2290 | 2A | 2F | 20 | 7A | 22 | 97 | 22 | 5F | 5A | 58 | F7 | EA | F7 | FF | 50 | AD |
| 22A0 | 97 | FF | 25 | 1 | 2F | 20 | 20 | 92 | 21 | AB | 22 | 5F | 58 | 99 | F7 | FF |
| 22B0 | 50 | AD | 97 | FF | 25 | 2 | 4D | 2A | 20 | 67 | 22 | BD | 22 | 58 | 5A | F7 |
| 22C0 | EA | 50 | 52 | AD | 97 | FF | 25 | 2 | 4D | 2F | 20 | B5 | 22 | CF | 22 | 5F |
| 22D0 | 5A | 58 | F7 | FF | 50 | AD | 97 | FF | 25 | 2 | 55 | 2A | 20 | F4 | 20 | E1 |
| 22E0 | 22 | 58 | 5A | F7 | E2 | 50 | 52 | AD | 97 | FF | 25 | 5 | 4D | 2F | 4D | C7 |
| 22F0 | 22 | F3 | 22 | 5F | 5A | 58 | F7 | F7 | 52 | 50 | AD | 97 | FF | 25 | 2 | 4D |
| 2300 | 2B | 20 | EB | 22 | 6 | 23 | 58 | 99 | E9 | C5 | FE | 2 | 54 | 2A | 20 | 53 |
| 2310 | 22 | 13 | 23 | 89 | 76 | FE | 89 | 5E | FC | 5B | 5E | 5F | 8B | C7 | F7 | E3 |
| 2320 | 50 | 8B | CA | 8B | C6 | F7 | E3 | 3 | C8 | 83 | D2 | 0 | B | F6 | 79 | 2 |
| 2330 | 2B | D3 | B | DB | 79 | 4 | 2B | CF | 1B | D6 | 51 | 52 | 8B | 5E | FC | 8B |
| 2340 | 76 | FE | AD | 97 | FF | 25 | 2 | 54 | 2F | 20 | B | 23 | 4E | 23 | 5F | 5A |
| 2350 | 58 | 59 | B | FF | 9C | 79 | 2 | F7 | DF | B | D2 | 79 | 2 | 3 | D7 | F7 |
| 2360 | F7 | 91 | F7 | F7 | 9D | 79 | 7 | F7 | D8 | 83 | D1 | 0 | F7 | D9 | 50 | 51 |
| 2370 | AD | 97 | FF | 25 | 3 | 4D | 2A | 2F | FE | 22 | 3A | 0 | 6 | 2 | 11 | 23 |
| 2380 | 17 | 2 | 4C | 23 | 4D | 0 | A | 42 | 41 | 43 | B2 | 21 | 3A | 0 | FF | B |
| 2390 | 6 | 2 | 56 | 2 | 88 | 2 | 28 | 2 | 4 | 6 | AA | 2 | 88 | 2 | 17 | 0 |
| 23A0 | F | 2E | 1 | 17 | 0 | F | 50 | 1 | 28 | 2 | CC | 21 | 28 | 2 | 4 | 6 |
| 23B0 | C8 | 2 | 88 | 2 | 24 | B | 46 | 2 | 4 | 6 | 88 | 2 | 24 | B | 79 | 2 |
| 23C0 | 12 | B | 17 | 2 | 88 | 2 | 17 | 0 | 3 | 99 | 2 | F6 | A | 4D | 0 | 5 |
| 23D0 | 42 | 55 | 49 | 86 | 23 | 3A | 0 | EB | 18 | B7 | 1 | 56 | 2 | 28 | 2 | B7 |
| 23E0 | 1 | 46 | 2 | C8 | 2 | C8 | 2 | E5 | 1 | 1D | 3 | 12 | 4 | 28 | 2 | EB |
| 23F0 | 18 | B7 | 1 | C8 | 2 | CA | 1 | 17 | 0 | 0 | 88 | | | | | |

```
2400    B 54 45 52 46 23 3A  0    FF  B 17  0 19  2  E 4D
2410    0  9 43 4F 4E  0 24 3A     0 28  2 05 23 28  2 17
2420    0  5 88  2 46  2 B7  1     F  0  E  0 88  2 17  0
2430   10 1D  3 4D  0  6 50 52    4F A3 22 3A  0 3D 19 94
2440    D F4  5 15  8  2 75 70    E4  5 8B  3 C6  9  4 53
2450   45 4E 11 24 3A  0 28  2    B7  1 60  4 B7  1 56  2
2460    F  0  8  0 88  2 B7  1    17  0 50 1D  3 B1  4 46
2470    2  F  0 1E  0 88  2 17  .  0 42 1D  3 CC  4 B7  1
2480   BA  4 C6  1 3D 19 8B  3    8F  9 C6  9  8 28 41 53
2490   AC 1A 3A  0 17  2 C8  2    46  2 C6  1 4D  0 86 41
24A0   53 53 CF 23 3A  0 37  B    92 24 37  B 3A  0 4D  0
24B0    5 49 4E 50 8F 22 B8 24    5A 28 C0 EC 50 AD 97 FF
24C0   25  6 4F 55 54 35 24 C9    24 5A 58 EE AD 97 FF 25
24D0    6 2F 44 49 C1 24 DB 24    58 59 2B D2 F7 77 22 91
24E0   F7 77 22 50 51 52 AD 97    FF 25  1 23 20 20 4E 24
24F0   3A  0 D6 24 A2  6 8E  6    4D  0  2 23 53 20 EA 24
2500   3A  0 F0 24 35  4  6 22    D6  0 F7 4D  0  4 28 44
2510   2E 8C 24 3A  0 46  2 56     2 2A 22 B8  6  0 25 DE
2520    6 C8  6 4D  0  2 44 2E    28 FA 24 3A  0 13 25 AB
2530    5 80  6 4D  0  3 44 2E    52 25 25 3A  0  6  2 13
2540   25 17  2 56  2 99  2 6C     6 AB  5 4D  0  6 2A 44
2550   49 B0 24 55 25 5F 5A 58    59 52 F7 67 22 91 F7 67
2560   22  3 C7 13 CA 5A 50 51    52 AD 97 FF 25  6 57 49
2570   54  D 25 75 25 5A 59 58    2B FF 3B C2 79  5 3B C1
2580   78  1 47 57 AD 97 FF 25     3 2B 49 27 66 21 90 25
2590   FF 46  2 AD 97 FF 25  8    28 4E 55 6D 25 3A  0 8B
25A0    3  6  2 28  2 AA  2 E5     1 17  0 2D 77  1 28  2
25B0    6  2 88  2 98 21 67  2    F5  8 D6  0  5 53 25 CC
25C0    8 F6 28  2 E5  1 28  2    17  0 3A 77  1 46  2 17
25D0    0 2C 17  0 30 73 25 88     2 D6  0  5 8E 25 CC  0
25E0   D7 E5  1 17  0 20 99  2    F6  7  1 3F 17  2 D6  0
25F0    2 E1 21 17  2 61  1 D6     0  2 38  2 4D  0  3 44
2600   2F 59 35 25 66  0 B5  5     4 4A 41 4E 4D 25 99  0
2610   A7 2D  4 4C 45 41 88 25    99  0  0  0  3 4D 54 48
2620   74 23 3A  0 5E  0 D6  B    E8 75 DA B7  1 17  0 3A
2630   56  2 9B  1 18 26 B7  1    F8  2 88  2  E 26 B7  1
2640   88  2 88  2 4D  0  3 4A    4I 4E  8 26 28 26  0  0
2650    3 46 45 42 D9 22 28 26    1F  0  3 4D 41 52 1C 26
2660   28 26 3B  0  3 41 50 52    9E 24 28 26 5A  0  3 4D
2670   41 59 5A 26 28 26 78  0     3 4A 55 4E 46 26 28 26
2680   97  0  3 4A 55 4C 78 26    28 26 B5  0  3 41 55 47
2690   64 26 28 26 D4  0  3 53    45 50 FE 25 28 26 F3  0
26A0    3 4F 43 54 D0 24 28 26    11  1  3 4E 4F 56 6E 26
26B0   28 26 30  1  3 44 45 43    96 26 28 26 4E  1  2 41
26C0   44 20 8C 26 3A  0  F  0    9D  7 99  2  4 26 17  0
26D0    4 80 22  F  0 6E  1 99     2  E 26 C6  1 17  0  3
26E0   77  1 18 26 C6  1 4D  0     3 2E 59 52 AA 26 3A  0
26F0    F  0 DB  2 88  2 17  0     4  4 26 80 22  F  0 9C
2700    7 88  2 46  2 17  0  4     9  3 AA  2 28  2 67  2
2710   61  1 D6  0 14 28  2 17     0 3C A9  1 99  2 46  2
2720   28  2 17  0 3B A9  1 99     2 67  2 4D  0  4 2E 4D
2730   54 E8 26 3A  0  F  0 4E    26 17  0  B 8B  3 E2  0
2740   35  4 17  0  A 88  2 B7     1 A9  1 D6  0  A 17  0
2750    A 88  2 95  3 CC  0  3    17  0  B 20  1 E2 46  2
2760   38  2 28  2 17  0  8 99     2  6  2 B7  1 99  2 17
2770    2 4D  0  2 27 27 20 97    25 3A  0 17  0 20 8E  6
2780   4D  0  6 28 44 41 73 27    3A  0 B8  6 EE 26 8B  3
2790   F0 24 F0 24 F0 24 F0 24    48  4 79 27 33 27 17  0
27A0   FD 72  4 D5  1 25  6 72     4 B7  1 46  2 1D  3 79
27B0   27 8B  3  0 25 C8  6 4D     0  5 2E 44 41 2D 27 3A
27C0    0 CF  3 D6  0  9 88 27    AB  5 80  6 CC  0  E 15
27D0    8  8 44 59 20 4D 54 48    20 4E 4F 57 20 4D  0  5
27E0   54 4F 44 B4 26 66  0 80    20  5 54 49 43 DF 27 66
27F0    0 82 20  4 31 44 41 BE    26 88 21 1E  4  0 B0 9C
```

LISTING NO. 2

Sheet 2

```
2800   50 1E B8   0 FE 8E D8 FF     6 84 20 75    4 FF    6 82
2810   20 1F 58 9D CF    5 43 4C    4F E9 27 1D 28 E4 C2 24
2820   FB E6 C2 AD 97 FF .25   6    2D 43 4C E9 27 2F 28 E4
2830   C2  C   4 E6 C2 AD 97 FF    25    7 43 4F 55 15 28 41
2840   28 FF 36 84 20 AD 97 FF    25    5 54 49 4D 39 28 3A
2850    0 3F 28 46   2 99   2 17     0    A 17   0    8 95 22 35
2860    7 4D   0   2 4D 53 20 27    28 .3A   0 3F 28 46   2 17
2870    0   8 17   0   A 95 22 88     2 7F   5 28   2 3F 28 9B
2880    1 D6   0 F5 38   2 4D   0     3 50 53 54 A0 26 3A   0
2890   2D 28 17   0 64 F1 22 17     0 3C F8   2 88   2  F   0
28A0   80 BB DF 22 EF 27 24   4    1B 28 4D   0   5 40 54 49
28B0   88 28 3A   0 2D 28 F9 27    EF 27 12   4 88 21 FB 21
28C0   28  2 85   1 61   3 D6   0     9 95   3 E5 27 D5   1 CC
28D0    0 EA CC 21 35   4 EF 27    24   4 1B 28 4D   0   5 2E
28E0   54 49 63 28 3A   0 8B   3    B8   6 F0 24 17   0   6 C3
28F0    4 C6   1 F0 24 E0   D 17     0 3A 8E   6 F0 24 F0 24
2900   C8   6 4D   0   4 54 49 4D    49 28 3A   0 82 28  F   0
2910   80 BB F1 22 E4 28 AB   5    80   6 38   2 4D   0   3 4E
2920   4F 57 DE 28 3A   0 E5 27    C6   1 4D   0 9C 50 53 1E
2930   B8   0 FE 8E D8 8C CB D1    E3 D1 E3 D1 E3 D1 E3 8B
2940   47   A 48 78 23 89 47   A    75   6 C7   7 FF D7 EB 18
2950   52 87 77   C 8B 57 14 AC -   87 77   C EE 82 C2   4 88
2960    2  0 EE 90 90 40 EE 5A    1F 5B 58 9D CF   7 28 50
2970   52 82 27 75 29 8B 57 14    FF 47   A CD  F E9   5 DC
2980    7 50 52 49 AC 28 99   0    10 2C   2 54 54 20 20 20
2990   20 20 19 CA   0 CB   0 7E    19 8A 29 8A 29   5 41 4C
29A0   4C 4F 54 20 20 20   8 32    39 33 33 6E 27 74 20 75
29B0   6E 69 71 75 65 20 38   2    CC   4 C6   1 73   0 4D   0
29C0    7 2E 43 52 .1E 29 3A   0    E4   5 15   8   5 20 20 20
29D0   20 20 CC   4 B7   1 17   0    20 75   8 25   6 AB   5 C3
29E0    4 B7   1 F1   D  4   6 80     6 53   7 C3   4 C6  1 CC
29F0    4 C6   1 73   0 4D   0  4    4C 4F 41 12 26 3A   0 E4
2A00    5 15   8   4 20 20 20 20    28   2 35   7   2  E 4D   0
2A10    4 46 49 4C 50 26 18 2A    58 8A E0 E9 85 D9   5 4E
2A20   46 49 C0 29 3A   0 67   2    67   2 D8   2 56   2 88   2
2A30   46   2 E2   0 28   2 79   2    C6   1 17   0   2   7  1 F4
2A40   38   2 4D   0   4 50 55 4D    8A 29 3A   0 56   2 88   2
2A50   46   2 E2   0 79   2 F7   1    F1   0 F9 4D   0   5 4E 50
2A60   55 1E 2A 3A   0 D8   2 56     2 88   2 46   2 E2   0 79
2A70    2 C6   1 17   0   2   7  1    F6 4D   0 85 4C 41 42 F7
2A80   29 3A   0 FF   B 36   D 4D     0   2 43 3B 20   4 29 3A
2A90    0 E7   4 B7   1 DE   4 C6     1 4D   0   2 3E 3C 20 5D
2AA0   2A A3 2A 59 86 CD 51 AD    97 FF 25   3 28 43 40 7B
2AB0   2A B3 2A 5F 8B   D 8A CD    2A ED 51 AD 97 FF 25   3
2AC0   2B 43 21 AB 2A C7 2A 5F    58 47 AA AD 97 FF 25   3
2AD0   44 54 43 89 2A 66   0 F0     0   2 52 44 20 F3 27 66
2AE0    0   8   0   2 57 52 20 6D    29 66   0   A   0   6 54 4F
2AF0   50 CF 2A 99   0   0   0   5    57 49 4E E3 2A 99   0   0
2B00    0   3 43 44 42 ED 2A 99     0   8   0 58   4   0 4F
2B10   54 20 20   A 53 45 4E   1    2B 99   0 4F 43   5 41 9C
2B20   50 1E B8   0 FE 8E D8 E4    F0 8A E0 E4 F1 A3 60 20
2B30   57 8B 3E 5E 20 C7   5 FF    D7 5F 1F 58 9D CF   8 44
2B40   54 43 13 2B 46 2B B8   9    2B E6 F4 8A C4 E6 F5 2B
2B50   C0 E6 F7 A2 60 20 58 50    E6 F2 8A C4 E6 F3 8B   6
2B60   F5 2A E6 F6 B0   1 E6 F0    E9 1A DA   3 57 44 43 F7
2B70   2A 3A   0   7 CA   0   0 FE    CA 2C CA   0   0 FE 10 2C
2B80    6   0   2 F0 A0 2C   1   0    75   0 40   6 8C 2B   0   0
2B90   50 52 49 4E 54 20 37 30    30 30 20 32 30 30 30 20
2BA0   54 54 20 50 20 20 43 52    20 43 52 20 20 34 30 30
2BB0   20 2B 4C 4F 4F 50 20 3B    20 43 52 20 13 32 35 36
2BC0   20 20 53 4D 4F 56 45 4C    4F 41 44 20 43 52 50 54
2BD0   59 2D 42 55 46 46 45 52    53 FF FF FF D2   6 86   6
2BE0    8 16 20 20 20 44 49 53    4B 20 45 52 52 4F 52 3A
2BF0   20 20 20 54 59 50 45 20    D2   6 7D   7 B8   7 FF 7A
```

```
"C00   10 2C  0 2C  0 30 5F 6C    0 2C  0 70 9D  9 8A 24
"C10   2E EA A0 8F  0 FE 8A 2B   90 2B  0  0 48  6 86 19
2C20   86 19 75 29 CA  0 C8  0   7E 19 8A 29 8A 29  0  0
2C30   12  0 18  0 12  0  0  0   51  0 B0 6B 37 6C 4D 6C
2C40   1C 66 A9 68 53 66 A0 6B   8F 6B 51  0 CE 8F 15  0
2C50    0  0  0  0  0  0  0  0    0  0  0  0  0  0  0  0
2C60    0  0  0  0  0  0  0  0    0  0  0  0  0  0  0  0
2C70    0  6 3F 45 52 44 2A 3A    0 3D  5 C8  2 E5  1 17
2C80    0  3 2E  5 50 52 49 80   29 3A  0 86 29 54 24 4D
2C90    0  4 46 49 4C 50 26 99   2C 58 8A E0 E9  4 D7  5
2CA0   4E 46 49 1E 29 3A  0 67    2 67  2 D8  2 56  2 88
2CB0    2 46  2 E2  0 28  2 79    2 C6  1 17  0  2  7  1
2CC0   F4 38  2 4D  0  4 50 55   4D 83 2C 3A  0 56  2 88
2CD0    2 46  2 E2  0 79  2 F7    1 F1  0 F9 4D  0  5 4E
2CE0   50 55 9F 2C 3A  0 D8  2   56  2 88  2 46  2 E2  0
2CF0   79  2 C6  1 17  0  2  7    1 F6 4D  0 85 4C 41 42
2D00   12 26 3A  0 FF  B 36  D   4D  0  2 43 3B 28  4 29
2D10   3A  0 E7  4 B7  1 DE  4   C6  1 4D  0  2 3E 3C 28
2D20   DE 2C 24 2D 59 86 CD 51   AD 97 FF 25  3 2B 43 40
2D30   FC 2C 34 2D 5F 8B  D 8A   CD 2A ED 51 AD 97 FF 25
2D40    3 2B 43 21 2C 2D 48 2D   5F 58 47 AA AD 97 FF 25
2D50    3 44 54 43  A 2D 66  0   F0  0  2 52 44 20 F3 27
2D60   66  0  8  0  2 57 52 28   6D 29 66  0  A  0  6 54
2D70   4F 50 50 2D 99  0  0  0    5 57 49 4E 64 2D 99  0
2D80    0  0  3 43 44 42 6E 2D   99  0  8  0  1 90  4  0
2D90   4F 54 20 20  A 53 45 4E   82 2D 99  0 4F 43  5 41
2DA0   9C 50 1E B8  0 FE 8E D8   E4 F0 8A E0 E4 F1 A3 60
2DB0   20 57 88 3E 5E 20 C7  5   FF D7 5F 1F 58 9D CF  8
2DC0   44 54 43 94 2D C7 2D 8B   8A 2D E6 F5 8A C4 E6 F5
2DD0   2B C0 E6 F7 A2 60 28 58   50 E6 F2 8A C4 E6 F3 8B
2DE0    6 76 2D E6 F6 B0  1 E6   F0 E9 99 D7  3 57 44 43
2DF0   78 2D 3A  0 88 2D 56  2   17  0 20 77  1 D6  0  6
2E00   17  0  A CC  0  3 17  0    6 CB 2C C5 2D 4D  0  5
2E10   53 45 4E BF 2D 3A  0 3D   .5 C8  2 E5  1  6  2 9A
2E20   2D 8B  3 28  2 35  4 79    2 17  0  3 F2 2D 17  2
2E30   48  4 4D  0  B 52 45 43   5A 2D 3A  0 3D  5 C8  2
2E40   E5  1  6  2 9A 2D 8B  3   28  2 35  4 79  2 95  3
2E50   F2 2D 17  2 48  4 4D  0    6 2E 53 45 1C 2D 3A  0
2E60   15  8 16 20 20 20 44 49   53 4B 20 45 52 52 4F 52
2E70   3A 20 20 20 54 59 50 45   20 9A 2D 28  2 E5  1 46
2E80    2 56  2 28  2 17  0 30   2E  1 17  0 10 A9 22 35
2E9    7 15  8  8 20 20 20 43   4F 44 45 20 17  0  F 2E
2EA0    1 35  7 15  8  7 20 20   20 4C 55 4E 20 AA  2 28
2EB0    2 E5  1 17  0 60 2E  1   17  0 20 A9 22 35  7 46
2EC0    2  F  0 80  0 2E  1 61    1 D6  0  5 38  2 CC  0
2ED0   1A 15  8  A 20 20 20 53   45 43 54 4F 52 20 AA  2
2EE0   B7  1 22 2D 53  7 17  0    3 6C  6 17  0  3 6C  6
2EF0   4D  0  6 3F 45 52 C5 2C   3A  0 3D  5 C8  2 E5  1
2F00   17  0  3 2E  1 61  1 61    3 4D  0  5 45 52 52 91
2F10   2C 3A  0 F8 2E D6  0 54   17  0  A 8B  3 E2  0 88
2F20   2D 88 2D 17  0  5 88  2   E2  0 79  2 E5  1 17  0
2F30   FF  7  1 F6 F2 2D F8 2E   61  3 D6  0  2  0  4 F1
2F40    0 DD F8 2E D6  0 25 17    0  7 AA 20 C3  4 B7  1
2F50   F1  D 74 2D B7  1 8B  3   74 2D C6  1 15 2E E4  5
2F60   5E 2E 3A 2E 74 2D C6  1   C3  4 C6  1 4D  0  7 43
2F70   44 42  F 2E 3A  0 8B  3   17  0  4 67  2 28  2 17
2F80    0  4 F8  2 28  2 22 2D   67  2  F  0 67  2 A9  1
2F90   D6  0  6 17  0 20 CC  0    2 88  3 7E 2D B7  1 3F
2FA0    1 4D  0  8 5B 42 55 48   2D 3A  0 3D  5  D 19 17
2FB0    A 74 2F 17  0  A F2 2D   11 2F 3D  5 19 19 4D  0
2FC0    7 5B 42 4C A3 2F 3A  0   B1  4 B7  1 8B  2 7F  5
2FD0   DF  9 8F  A 3D  5 2E 19   56  2 74 2F 17  0  B F2
2FE0   2D 11 2F 46  2 7B  A 3D    5 19 19 4D  0  2 46 3A
2FF0   20  B 2F 3A  0 8B  3 7E   2D C6  1 4D  0  2 57 3A
```

LISTING NO. 2

Sheet 4

```
3000   20 EC 2D 3A  0 17  0 20     7E 2D C6  1 4D  0  3 53
3010   43 40 6E 2F 16 30 8C DA     5F 1F 2B C0 8A  5 50 8E
3020   DA AD 97 FF 25  2 53 40     20  E 30 2D 30 8C DA 5F
3030   1F 8A  5 47 8A 25 50 8E     DA AD 97 FF 25  3 53 43
3040   21 25 30 45 30 8C C2 5F     .7 58 AA 8E C2 AD 97 FF
3050   25  2 53 21 20 3D 30 59     30 8C C2 5F  7 58 AA 8A
3060   C4 AA 8E C2 AD 97 FF 25     .5 .53 4D 4F 51 30 70 30
3070   59 5F 8C D8 8C C2 89 76     FE  7 5E 1F 50 F3 A4 1F
3080   8E C2 8B 76 FE AD ·97 FF    25  4 50 49 43 F2 2E 91
3090   30 8B FC 58 D1 E0  3 F8     FF 35 AD 97 FF 25  4 52
30A0   4F 1C 34 2E 3A  0 28  2     D8  2  6  2 8F 30 C0  3
30B0   28  2 C8  2 17  2 74  E     38  2 4D  0  5 35 55 53
30C0   ED 2F C4 30 59 87 C8 87     C8 87 C8 87 C8 E2 F6 AD
30D0   97 FF 25  4 4D 53 45 58     2E 3A  0  F  0 C8  0 F8
30E0    2 C2 30 4D  0  4 42 45     4C 9E 30 3A  0 17  0  7
30F0   AA 20 40  0  5 51 55 45     E5 30 3A  0 60  4 B7  1
3100   17  0 50 C7  5 4D  0  2     4E 3F 20 D3 30 3A  0 FA
3110   30 8B  3 CC  4 C6  1 17      0 20 75  8 5C  9 4D  0
3120    4 3C 4E 3C C0 2F 3A  0     15  8  4 20 20 5B 20 95
3130    3 8F 30 53  7 15  8  6     3C 20 4E 20 3C 20 17  0
3140    2 8F 30 53  7 15  8  3     5D 20 20  D 31 17  0  2
3150   8F 30 56  2 98  1 61  1     17  0  4 8F 30 17  0  3
3160   8F 30 9B  1 3F  1 28  2     D6  0  8 46  2 38  2 EB
3170   30 E4  5 61  1 D6  0 B0     67  2 67  2 48  4 4D  0
3180    4 42 49 54 F4 30 88 31     58 B9  8  0 D0 C0 BA  .1
3190    0 23 D0 52 E2 F6 AD 97     FF 25  4 53 54 49 68 30
31A0   A2 31 58 89  8  0 BA  1      0 23 D0 52 D0 C8 E2 F6
31B0   AD 97 FF 25  8 4D 4F 56      7 31 3A  0 28  2  6  2
31C0   3F  3 B9  2 2E  1 46  2     17  2 2E- 1 3F  1 4D  0
31D0    5 21 42 49 80 31 3A  0     28  2  6  2 B7  1 46  2
31E0   BA 31 17  2 C6  1 4D  0     . 7 53 43 21 9A 31 3A  0
31F0   35  4  6  2. 6  2 14 30     46 .2 BA 31 17  2 17  2
3200   43 30 4D  0  4 4D 41 53     B4 31  C 32 FA 59 E4 C2
3210    B C1 E6 C2 FB AD 97 FF     25  6 55 4E 4D BC 30 21
3220   32 FA 59 F6 D1 48 E4 C2     23 C1 E6 C2 FB AD 97 FF
3230   25  4 48 41 4C FD 2F 39     32 F4 AD 97 FF 25  5 56
3240   53 59 19 32 3A  0 17  0     40 1F 32 37 32 17  0 40
3250    A 32 40  0  6 56 53 59     3E 32 3A  0 8B  3 E2  0
3260   44 32 F1  0 FB 4D  0  4     3F 4B 45 89 30 6F 32 B8
3270    A  0 E6 C0 E4 C0 25 10      0 50 AD 97 FF 25  7 4B
3280   45 59 20 31 3A  0 8E 20     38  2 4D  0  5 44 32 35
3290   E8 31 94 32 58 5A D1 F8     D1 DA D1 F8 D1 DA D1 F8
32A0   D1 DA D1 F8 D1 DA D1 F8     D1 DA D1 F8 D1 DA D1 F8
32B0   D1 DA D1 F8 D1 DA 52 50     AD 97 FF 25  3 44 32 2F
32C0   8C 32 C4 32 58 5A D1 F8     D1 DA 52 50 AD 97 FF 25
32D0    4 53 2D 3E BC 32 D8 32     58 99 50 52 AD 97 FF 25
32E0    2 2B 2D 20 7E 32 E8 32     5A 59  B D2 79  2 F7 D9
32F0   51 AD 97 FF 25  2 44 2F     20 D0 32 FD 32 59 5A 58
3300   F7 F1 50 AD 97 FF 25  2     44 2A 20 F5 32  F 33 58
3310   59 F7 E1 50 52 AD 97 FF     25  2 54 2B 20  7 33 21
3320   33 5A 59 58 8B FC  1 45      4 11 4D  2 11 15 AD 97
3330   FF 25  6 54 4D 49 19 33     3A 33 5A 59 58 F7 D8 83
3340   D1  0 F7 D9 83 D2  0 F7     DA 50 51 52 AD 97 FF 25
3350    2 54 2D 20 32 33 3A  0     38 33 1F 33 4D  0  2 54
3360   3C 20 50 33 3A  0 56 33     85  1 46  2 38  2 46  2
3370   38  2 4D  0  5 43 4C 45     5E 33 3A  0 F9  4 B7  1
3380   9E  A 28  2 8B  3 46  2     C6  1 C8  2  F  0 FE  3
3390   38  6 66  A 4D  0  6 43     4C 45 74 33 3A  0 E2  0
33A0   79  2 F9  4 C6  1 7A 33     F1  0 F5 4D  0 · 2 2E 52
33B0   20  4 32 3A  0  6  2 D6     32 17  2 3B 25 4D  0  5
33C0   4E 44 55 AD 33 3A  0 8B      3 E2 ·0 E4  5 28  2 17
33D0    0  8 B3 33 17  0  8 8B      3 E2  0 28  2 B7  1 17
33E0    0  8 B3 33 17  0  2 8B      2 F1  0 EF 17  0  8  7
33F0    1 D9 38  2 4D  0  5 44     45 50 96 33 3A  0 60  4
```

```
3100   B7  1 C0  3 99  2 E8  2    17  0  2 99  2 4D  0  2
3110   2E 53 20 BF 33 3A  0 E4     5 FC 33 D6  0 1C C8  3
3120   60  4 B7  1 17  0  4 99     2 E2  0 79  2 B7  1 35
3130    7 17  0 FE  7  1 F4 CC     0  9 15  8  6 65 6D 70
3140   74 79 20 4D  0  3 43 56    44 F6 33 3A  0 C3  4 B7
3150    1 46  2 56  2  9  3 67     2  9  3 17  0  A F8  2
3160   88  2 17  0  A F8  2 88     2 4D  0  4 4C 49 53 E0
3170   32 3A  0 4B 34 65 10 4D     0  6 43 41 52 45 34 3A
3180    0 FF  B F6  A D6  B 58     3 F8 83 C7  2 57 AD 97
3190   FF 25  5 41 52 52 D0 31    3A  0 FF  B D8  2 F6  A
31A0   D6  B 58 D1 E0  3 F8 83    C7  2 57 AD 97 FF 25  7
31B0   43 4D 41 79 34 3A  0 FF     B 28  2 24  B F8  2 F6
31C0    A D6  B E8 DA CB 28  2    B7  1 A4 21 67  2 67  2
31D0   F8  2 88  2 88  2 C8  2    4D  0  6 4D 41 54  F 34
31E0   3A  0 FF  B 28  2 24  8    F8  2 D8  2 F6  A D6  B
31F0   E8 AD CB 28  2 B7  1 A4    21 67  2 67  2 F8  2 D8
3500    2 46  2 D8  2 88  2 88     2 C8  2 4D  0  5 54 41
3510   42 AF 34 3A  0 FF  B 8B     3 E2  0 24  B F1  0 FB
3520   D6  B 58 D1 E0  3 F8 FF    75  2 AD 97 FF 25  6 43
3530   54 41  D 35 3A  0 FF  B    8B  3 E2  0 12  B F1  0
3540   FB D6  B 58  3 F8 8A 45     2 50 AD 97 FF 25  5 43
3550   4D 45 2E 35 3A  0 35  4    88  2 67  2 88  3 8B  3
3560   A4 21 E2  0 79  2 E5  1    8B  3 CC 21 F1  0 F5 67
3570    2 CD 22 4D  0  5 53 46    49 4E 35 7D 35 58 59 5F
3580   8C C2  7 F3 AA BE C2 AD    97 FF 25  5 53 44 55 75
3590   35 3A  0 56  2 88  2 46     2 E2  0 E4  5 79  2 17
35A0    0  5 69  7 80  6 79  2    17  0 10 88  2 E2  3 69
35B0    3 79  2 E2  0 79  2 17     0  7 2E  1 61  1 D8  2
35C0   6C  6 95  3 8F 30 79  2    14 30 17  0  3 69  7 F1
35D0    8 E3 17  0 10 20  1 C3    38  2 E4  5 4D  0  8 57
35E0   4F 52 31 32 99  0 41 20     7 46 4F 52 54 32 3A  0
35F0   7F  9 E4 35 C6  1 17  0    10  A 32 E4 35 B7  1 9E
3600    5 6D 32 D6  0 F5 17  0    10 1F 32 4D  0  4 41 4D
3610   49 92 34 3A  0  F  0 FF    7F 67  2 67  2 D8  2 56
3620    2 88  2 46  2 E2  0 79     2 B7  1 69  3 17  0  2
3630    7  1 F4 4D  0  4 41 4D    41  D 36 3A  0  F  0  0
3640   80 67  2 67  2 D8  2 56     2 88  2 46  2 E2  0 79
3650    2 B7  1 7D  3 17  0  2     7  1 F4 4D  0  6 42 49
3660   4E 35 36 3A  0 17  0  2    C3  4 C6  1 4D  0  A 43
3670   4F 4D 88 35 76 36 58 F7    D8 48 50 AD 97 FF 25  7
3680   3F 43 52 67 32 3A  0 CC     4 B7  1 E0  0 61  3 D6
3690    0 19 E4  5  4  6 25  6    AB  5 15  8  E 20 69 73
36A0   6E 27 74 20 75 6E 69 71    75 65 20 38  2 CC  4 C6
36B0    1 73  0 4D  0  4 4C 4F    41 68 34 3A  0 28  2 35
36C0    7  2  E 4D  0  3 50 2F    52 7F 36 66  0  0  1  3
36D0   58 2F 43 C5 36 66  0  0     1  5 58 43 4D DE 35 66
36E0    0 20  8  5 23 50 4D 6E    36 66  0  1  0  6 58 45
36F0   4E CF 36 66  0 F8 F9  7    41 31 54 5D 36 66  0  0
3700    1  A 46 49 52 E8 35 66     0 11  0  9 4C 41 53 B5
3710   36 66  0 60  0  9 55 50    50  1 37 66  0 16 14  A
3720   4C 4F 57  B 37 66  0 F0    F8  2 4F 4E 20 ED 36 66
3730    0 FF FF  2 49 4E 20 82    26 66  0 FF FF  5 52 49
3740   47 F7 36 66  0 FF FF  3    4F 46 46 29 37 66  0  0
3750    0  3 4F 55 54 47 37 66     0  0  0  4 4C 45 46 1F
3760   37 66  0  0  8  5 23 4B    45 E3 36 66  0 80  0  8
3770   4B 45 59 5B 37 A2 34 22    56 6D 59 22 56 22 56 7B
3780   59 22 56 22 56 22 56 33    59 22 56 22 56 25 59 41
3790   59 B1 56 22 56 22 56 22    56 22 56 22 56 22 56 22
37A0   56 22 56 16 59 5E 59 22    56 22 56 50 59 22 56 22
37B0   56 22 56 22 56 22 56 A5    56 22 56 22 56 22 56 22
37C0   56 22 56 22 56 22 56 22    56 22 56 DB 57 22 56 C4
37D0   59 22 56 D3 59 C9 57 22    56 A7 59 DC 58 B6 59 EA
37E0   58 22 56 F8 58 8A 59  7    59 99 59 22 56 B7 57 E1
37F0   59 22 56 F0 59 22 56 22    56 73 57 22 56 5F 57 1A
```

LISTING NO. 2

Sheet 6

LISTING NO. 2

Sheet 7

```
3800   5A 22 56 26 5A 22 56 22   56 FE 59 22 56 22 56 22
3810   56 22 56 22 56  C 5A 22   56 22 56 22 56 89 57 22
3820   56 22 56 22 56 22 56 22   56 22 56 22 56 22 56 22
3830   56 22 56 22 56 22 56 22   56 22 56 22 56 22 56 22
3840   56 22 56 22 56 22 56 22   56 22 56 22 56 22 56 22
3850   56 22 56 22 56 22 56 22   56 22 56 22 56 22 56 22
3860   56 22 56 22 56 22 56 22   56 22 56 22 56 22 56 22
3870   56 22 56 22 56 22 56  A   4B 15 59 6F 37 66  0  3
3880    0 .A 43 59 43 65 37 A2   34 FD 37 F9 37 1D 38  B
3890   27 43 59 D9 36 99  0 41   42  7 56 52 41 15 37 66
38A0    0  0 20  7 4F 4C 41 51   37 66  0  0 30  7 43 52
38B0   41 81 38 66  0  0 60  7   46 52 41 99 38 66  0  0
38C0   70  7 43 54 52 AD 38 66    0  0 80  7 41 52 41 3D
38D0   37 66  0  0 90  7 53 52   41 C1 38 66  0  0  0  7
38E0   47 52 41 8F 38 66  0  0   10  8 45 50 52 B7 38 66
38F0    0  0 FF  2 56 30 20 E9   38 99  0 45 20  4 48 53
3900   43 DF 38 99  0 3C  0  3   53 45 47 D5 38 99  0  0
3910   20  4 56 41 44 F3 38 99    0 45 20  4 46 48 49 11
3920   39 99  0 45 20  5 58 59   50 FD 38 99  0 20 20  7
3930   58 59 50 25 39 99  0 20   20  6 4F 52 47 A3 38 99
3940    0 20 20  3 56 53 57 1B   39 99  0  0  0  2 56 52
3950   20 43 39 99  0 FF  0  2   48 53 20 2F 39 99  0  0
3960    0  2 56 53 20 4D 39 99    0  0  0  4 58 4D 49 57
3970   39 99  0 45 20  4 58 4D   41 6B 39 99  0 45 20  6
3980   58 52 41 75 39 99  0 20   20  4 59 4D 49 33 37 99
3990    0 45 20  4 59 4D 41 89   39 99  0 45 20  6 59 52
39A0   41 93 39 99  0 20 20  3   50 49 58 39 39 99  0 FF
39B0    0  4 58 4F 46 7F 39 99    0  0  0  4 59 4F 46 9D
39C0   39 99  0  0  0  5 46 52   41 61 39 99  0 11  0  5
39D0   43 59 43  7 39 99  0 20   20  6 54 41 52 CF 39 99
39E0    0 D0  0  5 54 42 41 D9   39 99  0 20  0  3 46 46
39F0   53 C5 39 99  0 45 20  7   46 46 53 ED 39 99  0  8
3A00    0  4 46 4F 4F F7 39 99    0 45 20  2 4C 43 20 77
3A10   38 99  0 40  0  2 52 43   20 CB 38 99  0 C0  0  5
3A20   57 49 44 B1 39 99  0 80    0  9 46 49 52  1 3A 99
3A30    0 14  0  8 4C 41 53  B   3A 99  0 B2  0  6 57 49
3A40   4E 1F 3A 99  0 20  0  5   53 4C 49 E3 39 99  0 20
3A50    0  3 57 53 51 30 3A 99    0 45 20  3 57 47 54 51
3A60   3A 99  0 45 20  4 58 57   47 5B 3A 99  0  1  0  4
3A70   58 4E 2D 65 3A 99  0 45   20  2 58 4E 20 6F 3A 99
3A80    1 45 20  4 59 57 47 BB   39 99  0  1  0  4 59 4E
3A90   2D 83 3A 99  0 45 20  2   59 4E 20 8D 3A 99  0 45
3AA0   20  5 58 4C 45 79 3A 99    0 20 20  6 58 52 49 A1
3AB0   3A 99  0 20 20  5 49 50   45 97 3A 99  0 20 20  5
3AC0   56 50 45 29 3A 99  0 20   20  4 58 53 55 AB 3A 99
3AD0    0 45 20  4 59 53 55 B5   3A 99  0 45 20  6 58 53
3AE0   51 C9 3A 99  0 20 20  5   41  6 59 53 51 D3 3A 99
3AF0    0 20 20  5 41  5 58 59   53 DD 3A 99  0 20 20  5
3B00   41  6 52 53 43 15 3A 99    0 E8  3  6 46 57 49 BF
3B10   3A 99  0 11  0  9 45 56   45  B 3B 99  0 54 48  6
3B20   57 47 54 F5 3A 99  0 20   20  3 58 50 46 1F 3B 99
3B30    0 10  0  5 53 4C 2D 47   3A 99  0 20 20  2 50 23
3B40   20 A7 39 99  0 45 20  3   49 50 49 E9 3A 99  0 18
3B50    0  5 54 48 45 33 3B 99    0 23  0  3 50 48 49 3D
3B60   3B 99  0 2D  0  6 53 54   48 51 3B 99  0 20 20  6
3B70   43 54 48 65 3B 99  0 20   20  4 53 50 48 6F 3B 99
3B80    0 45 20  4 43 50 48 79   3B 99  0 45 20  4 53 50
3B90   53 83 3B 99  0 45 20  5   43 50 53 8D 3B 99  0 20
3BA0   20  2 58 43 20 29 3B 99    0 45 20  2 59 43 20 47
3BB0   3B 99  0 45 20  4 41 5B   4D  1 3B 99  0 45 20  4
3BC0   41 5B 4E B5 3B 99  0 45   20  7 23 43 4F 97 3B 99
3BD0    0  8  0  A 43 4F 4C C9   3B 99  0 42 4C  7 49 58
3BE0   43 AB 3B 99  0 20 20  3   52 45 44 BF 3B 99  0 FF
3BF0    0  5 47 52 45 A1 3B 99    0 FF  0  4 42 4C 55 E7
```

| | | | | | | | | | | | | | | | | LISTING NO. 2 |
|---|---|---|---|---|---|---|---|---|---|---|---|---|---|---|---|---|
| 3C00 | 3B | 99 | 0 | FF | 0 | 5 | 4C | 4F | 4C | 33 | 3A | 99 | 0 | 1 | 0 | 5 | Sheet 8 |
| 3C10 | 55 | 50 | 4C | 15 | 3B | 99 | 0 | FF | 0 | 6 | 50 | 52 | 41 | 5B | 3B | 99 |
| 3C20 | 0 | 20 | 20 | 4 | 4C | 42 | 55 | 5 | 3C | 87 | 34 | 20 | 20 | 20 | 20 | 32 |
| 3C30 | 30 | 20 | 4D | 41 | 59 | 20 | 38 | 32 | 20 | 20 | 20 | 20 | 0 | 0 | 0 | 0 |
| 3C40 | 0 | 33 | 31 | 31 | 0 | 0 | 0 | 0 | 0 | 0 | 0 | 0 | 0 | 0 | 0 | 0 |
| 3C50 | 0 | 0 | 0 | 0 | 0 | 0 | 0 | 0 | 0 | 0 | 0 | 0 | 0 | 0 | 0 | 0 |
| 3C60 | 0 | 0 | 0 | 0 | 0 | 0 | 0 | 0 | 0 | 0 | 4 | 4B | 45 | 59 | 5A | 3A |
| 3C70 | 4 | 4B | 45 | 59 | 60 | 4 | 4B | 45 | 59 | 65 | 3A | 21 | 32 | 20 | 0 | 0 |
| 3C80 | 0 | 0 | 0 | 0 | 0 | 0 | 0 | 33 | 43 | 36 | 41 | 0 | 0 | 33 | 43 | 37 |
| 3C90 | 30 | 0 | 33 | 43 | 37 | 35 | 0 | 0 | 0 | 0 | 0 | 0 | 0 | 0 | 0 | 0 |
| 3CA0 | 0 | 0 | 0 | 0 | 0 | 0 | 0 | 0 | 0 | 0 | 0 | 0 | 0 | 0 | 0 | 0 |
| 3CB0 | 0 | 0 | 0 | 0 | 0 | 0 | 0 | 0 | 0 | 0 | 0 | 0 | 0 | 0 | 0 | 0 |
| 3CC0 | 0 | 0 | 0 | 0 | 0 | 0 | 0 | 0 | 0 | 0 | 0 | 0 | 0 | 0 | 0 | 0 |
| 3CD0 | 0 | 0 | 0 | 0 | 0 | 0 | 0 | 0 | 0 | 0 | 0 | 0 | 0 | 0 | 0 | 0 |
| 3CE0 | 7 | 58 | 43 | 4F | 8C | 38 | 3D | 31 | 20 | 20 | 20 | 0 | 0 | 0 | 0 | 0 |
| 3CF0 | 0 | 0 | 0 | 0 | 0 | 0 | 0 | 0 | 0 | 0 | 0 | 0 | 0 | 0 | 0 | 0 |
| 3D00 | 0 | 0 | 0 | 0 | 0 | 0 | 0 | 0 | 0 | 0 | 0 | 0 | 0 | 0 | 0 | 0 |
| 3D10 | 0 | 0 | 0 | 0 | 7 | 58 | 43 | 4F | C8 | 38 | B6 | 31 | 7 | 58 | 43 | 4F |
| 3D20 | C8 | 38 | BE | 31 | 20 | 20 | 20 | 0 | 5 | 46 | 57 | 8 | 4C | 50 | 52 | 23 |
| 3D30 | 3C | 87 | 34 | 45 | 20 | 20 | 20 | 0 | 0 | 33 | 44 | 31 | 43 | 0 | 0 | 0 |
| 3D40 | 0 | 0 | 0 | 0 | 0 | 0 | 0 | 0 | 0 | 0 | 0 | 0 | 0 | 0 | 0 | 0 |
| 3D50 | 0 | 0 | 5 | 41 | 54 | 45 | 96 | 38 | 3D | 31 | 20 | 0 | 5 | 46 | 57 | 47 |
| 3D60 | DE | 38 | B6 | 31 | 5 | 46 | 57 | 47 | E6 | 38 | BE | 31 | 20 | 0 | 0 | 0 |
| 3D70 | 0 | 0 | 0 | 0 | 7 | 58 | 43 | 4F | 20 | 39 | 7 | 58 | 43 | 4F | 26 | 7 |
| 3D80 | 58 | 43 | 4F | 2B | 39 | 21 | 32 | 20 | 20 | 20 | CA | 38 | B6 | 31 | 5 | 41 |
| 3D90 | 54 | 45 | D2 | 38 | BE | 31 | 20 | 33 | 44 | 37 | 41 | 0 | 33 | 44 | 37 | 46 |
| 3DA0 | 0 | 0 | 0 | 33 | 44 | 38 | 36 | 0 | 0 | 0 | 0 | 33 | 44 | 38 | 45 | 0 |
| 3DB0 | 0 | 0 | 0 | 0 | 0 | 0 | 0 | 0 | 0 | 0 | 0 | 0 | 5 | 46 | 57 | 47 |
| 3DC0 | 3E | 39 | 5 | 46 | 57 | 47 | 44 | 5 | 46 | 57 | 47 | 49 | 39 | 21 | 32 | 20 |
| 3DD0 | 0 | 0 | 0 | 0 | 0 | 0 | 0 | 0 | 0 | 33 | 44 | 42 | 43 | 0 | 0 | 33 |
| 3DE0 | 44 | 43 | 32 | 0 | 33 | 44 | 43 | 37 | 54 | 45 | 2A | 39 | 5 | 41 | 54 | 45 |
| 3DF0 | 30 | 5 | 41 | 54 | 45 | 35 | 39 | 21 | 32 | 20 | 0 | 0 | 0 | 0 | 0 | 0 |
| 3E00 | 0 | 0 | 0 | 33 | 44 | 45 | 36 | 0 | 0 | 33 | 44 | 45 | 43 | 0 | 33 | 44 |
| 3E10 | 46 | 31 | 0 | 0 | 0 | 0 | 0 | 0 | 0 | 0 | 0 | 0 | 0 | 0 | 0 | 0 |
| 3E20 | 0 | 0 | 0 | 0 | 0 | 0 | 0 | 0 | 0 | 0 | 0 | 0 | 0 | 0 | 0 | 0 |
| 3E30 | 0 | 0 | 0 | 6 | 43 | 41 | 4C | D3 | 3B | 87 | 34 | 20 | 20 | 0 | 0 | 0 |
| 3E40 | 0 | 0 | 0 | 0 | 0 | 0 | 0 | 0 | 0 | 0 | 0 | 0 | 0 | 0 | 0 | 0 |
| 3E50 | 0 | 0 | 0 | 0 | 0 | 0 | 0 | 0 | 0 | 0 | 0 | 0 | 0 | 0 | 0 | 0 |
| 3E60 | 0 | 0 | 0 | 0 | 0 | 0 | 0 | 0 | 0 | 0 | 0 | 0 | 0 | 0 | 0 | 0 |
| 3E70 | 0 | 0 | 0 | 0 | 0 | 0 | 0 | 0 | 0 | 0 | 0 | 0 | 0 | 0 | 0 | 0 |
| 3E80 | 0 | 0 | 0 | 0 | 0 | 0 | 0 | 0 | 0 | 0 | 0 | 0 | 0 | 0 | 0 | 0 |
| 3E90 | 0 | 0 | 0 | 0 | 0 | 0 | 0 | 0 | 0 | 0 | 0 | 0 | 0 | 0 | 0 | 0 |
| 3EA0 | 0 | 0 | 0 | 0 | 0 | 0 | 0 | 0 | 0 | 0 | 0 | 0 | 0 | 0 | 0 | 0 |
| 3EB0 | 0 | 0 | 0 | 0 | 0 | 0 | 0 | 0 | 0 | 0 | 0 | 0 | 0 | 0 | 0 | 0 |
| 3EC0 | 0 | 0 | 0 | 0 | 0 | 0 | 0 | 0 | 0 | 0 | 0 | 0 | 0 | 0 | 0 | 0 |
| 3ED0 | 0 | 0 | 0 | 0 | 0 | 0 | 0 | 0 | 0 | 0 | 0 | 0 | 0 | 0 | 0 | 0 |
| 3EE0 | 0 | 0 | 0 | 0 | 0 | 0 | 0 | 0 | 0 | 0 | 0 | 0 | 0 | 0 | 0 | 0 |
| 3EF0 | 0 | 0 | 0 | 0 | 0 | 0 | 0 | 0 | 0 | 0 | 0 | 0 | 0 | 0 | 0 | 0 |
| 3F00 | 0 | 0 | 0 | 0 | 0 | 0 | 0 | 0 | 0 | 0 | 0 | 0 | 0 | 0 | 0 | 0 |
| 3F10 | 0 | 0 | 0 | 0 | 0 | 0 | 0 | 0 | 0 | 0 | 0 | 0 | 0 | 0 | 0 | 0 |
| 3F20 | 0 | 0 | 0 | 0 | 0 | 0 | 0 | 0 | 0 | 0 | 0 | 0 | 0 | 0 | 0 | 0 |
| 3F30 | 0 | 0 | 0 | 0 | 0 | 0 | 0 | 0 | 0 | 0 | 0 | 4 | 4B | 45 | 59 | 2B |
| 3F40 | 3D | A2 | 34 | 20 | 0 | 0 | 0 | 0 | 0 | 0 | 0 | 0 | 0 | 0 | 0 | 0 |
| 3F50 | 0 | 0 | 0 | 0 | 0 | 0 | 0 | 0 | 0 | 0 | 7 | 50 | 52 | 4F | B4 | 38 |
| 3F60 | BB | 31 | 0 | 1 | 20 | 0 | 0 | 0 | 0 | 0 | 0 | 0 | 0 | 0 | 0 | 0 |
| 3F70 | 0 | 0 | 0 | 0 | 0 | 0 | 0 | 0 | 0 | 0 | 0 | 0 | 0 | 0 | 0 | 0 |
| 3F80 | 0 | 0 | 0 | 0 | 0 | 0 | 0 | 0 | 0 | 0 | 0 | 0 | 0 | 0 | 4 | 54 |
| 3F90 | 42 | 4C | 2 | 38 | 66 | 0 | 7 | 50 | 52 | 4F | F0 | 38 | C | 32 | 0 | 1 |
| 3FA0 | 20 | 0 | D6 | B | E8 | F9 | C0 | A0 | 35 | 46 | 2 | B7 | 1 | 4D | 0 | 7 |
| 3FB0 | 52 | 45 | 51 | 33 | 46 | 39 | 36 | 40 | 0 | 8 | 27 | 52 | 45 | 14 | 3D | A4 |
| 3FC0 | 3F | 40 | 0 | B | 27 | 43 | 4F | B9 | 3F | A4 | 3F | 42 | 0 | 6 | 27 | 54 |
| 3FD0 | 52 | C3 | 3F | A4 | 3F | 43 | 0 | 5 | 27 | 50 | 4F | CD | 3F | A4 | 3F | 44 |
| 3FE0 | 0 | 5 | 27 | 44 | 41 | D7 | 3F | A4 | 3F | 46 | 0 | 4 | 27 | 41 | 4 | 54 |
| 3FF0 | 42 | 4C | 62 | 3B | 7 | 50 | 52 | 4F | 4E | 7 | 50 | 52 | 4F | 53 | 39 | 6F |

```
4008    3Z  0   1  20 EB 99 CO  0    36 46   2 B7   1 4D   0   7    LISTING NO. 2
4010    52 33  46 46 34 66 33 46    46 39  27 52 45 74 3D   4
4020    40 40   0   8 27 43 4F 19    40   4  40 42   0   6 27 54    Sheet 9
4030    52 23  40   4 40 43   0   5    27 50  4F 2D 40   4 40 44
4040     0   5  27 44 41   7 58 43    4F F1  3B A2 34 20 20 20
4050    40   4  40 48   0   4 27 53    45 48  40   4 40 4A   0   6
4060    27 48  42 55 40   4 40 4C     0   6  27 56 42 5F 40   4
4070    40 4D   0   4 27 45 59 69    40   4  40 4E   0   3 27 4F
4080    45 73  40   4 40 4F   0   6    27 41  56 7D 40   5 46 57
4090    47  F  3C A2 34 20 40   4    40 54   0   6 27 4C 4F 91
40A0    40   4  40 55   0   4 27 5A    45 9B  40   4 40 56   0   7
40B0    27 48  4D A5 40   4 40   5    41 54  45 FB 3B A2 34 20
40C0    40 58   0   4 27 41 4D B9    40   4  40 59   0   5 27 48
40D0    4D C3  40   4 40 5A   0   6    27 55  50 CD 40   4 40 5D
40E0     0   B  27 43 4F D7 40   4    40 5E   0   C 27 42 49 E1
40F0    40   4  40 5F   0   5 45 58    56 BC  3D 99   0 4A 4B 34
4100    4B 6A  4D 78 4D 92 4D AC    4D   5  23 41 4F EE 3F 66
4110     0   0   0   4 23 41 49   9    41 66   0   1   0   8 23 41
4120    55 13  41 66   0   2   0   4    23 41  43 1D 41 66   0   3
4130     8   9  23 45 59 27 41 66     0   4   0   A 23 45 59 31
4140    41 66   0   5   0   4 3F 54    49 48  39 3A   0 17   0   2
4150    93 2F  B8 2F  F   0 C2   0    B6 24  17   0   2 2E   1 61
4160     1 61   3 4D   8 54 50 52    CE 3A  8B 31   0   1 20 20
4170    93 2F  5A 36 B7   1 64 36    B7   1  77   1 D6   0   5 8B
4180     3 CC   0   A 95   3 5A 36    B7   1  64 36 C6   1 4D   0
4190     6 56  49 44 F5 40 3A   0    F6 35  46   2 B8 2F B7 2D
41A0     8 54  50 52   A 3B   C 32     0   1  20 20 B7   1 95  ·3
41B0    96 41  4D   0   4 56 52 45    90 41  3A   0 28 34 31 41
41C0    30   1  17   0   2 96 41 4D     0   4  53 4E 41 3B 41 3A
41D0     0 8B   3 17   0   3 96 41    4D   0   7 48 53 43 EB 40
41E0    3A   0  28   2 8C 36 C6   1    17   0   4 96 41 4D   0   7
41F0    56 53  43 B4 41 3A   0 2B     2 96  36 C6   1 17   8 54
4200    50 52  68   8 54 50 52 6D    3B 6F  32   0   1 20 20   0
4210     6 96  41 4D   0   6 56 42    4C EF  41 34 31 46 45   0
4220    34 32  30 33 36 4A 2F A8    41 4D   0   4 41 56 49   F
4230    40 3A   0 17   0   1 17   0     3 78  36 4A 2F A8 41 4D
4240     0   5  44 41 56 C9 41 3A     0 17   0   2 17   0   3 78
4250    36 4A  2F A8 41 4D   0   4    56 52  41 15 42 3A   0 17
4260     8   3  17   0   3 78 36 4A    2F A8  41 4D   0   4 21 56
4270    53 2B  42 3A   0 28   2 67     2 2E   1 46   2 78 36 4A
4280    2F A8  41 4D   0   4 4C 49    56 6A  3C 3A   0 17   0   4
4290    73 42  A8 41 4D   0   3 52    45 47  6D 42 3A   0 17   0
42A0     8 73  42 A8 41 4D   0   6    42 38  43 96 42 3A   0 17
42B0     8 10  73 42 A8 41 4D   0     6 42  39 43 A7 42 3A   7
42C0    50 52  4F 19 3C F0 34   0     1 20  43 52 41 41 42 3A
42D0     0 28   2 17   0   2 6D 22    3F   3  AD 42 17   0   4 6D
42E0    22 EB   2 3F   3 BE 42 4D     0   7  52 45 41 B8 42 3A
42F0     0 17   0 40 73 42 A8 41    4D   0   7 43 4F 4C C9 42
4300    3A   0   F   0 80   0 73 42    A8 41  4D   0   8 3F 4C 45
4310    65 41  3A   0 79 40 88 2D    61   1  4D   0 9C 50 51 1E
4320    E4 C2   D   2   0 E6 C2 89     0 80  8E D9 2B C0 A0   6
4330     0 8A  E0 A0   7   0 1F   3    6 24  34 89   6 5C 36 59
4340    58 9D  CF CF   4 54 52 2F    FA 42  3A   0   6   2 17   0
4350     0 17   0 20 67   2 17   0    20 F8   2 28   2 96 2A 56
4360     2  F   0 80   9 A9   1 D6     0   6  17   0 20 CC   4 54
4370    42 4C  64 41 66   0   0   0     9 41  43 4F 52 3D 3A   0
4380    5E   0  D6   8 E8 19 BD 6C    35 46   2 B7   1 4D   0   7
4390    52 45  51 78 43 66   0 40     0   8  27 52 45 E0 3C 84
43A0    43 40   0   8 27 43 4F 99    43 84   4 54 42 4C A0 41
43B0    66   0   0   0   9 41 43 4F    8E 3D  3A   0 5E   0 D6   B
43C0    E8 DD  BC A8 35 46   2 B7     1 4D   0   7 52 45 51 B4
43D0    43 66   0 40   0   8 27 52    45 1C  3D C0 43 40   0   8
43E0    27 43  4F D5 43 C0 43 42     0   6  27 54 52 DF 43 C0
43F0    43 43   0   5 27 50 4F E9    43 C0  43 44   0   5 27 44
```

```
4100   41 F3 43 C0 43 46  0   4     4 54 42 4C FE  4 54 42    LISTING NO. 2
4110   4C  3 42 66  0   0   0   9    41 43 4F F1 3D 3A  0 5E    Sheet 10
4120    0 06  B E8 7A BC  B 36    46  2 B7  1 4D  0  7 52
4130   45 51 17 44 66  0 40  0     8 27 52 45 7F 3D 23 44
4140   40  0  8 27 43 4F 38 44    23 44 42  0  6 27 54 52
4150   42 44 23 44 43  0  5 27    50 4F 4C 44 23 44 44  0
4160    5 27 44 41 56 44 23 44    46  0  4 27 41 44 60 44
4170   23 44 48  0  4 27 53 45    6A 44 23 44 4A  0  6 27
4180   48 42 74 44 23 44 4C  0     6 27 56 42 7E 44 23 44
4190   4D  0  4 27 45 59 88 44    23 44 4E  0  3 27 4F 45
44A0   92 44 23 44 4F  0  6 27    41 56 9C 44 23 44 52  0
44B0    6 27 57 48 A6 44 23 44    54  0  6 27 4C 4F B0 44
44C0   23 44 55  0  4 27 5A 45    BA  8 54 50 52 33 3E F0
44D0   34  0  1 20 20 44 57  0     5 27 56 45 CE 44 23 44
44E0   58  0  4 27 41 4D DB 44    23 44 59  0  5 27 48 4D
44F0   EZ 44 23 44 5A  0  6 27    55 50 EC 44 23 44 5D  0
4500    B 27 43 4F F6 44 23 44    5E  0  C 27 42 49  0 45
4510   23 44 5F  0  5 45 58 56    C7 30 99  0 69 4F 53 4F
4520   89 51 97 51 B1 51 CB 51     5 23 41 4F  D 44 66  0
4530    0  0  4 23 41 49 28 45    66  0  1  0  8 23 41 55
4540   32 45 66  0  2  0  4 23    41 43 3C 45 66  0  3  0
4550    9 23 45 59 46 45 66  0     4  0  A 23 45 59 50 45
4560   66  0  5  0  4 3F 54 49    F9 3F 3A  0 17  0  2 9E
4570   2F C3 2F  F  0 CZ  0 B6    24 17  0  2 2E  1 61  1
4580   61  3 4D  0  4 3F 48 49    64 45 3A  0 17  0  2 9E
4590   2F 65 36 B7  1 6F 36 B7     1 77  1 D6  0  5 88  3
45A0   CC  0  A 95  3 65 36 B7     1 6F 36 C6  1 4D  0  6
45B0   56 49 44 14 45 3A  0  1    36 46  2 C3 2F CZ ZD 4D
45C0    0  4 4D 4F 44 59 32 3A     0 83 36 B7  1 95  3 B5
45D0   45 4D  0  4 56 52 45 AF    45 3A  0 28  2 8D 36 C6
45E0    1 17  0  2 B5 45 4D  0     4 53 7E 41 5A 45 3A  0
45F0   8B  3 17  0  3 B5 45 4D     0  7 48 53 43  A 45 3A
4600    8 28  2 97 36 C6  1 17     0  4 B5 45 4D  0  7 56
4610   53 43 D3 45 3A  0 28  Z    A1 36 C6  1 17  0  5 B5
4620   45 4D  0  4 57 49 50 F9    45 3A  0 8B  3 17  0  6
4630   B5 45 4D  0  6 56 42 4C    E 46 3A  0 17  0  0 17
4640    8  3 83 36 55 2F C7 45    4D  0  4 41 56 49 2E 44
4650   3A  0 17  0  1 17  0  3    83 36 55 2F C7 45 4D  0
4660    5 44 41 56 EB 45 3A  0    17  0  2 17  0  3 83 36
4670   55 2F C7 45 4D  0  4 56    52 41 34 46 3A  0 17  0
4680    3 17  0  3 83 36 55 2F    C7 45 4D  0  4 21 56 53
4690   4A 46 3A  0 28  2 67  2    2E  1 46  2 83 36 55 2F
46A0   C7 45 4D  0  4 4C 49 56    75 3C 3A  0 17  0  4 92
46B0   46 C7 45 4D  0  3 52 45    47 8C 46 3A  0 17  0  8
46C0   92 46 C7 45 4D  0  6 42    38 43 B5 46 3A  0 17  0
46D0   10 92 46  4 54 42 4C C9    44 66  0  0  0  9 41 43
46E0   4F B7 40 3A  0 5E  0 D6     B E8 B4 B9 D1 38 46  Z
46F0   B7  1 4D  0  7 52 45 51    DD 46 66  0 40  0  8 27
4700   52 45 45 40 E9 46 40  0     8 27 43 4F FE 46 E9 46
4710   42  0  6 27 54 52  8 47    E9 46 43  0  5 27 50 4F
4720   12 47 E9 46 44  0  5 27    44 41 1C 47 E9 46 46  0
4730    4 27 41 44 26 47 E9 46    48  0  4 27 53 45 30 47
4740   E9 46 4A  0  6 27 48 42    3A 47 E9 46 4C  0  6 27
4750   56 42 44 47 E9 46 4D  0     4 27 45 59 4E 47 E9 46
4760   4E  0  3 27 4F 45 58 47    E9 46 4F  0  6 27 41 56
4770   62 47 E9 46 52  0  6 27    57 48 6C 47 E9 46 54  0
4780    6 27 4C 4F 76 47 E9 46    55  0  4 27 5A 45 80 47
4790   E9 46 56  0  7 27 48 4D    8A 47 E9 46 57  0  5 27
47A0   56 45 94 47 E9 46 58  0     4 27 41 4D 9E 47 E9 46
47B0   59  0  5 27 48 4D A8 47    E9 46 5A  0  6 27 55 50
47C0   BZ 47 E9 46 5D  0  B 27    43 4F BC 47 E9 46 5E  0
47D0    C 27 42 49 C6 47 E9 46    5F  0  5 45 58 56 8D 40
47E0   99  0 2F 52 19 52 4F 54    5D 54 77 54 91 54  5 23
47F0   41 4F D3 46 66  0  0  0     4 23 41 49 EE 47 66  0
```

```
4800    1    0    8   23   41   55   F8   47     66    0    2    0    4   23   41   43
4810    2   48   66    0    3    0    9   23     45   59    C   48   66    0    4    0
4820    A   23   45   59   16   48   66    0      5    0    4   3F   54   49   BF   42
4830   3A    0   17    0    2   1F   32   44     32    F    0   C2    0   B6   24   17
4840    0    2   2E    1   61    1   61    3     4D    0    4   3F   48   49   2A   48
4850   3A    0   17    0    2   1F   32   28     39   B7    1   35   39   B7    1   77
4860    1   D6    0    5   8B    3   CC    0      A   95    3   28   39   B7    1   35
4870   39   C6    1   4D    0    6   56   49     44   DA   47   3A    0   C7   38   46
4880    2   44   32   43   30   4D    0    4     4D   4F   44   DA   34   3A    0   49
4890   39   B7    1   95    3   7B   48   4D      0    4   56   52   45   75   48   3A
48A0    0   28    2   53   39   C6    1   17      0    2   78   48   4D    0    4   53
48B0   4E   41   20   48   3A    0   8B    3     17    0    3   7B   48   4D    0    7
48C0   48   53   43   D0   47   3A    0   28      2   5D   39   C6    1   17    0    4
48D0   7B   48   4D    0    7   56   53   43     99   48   3A    0   28    2   67   39
48E0   C6    1   17    0    5   7B   48   4D      0    4   57   49   50   BF   48   3A
48F0    0   8B    3   17    0    6   7B   48     4D    0    6   56   42   4C   D4   48
4900   3A    0   17    0    0   17    0    3     49   39   D6   31   8D   48   4D    0
4910    4   41   56   49   F4   46   3A    0     17    0    1   17    0    3   49   39
4920   D6   31   8D   48   4D    0    5   44     41   56   AE   48   3A    0   17    0
4930    2   17    0    3   49   39   D6   31     8D   48   4D    0    4   56   52   41
4940   FA   48   3A    0   17    0    3   17      0    3   49   39   D6   31   8D   48
4950   4D    0    4   21   56   53   10   49     3A    0   28    2   67    2   2E    1
4960   46    2   49   39   D6   31   8D   48     4D    0    4   4C   49   56   3B   3F
4970   3A    0   17    0    4   58   49   8D     48   4D    0    3   52   45   47   52
4980   49   3A    0   17    0    8   58   49     8D   48   4D    0    6   42   38   43
4990   7B   49   3A    0   17    0   10   58     49   8D   48   4D    0    6   42   39
49A0   43   8C   49   3A    0   17    0   20     58   49   8D   48   4D    0    9   43
49B0   52   41   26   49   3A    0   28    2     17    0    2   6D   22   3F    3   92
49C0   49   17    0    4   6D   22   E8    2     3F    3   A3   49   4D    0    7   52
49D0   45   41   9D   49   3A    0   17    0     40   58   49   8D   48   4D    0    7
49E0   43   4F   4C   AE   49   3A    0    F      0   80   0   58   49   8D   48   4D
49F0    0    8   3F   4C   45   4A   48   3A      0   5E   47   14   30   61    1   4D
4A00    9   9C   50   51   1E   E4   C2    D      2    0   E6   C2   B9    0   80   8E
4A10   D9   2B   C0   A0    6    0   8A   E0     A0    7    0   1F    3    6   F5   36
4A20   89    6   2D   39   59   58   9D   CF     CF    4   54   52   2F   DF   49   3A
4A30    0    6    2   17    0    0   17    0     20   67    2   17    0   20   F8    2
4A40   28    2   22   2D   56    2    F    0     80    9   A9    1   D6    0    6   17
4A50    0   20   CC    0    2   8B    3   7E     2D   B7    1   3F    1   3D    5    D
4A60   19   17    2   F2   2D   11   2F   3D      5   19   19   4D    0    5   54   52
4A70   41   29   4A   3A    0    4    6   67      2   67    2   2F   4A   38    2   4D
4A80    0    3   49   46   44   DD   38   3A      0   8B    3   C5   48   8B    3   DA
4A90   48   8B    3   17   39   C6    1   4D      0    7   46   3E   54   3C   49   3A
4AA0    0   7E   2D   B7    1   D6    0    E     89    2   17    0    8   F8    2   FD
4AB0   36   88    2   CC    0   22   28    2     17    0    9   A9    1   D6    0   11
4AC0   17    0    A   99    2   17    0    8     F8    2   FD   36   88    2   CC    0
4AD0    7   B9    2   17    0    8   F8    2     4D    0    3   46   3C   44   99   4A
4AE0   3A    0   74   2D   B7    1   46    2     9F   38    F    0    0   10   A9   22
4AF0   74   2D   C6    1   9F   4A   8B    3     17    0    8   8B    3   E2    0   56
4B00    2   17    0    8   2F   4A    F    0      0   20   88    2   46    2   AA    2
4B10   46    2   3D    5   2E   19   F1    0     E6   48    4   74   2D   C6    1   4D
4B20    0    3   46   3E   44   DA   4A   3A      0   74   2D   B7    1   46    2   9F
4B30   38    F    0    0   10   A9   22   74     2D   C6    1   9F   4A   8B    3   17
4B40    0    8   8B    3   E2    0   56    2     17    0    A   2F   4A    F    0    0
4B50   20   88    2   46    2   AA    2   46      2   3D    5   2E   19   F1    0   E6
4B60   48    4   74   2D   C6    1   4D    0      8   46   52   4F   21   4B   3A    0
4B70   E0   4A   4D    0    6   54   4F   44     6D   4A   3A    0   27   4B   4D    0
4B80    4   48   46   3C   E9   48   3A    0     87   4A   9F   4A   17    0    8   F8
4B90    2   46    2   61    3   61    3    F      0   80    0   F8    2   17   39   D5
4BA0    1   28    2   17    0   40   88    2     46    2   E2    0    F    0    0    4
4BB0   8B    3   E2    0   8A   18   F1    3     9E    A   79    2   88    2    F    0
4BC0   80    0   88    2   9F   38   17   39     B7    1    F    0   80    0   6E   30
4BD0    F    0    0    1   17   39   D5    1      F    0    0    1    7    1   D5   F1
4BE0    0   CA   4D    0    3   49   4E   5A     81   4A   3A    0   28    2   61    1
4BF0   D6    0    2   AA    2   4D    0    3     44   43   5A   74   4B   3A    0   28
```

LISTING NO. 2

Sheet 11

LISTING NO. 2

Sheet 12

```
4C00   Z 61  I D6   0  2 89   Z    4D  0   8 44 4F 47 F7 4B
4C10   3A  0 7E 2D B7  1  3 30    95  3 8E 20 17  0   8 56
4C20   Z 77  1 D6   0 15 46   2    89  2 FD 4B  7 37 6D 22
4C30   28  Z E0 4A 46  2 8B   3    CC  0 21 17  0  C 56  2.
4C40   77  1 D6   0 15 46   2 AA   2  7 37 6D 22 EA 4B 28
4C50   Z E0 4A 46  2 8B  3 CC    0  2 95  3 46  2 38   2
4C60   D6  0 B7 38   2 7E 2D C6    1 4D  0  5 53 48 41  A
4C70   4C 3A  0 9F 38 8B  3  F    0  0 80 17  0  4 8F 30
4C80   7B 35 9F 38  F  0  0 80  .  F  0  0 80 17  0  4 8F
4C90   30 7B 35 4D  0  2 58 40   20 80 4B 9D 4C 2B C0 8A
4CA0    6 19 39 58 AD 97 FF 25    2 59 40 20 E4 4B B0 4C
4CB0   2B C0 8A  6 1A 39 50 AD   97 FF 25  2 58 21 20 95
4CC0   4C C3 4C 58 88  6 19 39   AD 97 FF 25  2 59 21 20
4CD0   A8 4C D4 4C 58 88  6 1A   39 AD 97 FF Z5  2 56 40
4CE0   20 68 4B 3A  0  D 39 B7    1 17 39 B7  1 14 30 4D
4CF0    0  2 56 21 20 DD 4C 3A    0  D 39 B7  1 17 39 B7
4D00    1 43 30 4D  0  5 48 4C   49 BB 4C 3A  0 D2 4C E2
4D10    0 28  2 79  2 C1 4C F7   4C F1  0 F5 38  2 4D  0
4D20    5 56 4C 49 F1 4C 3A  0   C1 4C E2  0 28  2 79  2
4D30   D2 4C F7 4C F1  8 F5 38    2 4D  0  4 52 47 42 CE
4D40   49 3A  0  F  0  0  1 F8    Z 88  Z 28  2  F  0  0
4D50   18 88  2 E2  0 B3 38 79    2 43 30  F  0  0 F8  7
4D60    1 F3 4D  0  5 43 52 41   6B 4C 3A  0  F  0  0  1
4D70   8B  3 E2  0 79  2 28  2   35  4 17  0  5 8F 30 41
4D80   4D F1  0 F0 38  2 4D  0    8 43 52 41 64 4D 90 4D
4D90   59 5F 5A  6 53 8B D9 8E    6 B5 38 86 F2  3 D7 8B
4DA0   FA 8A  6 EF 3B F3 AA 8A    6 F9 3B 81 C2  0  8 8B
4DB0   FA 8B CB F3 AA 8A  6  3   3C 81 C2  0  8 8B FA 8B
4DC0   CB F3 AA 58  7 AD 97 FF   25  3 50 4D 58 F1 49 99
4DD0    0 8B 16 27 3A D1 EA 2B   DA D1 E2  3 D3 8A FA 8A
4DE0   F3 8A 16 31 3A 8B CF 8B   F2 26 AC 3C FF 75  6 FF
4DF0    6 F5 39 EB  6 3A E8 73    2 8A E0 E2 EC 80 C6  1
4E00   3A FE 75 EI C3  4 50 4D   41 C9 4D  D 4E  6 8E  6
4E10   A1 38 56 53 2B C0 89  6   F5 39 8B 16 31 3A 8B  E
4E20   3B 3A 2B CA 8B F9 8B 1E   13 3A E8 A4 FF 8B 1E 1D
4E30   3A E8 9D FF 5B 5E  7 2B   D2 8A D4 52 AD 97 FF 25
4E40    7 49 4E 52 CC 4C 3A  0   28  2  F  0 FE  0 9B  1
4E50   46  2 DF 39 B7  1 E9 39   B7  1 99  2 A9  1 2E  1
4E60   4D  0  7 41 44 4A 3B 4D   3A  0 44 32  F  0 FE  0
4E70   9F 48 B4 48 44 32  B 4E   28  2  F  0 FF  0 DF 39
4E80   B7  1 95 22 28  2 67  2    F  0 FF  0 67  2 95 22
4E90   DF 39 B7  1 E9 39 B7  1   88  2 A9  1 D6  0  2 AA
4EA0    2 28  2  F  0 FE  0 A9    1 D6  0  6 38  2  F  0
4EB0   FE  0 9F 48 4D  0  8 46   4F 4F 20 4D 3A  0  B 4E
4EC0   46 4E 61  3 F3 39 B7  1   FD 39 B7  1 A9  1 3F  1
4ED0   4D  0  7 41 47 43 62 4E   3A  0 17  8 10  A 32 B4
4EE0   48 44 32 44 32 BC 4E D6    0  2 68 4E 6D 32 D6  0
4EF0   EE 17  0 10 1F 32 BC 4E   28  2  7 3A C6  1 D6  0
4F00   4B  F  0 80  0 8B  3 E2    0 EB 30 F1  0 FB E4  5
4F10   15  8 16 4F 75 74 20 6F   66 20 72 61 6E 67 65 2E
4F20   20 20 50 4D 41 58 20 3D   20  8 4E 35  7 17  8  3
4F30   6C  6 F3 39 45  7 15  8   11 70 69 78 65 6C 73 20
4F40   73 61 74 75 72 61 74 65   64 20 E4  5 4D  0  4 4F
4F50   41 44  5 4E 99  0 45 20    3 4F 43 58 4E 4F 99  0
4F60   40  0  3 4F 43 59 58 4F   99  0 40  0  3 4F 58 40
4F70   62 4F 74 4F 2B C0 8A  6   56 4F 50 AD 97 FF 25  3
4F80   4F 59 40 6C 4F 87 4F 2B   C0 8A  6 57 4F 50 AD 97
4F90   FF 25  3 4F 58 21 7F 4F   9A 4F 58 88  6 56 4F AD
4FA0   97 FF 25  3 4F 59 21 92   4F A8 4F 58 88  6 57 4F
4FB0   AD 97 FF 25  2 4F 21 20   A3 4F 3A  0 A9 38 54 4F
4FC0   B7  1 43 30 4D  0  2 2B   3E 20 6A 49 3A  0 9F 38
4FD0   68 4F B7  1  F  0  0  1   F8  2 9F 38 17  0  0  F
4FE0    0 80  0 6E 30 5E 4F B7    1 C1 4C  F  0 E0  0 8B
4FF0    3 E2  0 79  2 D2 4C E3   4C 9F 38  F  0  0  1 79
```

— 59 —

```
5000   Z 88  2 43 30 F1  0 EB    4D  0  2 3E 2B 20 87 48
5010  3A  0 9F 38 17  0  0 9F    38 68 4F B7  1  F  0  0
5020   1 F8  2  F  0 80  0 6E    30 5E 4F B7  1 C1 4C  F
5030   0 E0  0 8B  3 E2  0 9F    38  F  0  0  1 79  2 88
5040   2 14 30 79  2 D2 4C F7    4C F1  0 EB 4D  0  3 2B
5050  3E 2B C6 4F 3A  0 9F 38    68 4F B7  1  F  0  0  1
5060  F8  2 28  2  F  0 80  0    88  2 46  2 9F 38 46  2
5070   F  0 80  0 6E 30  F  0    E0  0 88  3 E2  0 5E 4F
5080  B7  1  F  0 80  0 88  2    C1 4C 79  2 D2 4C E3 4C
5090  5E 4F B7  1 C1 4C F7 4C    F1  0 E3 4D  0  6 4F 48
50A0  4C B4 4F 3A  0 A9 4F E2     0 28  2 79  2 98 4F BA
50B0  4F F1  0 F5 38  2 4D  0     6 4F 56 4C 9D 50 3A  0
50C0  98 4F E2  0 28  2 79  2    A9 4F BA 4F F1  0 F5 38
50D0   2 4D  0  7 2B 4F 48 4E    50 3A  0 95  3  F  0 80
50E0   0 17  0  0 68 4F B7  1    A3 50 10 50 8B  3  F  0
50F0   0  1  F  0 80  0 68 4F    B7  1 A3 50 68 4F D5  1
5100  8B  3  F  0 80  0 17  0     0 68 4F B7  1 A3 50 8B
5110   3  F  0 E0  0 17  0  0    5E 4F B7  1  F  0 80  0
5120  6D 22 BE 50 95  3  F  0    FF  0  F  0 80  0 68 4F
5130  B7  1 A3 50 95  3  F  0    E0  0 17  0  0 5E 4F B7
5140   1  F  0 80  0 88  2 BE    50 CC 4F 54 50 8B  3 4D
5150   0  7 2B 4F 56 D3 50 3A     0 95  3  F  0 E0  0 8B
5160   3 5E 4F B7  1 BE 50 10    50 8B  3  F  0 E0  0 8B
5170   3 5E 4F B7  1  F  0 80     0 88  2 BE 50 5E 4F D5
5180   1 8B  3  F  0 E0  0 8B     3 5E 4F B7  1 BE 50 8B
5190   3  F  0 80  0 88  3 68    4F B7  1 A3 50 95  3 · F
51A0   0 FF  0  F · 0 80  0 68    4F B7  1 A3 50 95  3  F
51B0   0 E0  0 17  0  0 5E 4F    B7  1  F  0 80  0 88  2
51C0  BE 50 CC 4F 54 50 8B  3    4D  0  8 21 52 45 D2 4E
51D0  3A  0  F  0 5A A5  4 47    57 30 4D  0  8 40 52 45
51E0  BB 50 3A  0  4 47 2B 30    4D  0  7 54 49 4C 88 4D
51F0  3A  0 E2 51  F  0 A5 5A    77  1 06  0 F5 4D  0  5
5200  49 4F 52 40 4E 3A  0  E    47 43 30 D0 51 F0 51 4D
5210   0  3 41 49 4E CA 51 3A     0 22 47 43 30 FE 47  5
5220  52 2C 47 11 30 4D  0  4    41 4F 55 11 52 3A  0 22
5230  47 43 30 2C 47 43 30 F4    47  5 52 4D  0  5 50 42
5240  4F DC 51 3A  0 44 32 17     0  2 2D 52 4D  0  5 50
5250  43 4F 3D 52 3A  0 17  0     4 2D 52 4D  0  5 41 57
5260  49 27 52 3A  0 BB  3 54    52 4D  0  5 41 53 4E 5D
5270  52 3A  0 95  3 54 52 4D     0  5 41 56 52 6B 52 3A
5280   0 28  2 72 47 43 30 43    52 17  0  2 54 52 4D  0
5290   5 57 48 45  5 4D 3A  0    28  2 7C 47 43 30 43 52
52A0  17  0  8 54 52 4D  0  3    5A 45 45 FF 51 3A  0 28
52B0   2 90 47 43 30 43 52 17     0  A 54 52 4D  0  4 48
52C0  4D 49 90 52 3A  0 28  2    9A 47 43 30 43 52 17  0
52D0   B 54 52 4D  0  4 56 45    52 B6 4E 3A  0 28  2 A4
52E0  47 43 30 43 52 17  0 20    54 52 4D  0  3 41 4D 41
52F0  79 52 3A  0 28  2 AE 47    43 30 43 52 17  0 21 54
5300  52 4D  0  4 48 4D 41 BE    52 3A  0 28  2 88 47 43
5310  30 43 52 17  0 22 54 52    4D  0  5 4C 4F 57 51 51
5320  3A  0 28  2 86 47 43 30    43 52 17  0  9 54 52 4D
5330   0  5 55 50 50 D5 52 3A     0 28  2 C2 47 43 30 43
5340  52 17  0 29 54 52 4D  0     9 57 52 49  3 53 3A  0
5350  CC 47 EE 31 CC 47 14 30    43 52 17  0 2A 54 52 4D
5360   0  7 43 41 4C EA 51 3A     0 95  3 4E 53 4D  0  6
5370  4D 4F 54  A 50 3A  0 17     0  2 4E 53 4D  0  8 4D
5380  41 49 6F 53 3A  0 17  0     4 4E 53 4D  0  8 46 41
5390  43 31 53 3A  0 17  0  8    4E 53 4D  0  7 41 43 41
53A0  EC 52 3A  0 17  0 20 4E    53 4D  0  9 53 49 54 61
53B0  53 3A  0 17  0 40 4E 53    4D  0  7 53 54 41 AB 53
53C0  3A  0  F  0 FF  0 50  1     F  0 80  0 4E 53 4D  0
53D0   7 57 52 49 48 53 3A  0    D6 47 EE 31 D6 47 14 30
53E0  43 52 17  0 2B 54 52 4D     0  6 4D 49 52 7E 53 3A
53F0   0  F  0 FF  0 50  1 17     0  4 D6 53 4D  0  7 43
```

```
5400   48 4F BA 53 3A  0  F  0    FF  0 50  1 17  0  4 D6
5410   53 4D  0  7 53 54 52 FE    53 3A  0 17  0  8 D6 53
5420   4D  0  A 43 41 4C 13 54    3A  0 17  0 10 D6 53 4D
5430    0  6 4D 41 4E E9 53 3A     0  F  0 FF  0 50  1  F
5440    0 80  0 D6 53 4D  0  7    41 55 54 9C 53 3A  0  8
5450   48  5 52 4D  0  3 41 43    40 47 54 3A  0 36 47 57
5460   30 40 47 57 30 12 48  5    52 2C 47 14 30 4D  0  8
5470   45 59 45 8D 53 3A  0 8B     3 4A 47 43 30 8B  3 54
5480   47 43 30 1C 48  5 52 4D     0  9 45 59 45 6F 54 3A
5490    0 26 48  5 52 4D  0  6    4F 43 52 4E 52 3A  0 35
54A0    4  6  2  6  2 67  2 28     2 A4 21 46  2 17  0  8
54B0   88  2 28  2 17  0 18 99     2 67  2 A3 50 17  2 17
54C0    2 17  0  8 88  2 28  2    17  0 18 99  2 67  2 BE
54D0   50 4D  0  5 5B 59 45 D0    53 3A  0  4 47 17  0 20
54E0   88  2 14 30 17  0 40 88     2 B7 39 B7  1 88  2  4
54F0   47 17  0 22 88  2 14 30    17  0 40 88  2 C1 39 B7
5500    1 88  2 4D  0  3 41 49    4D 55 54 3A  0 D9 54 35
5510    4 93 3A B7  1 77  1 46     2 75 3A B7  1 77  1 2E
5520    1 61  1 D6  0 25 44 32    88  3 75 3A B7  1 93 3A
5530   B7  1 9D 54 35  4 93 3A    C6  1 75 3A C6  1 95  3
5540   67  2 67  2 44 32 9D 54    CC  8  2 48  4 40  0  2
5550   4E 4D 20 31 54 3A  0 17     0  9 17  0 17 95 22 17
5560    0 1F 99  2 4D  0  4 41    46 49  5 55 3A  0 17  0
5570   14 37 53 8B  3 20 53 4D     0  4 44 46 49 22 54 3A
5580    0 F7 49 D6  0  A 17  0    78  F  0 D6  0 CC  0  8
5590   F. 0 84  0  F  0 B8  0    37 53 20 53 4D  0  9 4D.
55A0   4F 54 4F 55 3A  0 6C 55     F  0 30  2 55 55 96 52
55B0    F  0 9A  0 AD 52  F -0    88  0 C4 52  F  0 80  0
55C0   DB 52  F  0 C0  0 F2 52    17  0 32  9 53  F  0 9B
55D0    0 7F 52 4D  0  4 48 4F    4D D3 54 3A  0 2F 37 37
55E0   54 A4 55 2F 37 75 53 2F    37 C0 53 2F 37 81 53 2F
55F0   37 A2 53 2F 37  4 54 EF    48 16 49 17  0  4 6A 4D
5600    F  0 FF  0  F  0 FF  0     F  0 FF  0 17  0  0 17
5610    8  0 41 4D 4D 37 84 53    4D  0  3 4E 4F 50 9E 55
5620   3A  0 4D  0  2 3A 4B 20    A7 52 3A  0  4  6 17  0
5630    8 88  2 46  2 75 37 C6     1 26  0 4D  0  3 3A 4B
5640   43 24 56 3A  0  4  6 17     0  8 88  2 46  2 75 37
5650   28  2  6  2 C6  1 17  2    46  2 87 38 C6  1 26  0
5660   4D  0  6 2B 43 59 1A 53    3A  0 95 38 B7  1 AA  2
5670   7D 38 6D 22 2B  2 95 38    C6  1 87 38 B7  1 B7  1
5680   9E  5 4D  0  A 4D 4F 4E    1A 56 3A  0 15  8  3 28
5690   4F 4B E4  5 17  0 10 1F    32 61  1 4D  0  6 24 53
56A0   50 79 55 3A  0 68 56 4D     0  3 24 43 52 9D 56 3A
56B0    0 8A 56 4D  0  4 53 41    56 A9 56 3A  0 CB 39 B7
56C0    1 28  2  7 37 B9  2 A9     1 46  2 11 37 AA  2 9B
56D0    1 2E  1 D6  0 45 E4  5    CB 39 B7  1 28  2 15  8
56E0   10 53 61 76 69 6E 67 20    64 61 74 61 20 73 65 74
56F0   20 35  7 15  8  4 2D 20    2D 20 27 4B 17  0 28 88
5700    3 E2  0 EB 30 F1  0 FB    15  8  7 20 64 6F 6E 65
5710   2E 20 95  3 CB 39 D5  1    CC  0 3A E4  5 EB 30 15
5720    8 2D 44 69 73 6B 20 66    75 6C 6C 2E 20 20 49 6E
5730   73 65 72 74 20 61 6E 6F    74 68 65 72 20 64 69 73
5740   6B 20 74 6F 20 63 6F 6E    74 69 6E 75 65 2E 20  7
5750   37 CB 39 C6  1 4D  0  2    24 43 20 B5 56 3A  0 2F
5760   37 70 49 BB  3 95 38 C6     1 4D  0  2 24 41 20 57
5770   57 3A  0 4D 37 70 49 D8    4E 95  3 95 38 C6  1 4D
5780    0  2 24 53 20 6B 57 3A     0  7 3A B7  1 61  3 D6
5790    0  2 BB 56 17  0  2 95    38 C6  1 4D  0  2 24 52
57A0   20 81 57 3A  0  F  0 80     2 55 55 96 52 4D  0  2
57B0   24 59 20 9D 57 3A  0  F     0 44  2 55 55 96 52 4D
57C0    0  2 24 47 20 AF 57 3A     0  F  0 30  2 55 55 96
57D0   52 4D  0  2 24 42 20 C1    57 3A  0  F  0 AE  1 55
57E0   55 96 52 4D  0  2 4F 50    20 97 54 99  0 45 20  6
57F0   3F 52 41 E5 57 3A  0  6     2 56  2 9B  1 17  2 67
```

```
5800    Z A9  1 3F  1 61  3 4D     0  6 43 48 41 D3 57 3A     LISTING NO. 2
5810    0 14 30 28  2 67  2 88     2 28  2  F  0 FF  0 88     Sheet 15
5820    3 F5 57 D6  0  2 46  2    38  2 4D  0  6 2B 41 56
5830   62 56 3A  0 72 47  F 58    7F 52 4D  0  6 28 57 48
5840   2C 58 3A  0 7C 47  F 58    96 52 4D  0  6 2B 4C 4F
5850   3C 58 3A  0 86 47  F 58    20 53 4D  0  4 28 5A 45
5860   4C 58 3A  0 90 47  F 58    AD 52 4D  0  5 2B 56 45
5870   5C 58 3A  0 A4 47  F 58    DB 52 4D  0  4 28 41 4D
5880   6C 58 3A  0 AE 47  F 58    F2 52 4D  0  5 2B 48 4D
5890   7C 58 3A  0 B8 47  F 58     9 53 4D  0  6 2B 55 50
58A0   8C 58 3A  0 C2 47  F 58    37 53 4D  0  6 2B 48 42
58B0   9C 58 3A  0 4A 47 14 30    88  2 4A 47 43 30 4D  0
58C0    6 2B 56 42 AC 58 3A  0    54 47 14 30 88  2 54 47
58D0   43 30 4D  0  2 24 32 20     9 58 3A  0 95  3 A2 58
58E0   4D  0  2 24 34 20 D4 58    3A  0 95  3 52 58 4D  0
58F0    2 24 36 20 E2 58 3A  0    17  0 FF 52 58 4D  0  2
5900   24 38 20 F0 58 3A  0 17     0 FF A2 58 4D  0  5 24
5910   44 4F FF 58 3A  0 17  0    FF 72 58 4D  0  3 24 55
5920   50  E 59 3A  0 95  3 72    58 4D  0  5 24 4C 45 1D
5930   59 3A  0 95  3 92 58 4D     0  6 24 52 49 2B 59 3A
5940    0 17  0 FF 92 58 4D  0     3 24 2F 5A 39 59 3A  0
5950   95  3 C6 58 4D  0  3 24    2F 57 48 59 3A  0 17  0
5960   FF C6 58 4D  0  3 24 2F    41 56 59 3A  0 95  3 B2
5970   58 4D  0  3 24 2F 44 65    59 3A  0 17  0 FF B2 58
5980   4D  0  2 24 37 20 73 59    3A  0 17  0 FF 42 58 4D
5990    0  2 24 39 20 82 59 3A     0 95  3 42 58 4D  0  2
59A0   24 31 20 91 59 3A  0 17     0 FF 62 58 4D  0  2 24
59B0   33 20 9F 59 3A  0 95  3    62 58 4D  0  2 24 2C 20
59C0   AE 59 3A  0 17  0 FF 82    58 4D  0  2 24 2E 20 BC
59D0   59 3A  0 95  3 82 58 4D     0  2 24 3C 20 CB 59 3A
59E0    0 17  0 FF 32 58 4D  0     2 24 3E 20 D9 59 3A  0
59F0   95  3 32 58 4D  0  2 24    49 20 EB 59 3A  0 39 37
5A00   19 54 4D  0  2 24 4F 20    F6 59 3A  0 57 37 19 54
5A10   4D  0  2 24 44 20  4 5A    3A  0 2C 49 4D  0  2 24
5A20   46 20 12 5A 3A  0 16 49    4D  0  7 4D 4F 4E 84 56
5A30   3A  0 8B  3 28  2 17  0    10  A 32 6D 32 D6  0 11
5A40    F  0 D8  0 B6 24 17  0    10 1F 32 75 37 B7  1 9E
5A50    5  B 55 D6  0 DE 38  2    4D  0  3 4D 41 4E 2A 5A
5A60   3A  0 16 49 4D 37 70 49    6C 55 2F 37 93 53 2F 37
5A70   84 53 9F 38 8B  3 17  0    FF  F  0 FF  0 7B 35  F
5A80    0 FF  0 17  0  0 17  0     0 17  0  0 17  0  0 41
5A90   4D 17  0  2 C1 39 C6  1    17  0 F9 B7 39 C6  1 8B
5AA0    3  F  0 FF  0 8B  3  F     0 80  0 C1 39 B7  1 88
5AB0    2  B 4D 8B  3  F  0 FF     0 8B  3  F  0 80  0 B7
5AC0   39 B7  1 88  2 26 4D 30    5A 4D 37 93 53 4D  0  4
5AD0   44 41 56 1E 5A 3A  0 4D    37 93 53 2C 49 7F 55 9F
5AE0   38 8B  3 17  0 FF 17  0    40 7B 35  F  0 FF  0 17
5AF0    0  0 17  0  0 17  0  0    17  0  0 41 4D 17  0 FE
5B00   C1 39 C6  1 8B  3 B7 39    C6  1 88  3  F  0 FF  0
5B10   8B  3  F  0 80  0 C1 39    B7  1 88  2  B 4D 8B  3
5B20    F  0 FF  0 8B  3  F  0    80  0 B7 39 B7  1 88  2
5B30   26 4D 4D 37 68 47 43 30    88  3 ED 3B C6  1 8B  3
5B40   F7 38 C6  1  F  0 FF  0     1 3C C6  1 17  0  4 8B
5B50    3  F  0  0  1 8E 4D 75    30 5A 8F 54 4D  0  4
5B60   45 58 41 89 54 3A  0  3    30 E0  D EF 48 8B  3 6A
5B70   4D 17  0  4 6A 4D 2F 37    B1 53 4D 37 C0 53 42 49
5B80   2F 37 70 49 2F 37 68 47    43 30 75 54 E4  5 E4  5
5B90   15  8 27 50 72 65 73 73    20 20 27 59 27 20 20 74
5BA0   6F 20 73 74 6F 72 65 20    74 68 65 20 6E 65 78 74
5BB0   20 66 72 61 6D 65 20 69    6E 20 CB 39 45  7 E4  5
5BC0   15  8 17 20 20 20 20 20    20 20 27 4E 27 20 20 6F
5BD0   74 68 65 72 77 69 73 65    2E 20 8E 20 17  0 59 77
5BE0    1 61  3 D6  0 1B E4  5    15  8  8 46 72 61 6D 65
5BF0   20 3D 20 11 37 AA  2  7    37 B9  2 26 31 CB 39 C6
```

```
5C00    1 E4  5 8B  3 95 38 C6     1 30 5A 8F 54 4D 37 68
5C10   47 43 30 2F 37 37 54 2F    37 C0 53 4D 37 84 53 F3
5C20   2F 4D  0  6 4E 4F 52 5A    5A 22 35  4  0  C  0 1A
5C30    0 34  0 5C  0 90  0 C5     0 EF  0 FF  0 EF  0 C5
5C40    0 90  0 5C  0 34  0 1A     0  C  0  4  0  4 4C 53
5C50   41 C0 58 3A  0  F  0  0     1 F8  2 9F 38 46  2 8A
5C60   18 8B  3 29 3C  F  0  0     1 6E 38 4D  0  5 53 51
5C70   53 CF 5A 75 5C 59 58 53    56 8B F0 BA  0  0 8B DA
5C80   8B FA 2B C0 AC  3 D0 F6    E0  3 D8 83 D7  0 E2 F2
5C90   8B C3 5E 5B 52 50 57 AD    97 FF 25  6 50 52 44 EF
5CA0   57 A3 5C 59 5F 5A 53 55    56 8B F2 BB  0  0 8B EB
5CB0   2B C0 8A 15 47 AC F6 E2     3 D8 83 D5  0 E2 F1 8B
5CC0   C3 8B CD 5E 5D 5B 50 51    AD 97 FF 25  7 4E 50 52
5CD0   23 5C D4 5C 59 5F 5A 53    55 56 8B F2 BB  0  0 8B
5CE0   EB AD F7 2D 83 C7  2  3    D8 13 EA E2 F4 8B C3 8B
5CF0   CD 5E 5D 5B 58 51 AD 97    FF 25  7 52 53 51 66 55
5D00   3A  0 FB 3A 12  4 43 3A    B7  1 CD 22 CF 3A B7  1
5D10   D9 3A B7  1 57 3A B7  1    95 22 99  2 28  2 28  2
5D20   E3 3A 12  4 43 3A B7  1    CD 22 CF 3A B7  1 28  2
5D30   BB 22 57 3A B7  1 CD 22    99  2 95 22  7 3B B7  1
5D40   EF 3A 12  4 43 3A B7  1    CD 22 D9 3A B7  1 28  2
5D50   BB 22 57 3A B7  1 CD 22    99  2 95 22 46  2 E6 32
5D60   4D  0  A 58 43 4F D5 55    3A  0 A7 3A B7  1 43 3A
5D70   B7  1 73 5C E3 3A 24  4    CF 3A C6  1 4D 3A B7  1
5D80   8B  3 E2  0 B1 3A B7  1    79  2 88  2 43 3A B7  1
5D90   73 5C EF 3A 24  4 D9 3A    C6  1 A7 3A B7  1 B1 3A
5DA0   B7  1 79  2 88  2 43 3A    B7  1 A1 5C FB 3A 24  4
5DB0    0 5D 79  2 4B 40 C6  1    F1  0 C9 4D  0  4 50 45
5DC0   41 9B 5C 3A  0  F  0  1    80 C5 3A C6  1 8B  3 BB
5DD0   3A C6  1 D8  2 56  2 88     2 46  2 E2  0 C5 3A B7
5DE0    1 D6 32 79  2 87  1 D6    32 18 22 D6  0  E 79  2
5DF0   B7  1 C5 3A C6  1 79  2    BB 3A C6  1 17  0  2  7
5E00    1 DB BB 3A B7  1 4D  0     9 49 4E 54 3D 56 3A  0
5E10   28  2 17  0  2 99  2 56     2 17  0  2 88  2 B7  1
5E20   67  2 B7  1 67  2 B7  1    56  2 46  2 99  2 28  2
5E30    6  2 67  2 67  2 46  2    99  2 88  2 17  2 46  2
5E40   4D  0  A 53 51 55 6D 5C    3A  0 11 38 B7  1 8B  3
5E50   E2  0 95  3 F1  0 FB  F     0 95 40 11 3B B7  1 E4
5E60   2C 11 3B B7  1 28  2 25    3B C6  1 E8  2 D8  2 1B
5E70   3B C6  1 4D  0  4 46 4C    54 5F 5B 3A  0 1B 3B B7
5E80    1 99  2  F  0 95 40 11    3B B7  1 D2 5C 25 3B B7
5E90    1 CD 22 4D  0  6 46 49    4C 75 5E 3A  0 11 3B B7
5EA0    1 E8  2 D8  2 1B 3B C6     1  6  2 46  2 17  2 D8
5EB0    2 8B  3 E2  0 35  4 79     2 88  2 7B 5E 46  2 79
5EC0    2 88  2 C6  1 17  0  2     7  1 EA 48  4 4D  0  B
5ED0   41 4C 54 FA 5C 3A  0 53    5C 68 5D 8B  3 4B 40 1B
5EE0   3B B7  1 8B  2 8B  3 4B    48 39 3B B7  1 9B 5E 8B
5EF0    3 4B 40 39 3B B7  1 C3    5D 28  2 B7  1 46  2 28
5F00    2 8B  3 4B 40 99  2 E8     2 8B  3 56  2 9B  1 56
5F10    2 39 3B B7  1 B9  2 9B     1 2E  1 D6  0  F B9  2
5F20   2F 3B B7  1 F8  2 46  2    E 5E CC  0 1D 46  2 38
5F30    2 8B  3 56  2 77  1 D6     0  B 8B  3 2F 3B B7  1
5F40   D8  2 CC  0  5 95  3 17     0  2 2F 3B B7  1 D8  2
5F50   46  2 95 22 88  2 4D  0     8 46 49 4C 95 5E 3A  0
5F60   B9  2 43 3B C6  1 43 3B    B7  1 8B  3 C5 42 2F 3A
5F70   B7  1 43 3B B7  1 2F 3A    B7  1 C5 42 B7  1 A5 2C
5F80   43 3B B7  1 39 3A B7  1    C5 42  F  0  0  1 39 3A
5F90   B7  1 99  2 43 3B B7  1    39 3A B7  1 C5 42 17  0
5FA0    2 99  2 B7  1 A5 2C 4D     0  9 53 45 54 42 5E 3A
5FB0    0  F  0 6B  3 56  2 9B     1 D6  0  9 95  3 11 3B
5FC0   C6  1 CC  0 84  F  0 EE     2 56  2 9B  1 D6  0  A
5FD0   17  0  3 11 3B C6  1 CC     0 6F  F  0 71  2 56  2
5FE0   9B  1 D6  0  A 17  0  5    11 3B C6  1 CC  0 5A  F
5FF0    0 F4  1 56  2 9B  1 D6     0  A 17  0  7 11 3B C6
```

LISTING NO. 2

Sheet 17

```
6000    1 CC   0 45   F   0 77   1     56   2 9B   1 D6   0   A 17
6010    0  9  11 3B  C6   1 CC   0     30   F   0 FA   0 56   2 9B
6020    1 D6   0   A 17   0   B 11     3B C6   1 CC   0 1B 17   0
6030   7D 56   2 9B   1 D6   0   A     17   0   D 11 3B C6   1 CC
6040    0  7  17   0   F 11 3B C6      1 38   2 48 5E 4D   0   8
6050   50 58  46 BD 5D 3A   0 B9       2 43 3B C6   1 43 3B 87
6060    1 AA   2 5E 5F 43 3B B7        1 8B   3 C5 42 43 3B B7
6070    1 2F 3A B7   1 C5 42 39       3A B7   1 2F 3A B7   1 99
6080    2 D8   2 8B   3 E2   0 43     3B B7   1 2F 3A B7   1 79
6090    2 E8   2 8J   2 CF 44 B7       1 AF 5F 35   4 79   2 88
60A0    2 7B 5E 46   2 79   2 88       2 C6   1 17   0   2   7   1
60B0   D6 48   4 43 3B B7   1 8B       3 C5 42 43 3B B7   1 2F
60C0   3A B7   1 C5 42 39 3A B7       1 2F 3A B7   1 99   2 D8
60D0    2 74   E 4D   0   4 4C 53     55 4D 5C 99   0 45 20   4
60E0   4C 43 4E D5 60 99   0 45       20   4 4C 4D 41 DF 60 99
60F0    0 45 20   5 54 52 55 A9       5F 99   0 F0   0   7 41 56
6100   4D CF 5E 3A   0 8B   3 EF      60 C6   1 8B   3 DB 60 C6
6110    1 8B   3 E5 60 C6   1 56       2 88   2 46   2 E2   0 79
6120    2 29 3C E5   1 F9 60 B7        1 9B   1 D6   0 38 79   2
6130   29 3C E5   1 EF 60 B7   1      35   4 77   1 D6   0   C 79
6140    2 DB 60 D5   1 95   3 E5      60 D5   1 A9   1 D6   0 16
6150   79   2 29 3C E5   1 EF 60      F7   1 79   2 DB 60 C6   1
6160   95   3 E5 60 C6   1 F1   0     B6 4D   0   5 4C 43 41 E9
6170   60 3A   0 53 5C   3 61 DB      60 B7   1 E5 60 B7   1 A9
6180   22 4D   0   7 4C 55 4D 6B      61 3A   0 39 3A B7   1 2F
6190   3A B7   1 E2   0 35   4 79      2 71 61 79   2 31 3D F7.
61A0    1 F1   0 F1 48   4 4D   0      5 50 50 4C 4F 60 3A   0
61B0   B9   2 43 3B C6   1 39 3A      B7   1 2F 3A B7   1 E2   0
61C0   43 3B B7   1 79   2 C5 42      B7   1 87 39 B7   1 88   2
61D0   C1 4C 79   2 D2 4C AD 39      B7   1 F7 4C F1   0 E1 4D
61E0    0  9 46 49 4E 58 5F 3A       0 17   0 20 17   0 40 89
61F0   61 8B   3 31 3D 2F 3A B7       1 88   2 39 3A B7   1 2F
6200   3A B7   1 99   2 54 35 11      3A C6   1   F   0 A0   0 17
6210    0 40 89 61 8B   3 31 3D      2F 3A B7   1 88   2 39 3A
6220   B7   1 2F 3A B7   1 99   2     54 35 1B 3A C6   1 4D   3
6230    8 49 57 49   8 5E 3A   0     8B   3 29 3C 11 3A B7   1
6240   88   2 43 3A B7   1 E8   2     99   2 A7 3A C6   1 8B   3
6250   29 3C 1B 3A B7   1 88   2     43 3A B7   1 4D 3A B7   1
6260   88   2 E8   2 99   2 B1 3A     C6   1 43 3A B7   1 28   2
6270   F8   2 57 3A C6   1 4D   0      A 4E 4F 52 CC 5C 3A   0
6280   88   3 25 3B C6   1 11 3B     B7   1 E8   2 D8   2 1B 3B
6290   C6   1 8B   3 25 3B C6   1     11 3B B7   1 8B   3 E2   0
62A0   79   2 29 5C 28   2 25 3B     D5   1 79   2 93 40 C6   1
62B0   F1   0 ED 4D   0   4 43 50     52 F3 60 3A   0 17   0 11
62C0   11 3B C6   1 7E 62 4D 3A      B7   1 1B 3B B7   1 99   2
62D0   39 3B C6   1 B9   2 28   2     28   2 43 3B C6   1 8B   3
62E0   C5 42   F   0   0   2 9F   3     8B   3 CF 44   F   0   0   2
62F0   9F   3 2F 3A B7   1 D5 5E     75 3A C6   1 38   2 39 3A
6300   B7   1 2F 3A B7   1 E2   0     79   2 D5 5E 46   2 43 3B
6310   B7   1 79   2 CF 44 C6   1     28   2 43 3B B7   1 79   2
6320   C5 42 C6   1 C1 4C 79   2     D2 4C E3 4C AD 39 B7   1
6330   F7 4C F1   0 D3 2F 3A B7       1 39 3A B7   1 B9   2 E2
6340    0 43 3B B7   1 79   2 C5     42 B7   1 C1 4C 79   2 D2
6350   4C F7 4C 17   0 FF   7   1     EB 43 3B B7   1 8B   3 C5
6360   42 8B   3 BD 40   F   0   0      2 1D   3 43 3B B7   1 AA
6370    2 55 60 43 3B B7   1 AA       2 AE 61 8B   3 BD 40 43
6380   3B B7   1 8B   3 C5 42   F      0   0   2 1D   3 4D   0   4
6390   58 52 45 62 5D 66   0 99       0   4 59 52 45 30 62 66
63A0    0 20   0   4 46 52 4F E1     61 66   0 38   0   4 4C 52
63B0   4F 83 61 66   0 90   0   4     46 43 4F A3 63 66   0 90
63C0    0  4 4C 43 4F AD 63 66       0 D8   0   4 4B 52 45 C1
63D0   63 99   0 FF   0   4 35 42     49 B7 63 3A   0 17   0 FF
63E0   8B   3 E2   0 79   2 17 39     C6   1 E3 4C 28   2   F   0
63F0   F7   0 A9   1 D6   0   5 38      2 CC   0 15 28   2 17   0
```

```
6400   4 2E  1 D8  2 46  2  F      0 F8  0 2E  1 88  2 F7
6410  4C F1  0 D0 4D  0  7 52     45 47 FD 60 3A  0  F  0
6420   0  4 88  3 E2  0 79  2      F  0 E0  3 2E  1 79  2
6430  17  0 1F 2E  1 17  0 20     F8  2 99  2 17  0  8 A9
6440  22  F  0 80  0 88  2 28      2 28  2 79  2 8B  3 41
6450  4D F1  0 D2 4D  0  B 4C     49 4E CB 63 3A  0 71 39
6460  B7  1 99  2 A3 39 B7  1     85 39 B7  1 95 22 8F 39
6470  B7  1 88  2 4D  0  8 50     4C 4F A8 61 3A  0 35  4
6480  8F 39 C6  1 99 39 C6  1     99  2 A3 39 C6  1 88  3
6490  BD 40 D5 36 35  4 13 36     71 39 C6  1 3B 36 7B 39
64A0  C6  1 7B 39 B7  1 71 39     B7  1 99  2 85 39 C6  1
64B0  D5 36 8B  3 E2  0  F  0     FF  0 79  2 BD 48 B7  1
64C0  5C 64 B7 39 B7  1 88  2     B7 39 B7  1 79  2 E8  2
64D0  17  0 40 88  2  B 4D 17      0  2  7  1 D9 4D  0  9
64E0  28 45 4E 8F 63 3A  0 35      4 71 39 C6  1 7B 39 C6
64F0   1 99  2 85 39 C6  1 35      4 8F 39 C6  1 99 39 C6
6500   1 99  2 A3 39 C6  1 17      0 FF 8B  3 E2  0 79  2
6510  17 39 C6  1 E3 4C 71 39     B7  1 56  2 A9  1 46  2
6520  7B 39 B7  1 56  2 98  1     67  2 8B  3 A9  1 D6  0
6530   9 48  4 8F 39 B7  1 CC      0 12 8B  3 A9  1 D6  0
6540   9 38  2 99 39 B7  1 CC      0  2 5C 64 F7 4C 95  3
6550  28  1 8B 4D  0  4 56 4D     49 D5 63 99  0 45 20  4
6560  56 4D 41 55 65 99  0 45     20  6 56 52 41 5F 65 99
6570   0 20 20  7 56 4D 49 69     65 3A  0  F  0 FF 7F 5B
6580  65 C6  1 8B  3 65 65 C6      1 B3 63 A9 63 E2  0 C7
6590  63 BD 63 E2  0 79  2 C1     4C F1  3 D2 4C E3 4C 28
65A0   2 5B 65 B7  1 69  3 5B     65 C6  1 65 65 B7  1 7D
65B0   3 65 65 C6  1 F1  0 D0     F1  0 D4 65 65 B7  1 5B
65C0  65 B7  1 99  2 6F 65 C6      1 4D  0  7 45 4E 48 73
65D0  65 3A  0 79 65  F  0 FF      0 8B  3 65 65 65 B7 1 5B
65E0  65 B7  1 E5 64 4D  0  4     46 4C 49 CB 65 EF 65 1E
65F0  53 8E 1E B5 38 B9  0  4     BA E0  3 BB  0  8 8B F9
6600  8B C1 23 C2 D1 E8 D1 E8     88  5  3 FB 88  5  3 FB
6610  88  5  3 FB E2 E8 5B 1F     AD 97 FF 25  4 46 4C 4F
6620  E7 65 24 66 1E 53 8E 1E     B5 38 B9  0  4 BA 1F  0
6630  BB  0  8 8B F9 8B C1 23     C2 D1 E0 D1 E8 D1 E0 88
6640   5  3 FB 88  5  3 FB 88      5  3 FB E2 E6 5B 1F AD
6650  97 FF 25  8 49 4E 4E 99     63 3A  0 EB 18 B7  1 17
6660   0 16 88  2 B7  1 B6 24     17  0 16 56  2 77  1 D6
6670   0 12 17  0  B AA 20 67     39 B7  1 B9  2 DA 48 8B
6680   3 CC  0 56 17  0  B 56      2 77  1 D6  0 12 17  0
6690  16 AA 20 67 39 B7  1 AA      2 DA 48 8B  3 CC  0 3A
66A0  17  0  8 56  2 77  1 D6      0 12 17  0  C AA 20 5D
66B0  39 B7  1 AA  2 C5 48 8B      3 CC  0 1E 17  0  C 56
66C0   2 77  1 D6  0 12 17  0      8 AA 20 5D 39 B7  1 B9
66D0   2 C5 48 8B  3 CC  0  2     95  3 46  2 38  2 4D  0
66E0   6 50 52 45 76 64 3A  0     95  3 46  2 86 4B 8B  3
66F0  46  2 86 4B  F  0  0  1     F9 38 C6  1 8B  3 C5 48
6700  8B  3 DA 48 4D  0  8 52     45 47 16 64 3A  0  3 30
6710  E6 66 2F 37 81 49 E4  5     E4  5 17  0  B AA 20  F
6720   0  0  1 F9 38 C6  1 17      0 10  A 32 ED 65 F9 38
6730  B7  1 D9 30 22 66 F9 38     B7  1 D9 30 6D 32 D6  0
6740   5 59 66 CC  0  2 8B  3     D6  0 E1 17  0 10 1F 32
6750  4D 37 81 49 8B  3 6A 4D     4D  0  9 52 45 46  6 67
6760  3A  0 17  0  0 9F 63 17      0  2 88  2 9F 63 B9  2
6770  E2  0 95 63 17  0  2 88      2 95 63 B9  2 E2  0 79
6780   2 C1 4C F1  3 D2 4C E3     4C 88  2 F1  0 F1 F1  0
6790  E1 17  0  9 A9 22 4D  0      5 4B 52 45 56 64 3A  0
67A0   3 30 E0 4A 60 67 46  2     E0 4A 60 67  F  0 FF  0
67B0  46  2 95 22 D1 63 C6  1     4D  0  4 50 41 4C E0 66
67C0  3A  0 56  2 88  2 46  2     E2  0 D1 63 B7  1 E3 4C
67D0  79  2 E5  1 95 22 28  2      F  0 FF  0 A9  1 D6  0
67E0   6 38  2  F  0 FF  0 F7     4C 95  3 17 39 D5  1 F1
67F0   0 D8 4D  0  5 52 41 54     5A 67 3A  0  3 30 35  4
```

```
6800   9E 67 46   2 E0  4A 9F  4A    67 39 B7   1 85   1 D6   0
6810   1B 67 39 B7   1 3F   3  F      0  0   1 F8   2 5D 39 B7
6820    1 99   2 17 39 C6   1 8B     3 CC   0 18 8B   3 17 39
6830   C6   1 67 39 B7   1 3F   3    F  0   0   1 F8   2 5D 39
6840   B7   1 8B   2 46   2 67 39    B7   1 17   0 20 A9 22 28
6850    2 85   1 D6   0   7 38   2    8B   3 CC   0   2 4F   3 35
6860    4 8B   2 60 2D 73 4A 67      2 2B   2   6   2   4   6 56
6870    2 88   2  F   0   0 20 67     2 99   2 C0 67 17   2 67
6880    2 67   2 56   2 17   0  8    88   2 67   2 67   2 88   2
6890   AA   2 E2   0 79   2 60 2D    73 4A   4   6  F   0   0 20
68A0   C0 67 F1   0 EF 38   2 4D     0   6 48 52 41 DF 64 3A
68B0    0 D2 4C 8B   3 67   2 67     2 E2   0 79   2 C1 4C 28
68C0    2 F7 4C 17   0   2 88   2    F1   0 F0 38   2 4D   0   6
68D0   4C 45 47 98 67 3A   0  F      0 FC   0  F   0 F4   0 E2
68E0    0 17   0   0 17   0 40 8B     3 79   2  B 4D 17   0   0
68F0    F   0   0   1  F   0 C0   0   79   2  B 4D  F   0 C0   0
6900   17   0 40 79   2 AF 68 F1     0 D7 17   0 10 17   0  8
6910   E2   0 8B   3  F   0 FF   0   8B   3 79   2  B 4D F1   0
6920   F1 8B   3  F   0   0   1 8B   3  F   0 F3   0  B 4D 4D
6930    0   7 52 41 49 F4 67 C3     34   3   0 FF 40   0 FF 80
6940    0 FF FF   0   0 FF   0   0   FF FF   0 80 FF   0   0 FF
6950   FF   0 FF   8 52 41 49 31    69 3A   0 8B   3 37 69 28
6960    2 AA   2 28   2 AA   2 67     2 E5   1 67   2 E5   1 67
6970    2 E5   1 4D   0   6 42 45    59 53 69 3A   0 17   0   0
6980   17   0   0  F   0 C0   0 ED   3B C6   1   1 3C C6   1 F7
6990   3B C6   1 D9 3B B7   1 95     3   B 3C B7   1 8E 4D  F
69A0    0 FF   0 28   2 28   2 ED    3B C6   1 F7 3B C6   1   1
69B0   3C C6   1 D9 3B B7   1 15    3C B7   1  F   0   0   1 56
69C0    2 99   2 8E 4D 4D   0   6    53 57 49 B5 62 3A   0 D9
69D0   3B B7   1 61   1 D6   0   D   8B   3 B4 49 95   3 D9 3B
69E0   C6   1 CC   0  A 95   3 B4    49 8B   3 D9 3B C6   1 4D
69F0    0  A 43 52 41 C7 69 F9     69 8B   6 21 3C 59 F7 E9
6A00   8B   E D1 3B F7 F9   3   6    D 3C 8B   E E5 3B 89   6
6A10   E5 3B 2B C1 FF 36 DB 3B     51 50 AD 97 FF 25   6 52
6A20   47 42 75 69 26 6A 8F   6     3 3C 8F   6 F9 3B 8F   6
6A30   EF 3B AD 97 FF 25   7 53    50 45 F1 69 3A   0  B 3C
6A40   B7   1 15 3C B7   1 56   2   99   2 1F 3C C6   1 E3 3B
6A50   C6   1 CF 3B B7   1 AA   2   95   3 E2   0 79   2 B9   2
6A60   59 69 24 6A 79   2 F7 69    8E 4D F1   0 EF 4D   0   4
6A70   4C 54 41 CF 68 3A   0   B    3C B7   1 88   2 95   3 56
6A80    2 A9   1 D6   0   7 38   2   95   3 CC   0 1E 15 3C B7
6A90    1 17   0   8 99   2 56   2   9B   1 D6   0  E 38   2 15
6AA0   3C B7   1 17   0   8 99   2   CC   0   0   B 3C C6   1 4D
6AB0    0   4 52 54 41 1E 6A 3A     0 15 3C B7   1 88   2  F
6AC0    0 FF   0 56   2 9B   1 D6     0   9 38   2  F   0 FF   0
6AD0   CC   0 1E  B 3C B7   1 17     0   8 88   2 56   2 A9   1
6AE0   D6   0  E 38   2  B 3C B7     1 17   0   8 88   2 CC   0
6AF0    0 15 3C C6   1 4D   0   4   54 41 42 36 6A 3A   0 28
6B00    2 15 3C D5   1  B 3C D5     1  B 3C B7   1 95   3 9B
6B10    1 D6   0 13 95   3  B 3C    C6   1 1F 3C B7   1 AA   2
6B20   15 3C C6   1 CC   0 24 15    3C B7   1  F   0 FF   0 A9
6B30    1 D6   0 17  F   0 FF   0   28   2 15 3C C6   1 1F 3C
6B40   B7   1 99   2  B 3C C6   1   CC   0   0 4D   0   2 2D 4C
6B50   20 78 62 3A   0 17   0 FF    75 6A 8B   3 4D   0   2 2B
6B60   4C 20 6F 6A 3A   0 95   3    75 6A 8B   3 4D   0   2 2D
6B70   52 20 4D 6B 3A   0 17   0    FF B7 6A 8B   3 4D   0   2
6B80   2B 52 20 5E 6B 3A   0 95     3 B7 6A 8B   3 4D   0   2
6B90   2D 54 20 6E 6B 3A   0 17     0 FF FD 6A 8B   3 4D   0
6BA0    2 2B 54 20 7F 6B 3A   0    95   3 FD 6A 8B   3 4D   0
6BB0    A 50 41 4C BA 67 3A   0    42 49 8B   3 6A 4D 95   3
6BC0   6A 4D 95   3 D9 3B C6   1    D5 68 8E 20 17   0 16 56
6BD0    2 77   1 D6   0   5 53 6B   CC   0 4D 17   0  B 56   2
6BE0   77   1 D6   0   5 64 6B CC    0 3E 17   0  8 56   2 77
6BF0    1 D6   0   5 74 68 CC   0   2F 17   0  C 56   2 77   1
```

```
6C00    D6   0   5  85  6B  CC   0  20     17   0  2C  56   2  77   1  D6
6C10     0   5  95  6B  CC   0  11  17      0  2E  56   2  77   1  D6   0
6C20     5  A6  6B  CC   0   2  95   3     46   2  38   2  7B  69  3C  6A
6C30    CD  69  D6   0  95  4D   0   3     42  26  57  B1  6A  3A   0  8B
6C40     3  6A  4D  95   3  6A  4D  8B      3  B4  49  4D   0   2  54  54
6C50    20  F7  6A  3A   0  E2   0  79      2   F   0   0   4  87   7  E4
6C60     5  E4   5   F   0   0   4   7      1  ED  4D   0   0  20  20  20
6C70    4E  54  20  20  20   9  6C  56      2  17   0  20  77   1  D6   0
6C80     6  17   0   A  CC   0   3  17     46  36  43  36  43  6C  4D   0
6C90     5  53  45  4E  40  6C  3A   0     3D   5  C8   2  E5   1   6   2
6CA0    1B  6C  8B   3  28   2  35   4     79   2  17   0   3  73  6C  17
6CB0     2  48   4  4D   0   B  52  45     43  DB  6B  3A   0  3D   5  C8
6CC0     2  E5   1   6   2  1B  6C  8B      3  28   2  35   4  79   2  95
6CD0     3  73  6C  17   2  48   4  4D      0   6  2E  53  45  9D  6B  3A
6CE0     0  15   8  16  20  20  20  44     49  53  4B  20  45  52  52  4F
6CF0    52  3A  20  20  20  54  59  50     45  20  1B  6C  28   2  E5   1
6D00    46   2  56   2  28   2  17   0     30  2E   1  17   0  10  A9  22
6D10    35   7  15   8   8  20  20  20     43  4F  44  45  20  17   0   F
6D20    2E   1  35   7  15   8   7  20     20  20  4C  55  4E  20  AA   2
6D30    28   2  E5   1  17   0  60  2E      1  17   0  20  A9  22  35   7
6D40    46   2   F   0  80   0  2E   1     61   1  D6   0   5  38   2  CC
6D50     0  1A  15   8   A  20  20  20     53  45  43  54  4F  52  20  AA
6D60     2  B7   1  A3  6B  53   7  17      0   3  6C   6  17   0   3  6C
D70      6  4D   0   6  3F  45  52  46     6B  3A   0  3D   5  C8   2  E5
6D80     1  17   0   3  2E   1  61   1     61   3  4D   0   5  45  52  52
6D90    12  6B  3A   0  79  6D  D6   0     54  17   0   A  8B   3  E2   0
6DA0     9  6C   9  6C  17   0   5  88      2  E2   0  79   2  E5   1  17
6DB0     0  FF   7   1  F6  73  6C  79     6D  61   3  D6   0   2   0   4
6DC0    F1   0  DD  79  6D  D6   0  25     17   0   7  AA  20  C3   4  87
6DD0     1  F1   0  F5  6B  B7   1  8B      3  F5  6B  C6   1  96  6C  E4
6DE0     5  DF  6C  8B  6C  F5  6B  C6      1  C3   4  C6   1  4D   0   7
6DF0    43  44  42  90  6C  3A   0  8B      3  17   0   4  67   2  28   2
6E00    17   0   4  F8   2  28   2  A3     6B  67   2   F   0  67   2  A9
6E10     1  D6   0   6  17   0  20  CC      0   2  8B   3  FF  6B  B7   1
6E20    3F   1  4D   0   8  5B  42  55     C1  6B  3A   0  30   5   D  19
6E30    17   A  F5  6D  17   0   A  73     6C  92  6D  3D   5  19  19  4D
6E40     0   7  5B  42  4C  24  6E  3A      0  B1   4  B7   1  8B   2  7F
6E50     5  DF   9  8F   A  3D   5  2E     19  56   2  F5  60  17   0   8
6E60    73  6C  92  6D  46   2  7B   A     3D   5  19  19  4D   0   2  46
6E70    3A  20  8C  6D  3A   0  8B   3     FF  6B  C6   1  4D   0   2  57
6E80    3A  20  60  6C  3A   0  17   0     20  FF  6B  C6   1  4D   0   3
6E90    53  43  40  EF  6D  97  6E  8C     DA  5F  1F  2B  C0  8A   5  58
6EA0    8E  DA  AD  97  FF  25   2  53     40  20  8F  6E  AE  6E  8C  DA
6EB0    5F  1F  8A   5  47  8A  25  50     8E  DA  AD  97  FF  25   3  53
6EC0    43  21  A6  6E  C6  6E  8C  C2     5F   7  58  AA  8E  C2  AD  97
6ED0    FF  25   2  53  21  20  BE  6E     DA  6E  8C  C2  5F   7  58  AA
6EE0    8A  C4  AA  8E  C2  AD  97  FF     25   5  53  4D  4F  02  6E  F1
6EF0    6E  59  5F  8C  D8  8C  C2  89     76  FE   7  5E  1F  50  F3  A4
6F00    1F  8E  C2  88  76  FE  AD  97     FF  25   4  50  49  43  73  60
6F10    12  6F  8B  FC  58  D1  E0   3     F8  FF  35  AD  97  FF  25   4
6F20    52  4F  4C  B5  6C  3A   0  28      2  D8   2   6   2  10  6F  C0
6F30     3  28   2  C8   2  17   2  74     E  38   2  4D   0   5  35  55
6F40    53  6E  6E  45  6F  59  87  C8     87  C8  87  C8  87  C8  E2  F6
6F50    AD  97  FF  25   4  4D  53  45     D9  6C  3A   0   F   0  C8   0
6F60    F8   2  43  6F  4D   0   4  4Z     45  4C  1F  6F  3A   0  17   0
6F70     7  AA  20  4D   0   5  51  55     45  66  6F  3A   0  60   4  B7
6F80     1  17   0  50  C7   5  4D   0      2  4E  3F  20  54  6F  3A   0
6F90    7B  6F  8B   3  CC   4  C6   1     17   0  20  75   8  5C   9  4D
6FA0     0   4  3C  4E  3C  41  6E  3A      0  15   8   4  20  20  5B  20
6FB0    95   3  10  6F  53   7  15   8      6  3C  20  4E  20  3C  20  17
6FC0     0   2  10  6F  53   7  15   8      3  5D  20  20  8E  6F  17   0
6FD0     2  10  6F  56   2  9B   1  61      1  17   0   4  10  6F  17   0
6FE0     3  10  6F  98   1  3F   1  28      2  D6   0   8  46   2  38   2
6FF0    6C  6F  E4   5  61   1  D6   0     B0  67   2  67   2  48   4  4D
```

| | | | | | | | | | | | | | | | | |
|---|---|---|---|---|---|---|---|---|---|---|---|---|---|---|---|---|
| 0 | 22 | E | 0 | 0 | 22 | E | 0 | 0 | 22 | E | 0 | 0 | 22 | E | 0 | 0 |
| 10 | 22 | E | 0 | 0 | 22 | E | 0 | 0 | 22 | E | 0 | 0 | 22 | E | 0 | 0 |
| 20 | 22 | E | 0 | 0 | 22 | E | 0 | 0 | 22 | E | 0 | 0 | 22 | E | 0 | 0 |
| 30 | 22 | E | 0 | 0 | 22 | E | 0 | 0 | 22 | E | 0 | 0 | 22 | E | 0 | 0 |
| 40 | A5 | 5A | FF | FF | FF | FF | FF | FF | FF | FF | FF | FF | FF | FF | FF | FF |
| 50 | FF | FF | FF | FF | FF | FF | FF | FF | FF | FF | FF | FF | FF | FF | 0 | 67 |
| 60 | FF | FF | FF | FF | FF | FF | FF | FF | FF | FF | FF | FF | FF | FF | FF | FF |
| 70 | FF | FF | FF | FF | FF | FF | FF | FF | FF | FF | FF | FF | FF | FF | FF | FF |
| 80 | A1 | 40 | 0 | F7 | D0 | A3 | 40 | 0 | B8 | 0 | 0 | 8E | C0 | 8E | D0 | 8E |
| 90 | D8 | BD | FE | F | BC | 7E | F | B8 | 90 | 0 | E6 | 6 | BE | 23 | E | FC |
| A0 | AD | 97 | FF | 25 | FF | FF | FF | FF | FF | FF | FF | FF | FF | FF | FF | FF |
| B0 | FF | FF | FF | FF | FF | FF | FF | FF | FF | FF | FF | FF | FF | FF | FF | FF |
| C0 | FF | FF | FF | FF | FF | FF | FF | FF | FF | FF | FF | FF | FF | FF | FF | FF |
| D0 | FF | FF | FF | FF | FF | FF | FF | FF | FF | FF | FF | FF | FF | FF | FF | FF |
| E0 | FF | FF | FF | FF | FF | FF | FF | FF | FF | FF | FF | FF | FF | FF | FF | FF |
| F0 | FF | FF | FF | FF | FF | FF | FF | FF | FF | FF | FF | FF | FF | FF | FF | FF |
| 100 | 2 | 1 | AD | 50 | AD | 97 | FF | 25 | A | 1 | AC | 98 | 50 | AD | 97 | FF |
| 110 | 25 | 47 | 47 | 4D | 4D | 89 | 76 | 0 | 8B | F7 | AD | 97 | FF | 25 | 20 | 1 |
| 120 | 8B | 76 | 0 | 45 | 45 | AD | 97 | FF | 25 | FF | 75 | 2 | AD | 97 | FF | 25 |
| 130 | 47 | 47 | 57 | AD | 97 | FF | 25 | 4D | 4D | 89 | 76 | 0 | 5E | 47 | 47 | 57 |
| 140 | AD | 97 | FF | 25 | 8A | 45 | 2 | 98 | 3 | C3 | 50 | AD | 97 | FF | 25 | 51 |
| 150 | 1 | AC | 98 | 3 | F0 | AD | 97 | FF | 25 | 5B | 1 | 58 | 8 | C0 | 74 | F1 |
| 160 | 46 | AD | 97 | FF | 25 | 67 | 1 | 83 | ED | 4 | 8F | 46 | 0 | 8F | 46 | 2 |
| 170 | AD | 97 | FF | 25 | 76 | 1 | 8B | 46 | 0 | 40 | 3B | 46 | 2 | 73 | 1C | 89 |
| 180 | 46 | 0 | AC | 98 | 3 | F0 | AD | 97 | FF | 25 | 8C | 1 | 58 | 8B | D0 | 3 |
| 190 | 46 | 0 | 8B | F8 | 2B | 7E | 2 | 33 | D7 | 78 | E4 | 83 | C5 | 4 | 46 | AD |
| 1A0 | 97 | FF | 25 | A5 | 1 | 58 | 3 | 46 | 0 | EB | CF | AD | 1 | 5A | 58 | 23 |
| 1B0 | C2 | 50 | AD | 97 | FF | 25 | B8 | 1 | 5A | 58 | B | C2 | 50 | AD | 97 | FF |
| 1C0 | 25 | C3 | 1 | 5A | 58 | 33 | C2 | 50 | AD | 97 | FF | 25 | CE | 1 | 58 | B |
| 1D0 | C0 | B8 | 0 | 0 | 75 | 1 | 40 | 50 | AD | 97 | EF | 25 | DE | 1 | 5A | 58 |
| 1E0 | 3B | C2 | EB | ED | E6 | 1 | 58 | B | C0 | B8 | 0 | 0 | 79 | 1 | 40 | 50 |
| 1F0 | AD | 97 | FF | 25 | F6 | 1 | 5A | 58 | 2B | C2 | EB | ED | FE | 1 | 58 | 5A |
| 200 | 2B | C2 | EB | E5 | 6 | 2 | 5F | FF | 35 | AD | 97 | FF | 25 | F | 2 | 5F |
| 210 | 58 | AB | AD | 97 | FF | 25 | 18 | 2 | 5F | 58 | 1 | 5 | AD | 97 | FF | 25 |
| 220 | 22 | 2 | 5F | 2B | C0 | 8A | 5 | 50 | AD | 97 | FF | 25 | 2E | 2 | 5F | 58 |
| 230 | AA | AD | 97 | FF | 25 | 37 | 2 | 4D | 4D | 8F | 46 | 0 | AD | 97 | FF | 25 |
| 240 | 42 | 2 | FF | 76 | 0 | 45 | 45 | AD | 97 | FF | 25 | 4D | 2 | 88 | FC | FF |
| 250 | 35 | AD | 97 | FF | 25 | 57 | 2 | 44 | 44 | AD | 97 | FF | 25 | 5F | 2 | 5A |
| 260 | 58 | 52 | 50 | AD | 97 | FF | 25 | 69 | 2 | 88 | FC | FF | 75 | 2 | AD | 97 |
| 270 | FF | 25 | 74 | 2 | 5F | 5A | 58 | 52 | 57 | 50 | AD | 97 | FF | 25 | 80 | 2 |
| 280 | FF | 76 | 0 | AD | 97 | FF | 25 | 89 | 2 | 5A | 58 | 3 | C2 | 50 | AD | 97 |
| 290 | FF | 25 | 94 | 2 | 5A | 58 | 2B | C2 | 50 | AD | 97 | FF | 25 | 9F | 2 | 58 |
| 2A0 | 40 | 50 | AD | 97 | FF | 25 | A8 | 2 | 58 | 48 | 50 | AD | 97 | FF | 25 | B1 |
| 2B0 | 2 | 58 | 40 | 40 | 50 | AD | 97 | FF | 25 | BB | 2 | 58 | D1 | E0 | 50 | AD |
| 2C0 | 97 | FF | 25 | C5 | 2 | 58 | D1 | F8 | 50 | AD | 97 | FF | 25 | CF | 2 | 5F |
| 2D0 | 58 | F7 | E7 | 50 | AD | 97 | FF | 25 | DA | 2 | 5F | 58 | 2B | D2 | F7 | F7 |
| 2E0 | 52 | 50 | AD | 97 | FF | 25 | E8 | 2 | 59 | 5F | 58 | E3 | 11 | 96 | D1 | CF |
| 2F0 | D1 | C7 | 73 | 2 | A4 | 49 | D1 | E9 | F3 | A5 | 73 | 1 | A4 | 96 | AD | 97 |
| 300 | FF | 25 | 4 | 3 | 58 | F7 | D8 | 50 | AD | 97 | FF | 25 | E | 3 | 58 | B |
| 310 | C0 | 78 | F2 | 50 | AD | 97 | FF | 25 | 81 | 53 | 1C | 3 | 58 | 5A | 3B | C2 |
| 320 | 79 | 1 | 92 | 52 | AD | 97 | FF | 25 | 2A | 3 | 58 | 5A | 3B | D0 | EB | F0 |
| 330 | 32 | 3 | 5F | 2B | D2 | 58 | F7 | F7 | 52 | AD | 97 | FF | 25 | 3F | 3 | 5F |
| 340 | 5A | 58 | F7 | E2 | F7 | F7 | 52 | 50 | AD | 97 | FF | 25 | 4E | 3 | 5F | 5A |
| 350 | 58 | F7 | EA | F7 | FF | 50 | AD | 97 | FF | 25 | 5C | 3 | 5F | 58 | 99 | F7 |
| 360 | FF | 50 | AD | 97 | FF | 25 | 68 | 3 | 58 | 5A | F7 | EA | 50 | 52 | AD | 97 |
| 370 | FF | 25 | 74 | 3 | 5F | 5A | 58 | F7 | FF | 50 | AD | 97 | FF | 25 | 80 | 3 |
| 380 | 58 | 5A | F7 | E2 | 50 | 52 | AD | 97 | FF | 25 | 8C | 3 | 5F | 5A | 58 | F7 |
| 390 | F7 | 52 | 50 | AD | 97 | FF | 25 | 99 | 3 | 5F | FF | 75 | 2 | FF | 35 | AD |
| 3A0 | 97 | FF | 25 | A5 | 3 | 5F | 58 | AB | 58 | AB | AD | 97 | FF | 25 | B0 | 3 |
| 3B0 | 8B | FC | FF | 75 | 2 | FF | 35 | AD | 97 | FF | 25 | BD | 3 | 83 | C4 | 4 |
| 3C0 | AD | 97 | FF | 25 | 29 | 1 | 0 | 0 | 29 | 1 | 0 | 0 | 29 | 1 | 0 | 10 |
| 3D0 | 29 | 1 | 0 | FF | 29 | 1 | 10 | 0 | 29 | 1 | 10 | 0 | 29 | 1 | 6F | 0 |
| 3E0 | 29 | 1 | 6F | 0 | 29 | 1 | FF | FF | 29 | 1 | FF | FF | 29 | 1 | 0 | 0 |
| 3F0 | 29 | 1 | 0 | 0 | 30 | 1 | FF | FF | 30 | 1 | 0 | 0 | 30 | 1 | 9 | 0 |

```
400   30   1 FF FF 30   1 FF FF     30   1 FF FF FF FF FF FF
410   FF FF FF FF FF FF FF FF       FF FF FF FF 30   1 FF FF
420   FF FF FF FF FF FF FF FF       FF FF FF FF FF FF FF FF
430   FF FF FF FF FF FF FF FF       FF FF FF FF FF FF FF FF
440   FF FF FF FF FF FF FF FF       FF FF FF FF FF FF FF FF
450   FF FF FF FF FF FF FF FF       FF FF FF FF FF FF FF FF
460   FF FF FF FF FF FF FF FF       FF FF FF FF FF FF FF FF
470   FF FF FF FF FF FF FF FF       FF FF FF FF FF FF FF FF
480   FF FF FF FF FF FF FF FF       FF FF FF FF FF FF FF FF
490   FF FF FF FF FF FF FF FF       FF FF FF FF FF FF FF FF
4A0   FF FF FF FF FF FF FF FF       FF FF FF FF FF FF FF FF
4B0   FF FF FF FF FF FF FF FF       FF FF FF FF FF FF FF FF
4C0   FF FF FF FF FF FF FF FF       FF FF FF FF FF FF FF FF
4D0   FF FF FF FF FF FF FF FF       FF FF FF FF FF FF FF FF
4E0   FF FF FF FF FF FF FF FF       FF FF FF FF FF FF FF FF
4F0   FF FF FF FF FF FF FF FF       FF FF FF FF FF FF FF FF
500   FF FF FF FF FF FF FF FF       FF FF FF FF FF FF FF FF
510   FF FF FF FF FF FF FF FF       FF FF FF FF FF FF FF FF
520   FF FF FF FF FF FF FF FF       FF FF FF FF FF FF FF FF
530   FF FF FF FF FF FF FF FF       FF FF FF FF FF FF FF FF
540   FF FF FF FF FF FF FF FF       FF FF FF FF FF FF FF FF
550   FF FF FF FF FF FF FF FF       FF FF FF FF FF FF FF FF
560   FF FF FF FF FF FF FF FF       FF FF FF FF FF FF FF FF
570   FF FF FF FF FF FF FF FF       FF FF FF FF FF FF FF FF
580   FF FF FF FF FF FF FF FF       FF FF FF FF FF FF FF FF
590   FF FF FF FF FF FF FF FF       FF FF FF FF FF FF FF FF
5A0   FF FF FF FF FF FF FF FF       FF FF FF FF FF FF FF FF
5B0   FF FF FF FF FF FF FF FF       FF FF FF FF FF FF FF FF
5C0   FF FF FF FF FF FF FF FF       FF FF FF FF FF FF FF FF
5D0   FF FF FF FF FF FF FF FF       FF FF FF FF FF FF FF FF
5E0   FF FF FF FF FF FF FF FF       FF FF FF FF FF FF FF FF
5F0   FF FF FF FF FF FF FF FF       FF FF FF FF FF FF FF FF
600   FF FF FF FF FF FF FF FF       FF FF FF FF FF FF FF FF
610   FF FF FF FF FF FF FF FF       FF FF FF FF FF FF 29   1
620   40   0 29   1 40   0 29   1   42   0 29   1 43   0 29   1
630   44   0 29   1 46   0 29   1   48   0 29   1 4A   0 29   1
640   4C   0 29   1 4D   0 29   1   4E   0 29   1 4F   0 29   1
650   52   0 29   1 54   0 29   1   55   0 29   1 56   0 29   1
660   57   0 29   1 58   0 29   1   59   0 29   1 5A   0 29   1
670   5D   0 29   1 5E   0 29   1   5F   0 30   1 FB   7   B   8
680   15   A 11   8 ED   D 18   E   29   1   0   0 29   1   1   0
690   29   1   2   0 29   1   3   0   29   1   4   0 29   1   5   0
6A0   A2   6 8C DA 5F 1F 2B C0       8A   5 50 8E DA AD 97 FF
6B0   25 B3   6 8C DA 5F 1F 8A       5 47 8A 25 50 8E DA AD
6C0   97 FF 25 C5   6 8C C2 5F       7 58 AA 8E C2 AD 97 FF
6D0   25 D3   6 8C C2 5F   7 58     AA 8A C4 AA 8E C2 AD 97
6E0   FF 25 E4   6 58 59 5F 8C      C2   7 F3 AA 8E C2 AD 97
6F0   FF 25 11   1 4B   2 35   2     2   3 A6   2 AB   1 5D   2
700   46   2 AB   1 B6   1 1E   1   11   1 4B   2 35   2   4   2
710   5D   2 F2   6 40   2   D   2   1E   1 11   1 AE   3 35   2
720   35   2 A0   6 5D   2 F2   6   40   2 40   2 C3   6 1E   1
730   32   7 59 87 C8 87 C8 87      C8 87 C8 E2 F6 AD 97 FF
740   25 11   1   0   1 C8   0 CD     2 30   7 1E   1 4F   7 5A
750   2B C0 EC 50 AD 97 FF 25      5A   7 5A 58 EE AD 97 FF
760   25 63   7 8B 46   0 89 46       2 AD 97 FF 25 6F   7 5A
770   5F 58 59 57 52 51 58 AD      97 FF 25 7D   7 8B FC FF
780   75   6 FF 75   4 AD 97 FF     25 8B   7 5F 4F 4F FF 25
790   AD 97 FF 25 96   7 FB F4      FA AD 97 FF 25 9F   7 B0
7A0    8 E6   6 90 B0   9 E6   6    AD 97 FF 25 AE   7 8B   6
7B0   40   0 3D 5A A5 75   5 B8      1   0 EB   2 2B C0 50 AD
7C0   97 FF 25 C5   7 C7   6 40      0 A5 5A AD 97 FF 25 AD
7D0   97 FF 25 11   1 5D   2   8     1 10 B6   1 5D   2 58   7
7E0   9D   7 1E   1 11   1 4B   2    8   1   4 DC   1 59   1   5
7F0   D3   7 4F   1   2 58   7 1E    1 11   1 32   6 20   2 2E
```

```
800    6 20  2 E4  7 C3  7 1E     1 11  1 C3  7 1E  1 11
810    1 3A  6  4  2 36  6  4     2 A0  6 32  6 2C  2 C3
820    7 1E  1 11  1 B9  2 67     2 87  2 5D  2 65  1 7E
830    2  D  2  8  1  2 8A  1     F6 1E  1 11  1 E4  1 5D
840    2 E4  1 DC  1 1E  1 11     1 AE  3 3B  8 59  1  5
850    28 3 4F  1  B 67  2 E4     1 59  1  2 5D  2 55  2
860    1E 1 11  1 AE  3  4  2     5D  2  4  2 67  2 47  8
870    DC  1 59  1  2 5D  2 55     2 1E  1 30  1  0 80 11
880    1 7B  8 72  2 72  2  B     3  2 CD  2 67  2 87  2
890    5D  2 65  1 7E  2 62  8     8  1  2 8A  1 F6 1E  1
8A0    A2  8 59 5F 5A 53 55 56     8B F2 BB  0  0 8B EB AD
8B0    F7 2D 83 C7  2  3 D8 13     EA E2 F4 8B C3 8B CD 5E
8C0    5D 5B 50 51 AD 97 FF 25     11  1 FC  3  4  2  8  1
8D0    0 65  1  8  1  1 74  1     FA  0  1  A  4 FC  3  4
8E0    2 23  8 FC  3  4  2 4B     2  4  4  D  2 C3  2 B9
8F0    2  0  4  D  2 1E  1 11     1  0  4  4  2 92  2  0
900    1  A  4 FC  3  4  2 A0     8  4  4  4  2 72  3 1E
910    1 11  1 FC  3  4  2 C3     2 B9  2  0  4  D  2 35
920    2 5D  2 40  2 B9  2  8     1  0 65  1 AE  3 7E  2
930    87  2 F7  8 5D  2 7E  2     87  2  D  2  8  1  2 8A
940    1 EA BB  3 1E  1 11  1     A6  2 B9  2 67  2 87  2
950    5D  2 65  1 7E  2 AF  2     4  2 7E  2  4  2 92  2
960    7E  2  D  2  8  1  2 8A     1 EA 1E  1 6E  9  6 1E
970    56 53 8E  6 CE  3 2B F6     BB  0  0 2B D2 B9  0  8
980    8E 1E D2  3 2B C0 26 AC     D7  3 D0 E2 F9 5B 5E 1F
990    7 52 AD 97 FF 25 11  1     94  7  8  1  F 41  7  0
9A0    1  0  1  8  1  0 65  1     7E  2  8  1  2 58  7  8
9B0    1  2  8  1  4 D3  7  8     1  1  8  1  4 D3  7  8
9C0    1  2 41  7 94  7 6C  9     1C  4 7E  2 B9  2 87  2
9D0    D  2 74  1 D3 1E  1 11     1 C8  8 1C  4  0  4  4
9E0    2 87  2 1C  4  0  1  0     1  0  4  4  4  2 92  2 11
9F0    9 1C  4  0  1  0  1  0     4  4  2 92  2 AE  3 46
A00    9 A6  2 7F  8  0  4  4     2 87  2 1C  4 92  2 C3
A10    2 1E  1 11  1 96  9 D7     9  8  1  8 87  2 4B  2
A20    8  1  2 58  7  8  1  2     8  1  4 D3  7 4E  6 2C
A30    2 C3  7 1E  1 11  1 AC     7 59  1  E 26  6 20  2
A40    B9  2 7A  6 87  2  4  2     89  7 1E  1 4E  A 58 B9
A50    8  0 2B D2 8A 16 4E  0     80 FA  0 75  D BA  1  0
A60    D1 EA D0 DA 84 C2 E1 FA     EB 10 BA  1  0 D1 EA D0
A70    D2 84 C2 E1 FA F7 D9 83     C1  7 51 AD 97 FF 25 81
A80    A 5A 59 D1 E1 D1 E1 D1     E1 D1 E1 2B C0 8A 16 4E
A90    0 3C  0 58 75  C 2B CA     3A C8 73  3 51 EB  1 50
AA0    EB  A  3 CA 3A C8 73  3     50 EB  1 51 AD 97 FF 25
AB0    B2  A 5F 2B C0 A0 4E  0     1E 8E 1E CE  3 3C  0 75
AC0    5 BA FF FF EB  3 BA  1     0 8A  5  A C0 75  D 2B
AD0    C0 B9  8  0  3 FA 8A  5     A C0 E1 F8 1F 57 AD 97
AE0    FF 25 11  1 46  6 20  2     EB  3  0  1 FF  0 AB  1
AF0    DC  1 59  1  F  0  1 80     3C  0  1 49  4  0  1 C8
B00    3 4F  1  C  0  1 70 3C     0  1 48  4  0  1 C7  3
B10    65  1 7E  2 80  A CC  3     67  2 A0  6 4C  A 7F  A
B20    8  1 40 8A  1 EC 4B  2     8  1 10 5A  3 5D  2  8
B30    1  7 AB  1 1E  1 11  1     72  2 87  2 4B  2 E4  1
B40    59  1  5  8  1  8 92  2     4B  2  8  1  8 5A  3 72
B50    2 87  2 5D  2  8  1  8     30  3 1E  1 5E  B 59 58
B60    B C9 79  6 F7 D9 D3 EB     EB  2 D3 E0 50 AD 97 FF
B70    25 73  B 58 8A E0 5F 5A     D1 E2 D1 E2 D1 E2 D1 E2
B80    B9 40  0 1E 8E 1E CE  3     3 FA 8A  5 22 C4 E1 F8
B90    1F 57 AD 97 FF 25 11  1     46  6 20  2 F0  3  0  1
BA0    FF  0 AB  1 DC  1 59  1     6  8  1  1 4F  1  3  8
BB0    1 FF E2  A 36  B  8  1     1 5D  2 5C  B AE  3  8
BC0    1  1 72  2 72  2 71  B     8  1 FF 6D  7 71  B AE
BD0    3 22  6  8  1 24 87  2     D  2 22  6  8  1 26 87
BE0    2  D  2 87  2  8  1  2     DB  2 87  2  8  1 10 5A
BF0    3 1E  1 F5  B 5F 1E 8E     1E CE  3 BA 10  0 B9 40
```

```
C00     0 B4 FF  3 FA 8A  5 3A    C4 E0 F8 1F 57 AD 97 FF    LISTING NO. 3
C10    25 13  C 59 8B F9 1E 8E    1E CE  3 B4 FF 8A  5 3A
C20    E0 75 1F 4F 8A  5 3A E0    75 13 83 C7  2 8A  5 3A       Sheet 4
C30    E0 75  5 B8  0  0 EB  3    B8 11  0 EB  3 B8  F  0
C40    EB  3 B8 10  0 1F 50 AD    97 FF 25 11  1  8  1  0
C50    87  2 4B  2 11  C 4B  2    CC  1 59  1 F3 55  2 1E
C60     1 63  C 5F 2B C0 A0 4E     0 1E 8E 1E CE  3 3C  0
C70    75  C BA  1  0 81 E7 F0     7 83 C7  2 EB  9 BA FF
C80    FF 83 CF  F 83 EF  2 B4    FF B9  C  0  3 FA 8A  5
C90    3A C4 E1 F8 1F 57 AD 97    FF 25 11  1 46  6 20  2
CA0     0  1 FF  0 C1  1 46  6    2C  2 1E  1 11  1 61  C
CB0    B0  A CC  3 67  2 A0  6    4C  A 50  2  8  1  F AB
CC0     1  8  1  8 CD  2 87  2    1E  1 11  1  0  1  8  4
CD0    F3  B 4B  C 4B  2 22  6     8  1 2C 87  2  D  2  8
CE0     1 40 92  2 4B  2 9A  C    AC  C 9A  C 5D  2 AC  C
CF0    AE  3 22  6  8  1 2A 87     2  D  2 22  6  8  1 28
D00    87  2  D  2 87  2  8  1     2 D8  2 87  2 1E  1 11
D10     1 42  6 20  2 87  2  8     1  2 E4  7  8  1 20  8
D20     1  4 E4  7 3E  6 20  2    87  2  8  1  2 E4  7  8
D30     1  B  8  1  4 E4  7 1E     1 11  1 76  6  8  7 76
D40     6 20  2  8  1  2 E4  7     8  1 2B  8  1  4 E4  7
D50    1E  1 11  1  0  1 FF  0    C1  1  0  1 80  0 39  D
D60    1E  1 11  1 AE  3 D8  3    F4  1 5D  2 D4  3 F4  1
D70    B6  1 72  2 72  2 E0  3    FC  1 5D  2 DC  3 FC  1
D80    B6  1 B6  1 CC  1 1E  1    11  1  8  1  1  8  1  4
D90    E4  7 94  7  8  1  2 41     7 94  7 96  B CA  C 5D
DA0     2 AE  3 22  6  8  1 22    87  2  D  2 22  6  8  1
DB0    20 87  2  D  2 1E  1 11     1 88  D 4A  6 20  2 59
DC0     1 25 AE  3 62  D 59  1    19 F8  3  4  2 CC  1 59
DD0     1  B EC  3 52  0  8  1    FF F8  3  D  2  F  D 4F
DE0     1  2 BB  3 4F  1  2 BB     3 1E  1 11  1 C3  7  8
DF0     1  0 F8  3  D  2 E4  3    2A  6 2C  2 B7  D 35  A
E00     8  1  1 41  7 35  A 2A     6 20  2 CC  1 59  1 EC
E10    E4  3 52  D 1E  1 11  1    EC  3 2A  6 2C  2 C3  7
E20    1E  1 CF  0  1  0  0 C4     3  8  1  4 87  2  8  1
E30    3C E6  2 35  A  8  1  0    59  1 F8 1E  1
```

## Claims

1. Apparatus for examining the ocular fundus of an eye, comprising:

(a) means for illuminating the ocular fundus with light;

(b) means, responsive to the light reflected by the illuminated ocular fundus, for forming an image of the ocular fundus at a detecting plane;

(c) means for sensing and scanning the image of the ocular fundus at the detecting plane; and

(d) data processing means, coupled to said sensing and scanning means, for storing and displaying data about the ocular fundus.

2. Apparatus according to claim 1 wherein said means for illuminating comprises means for filtering the light.

3. Apparatus according to claim 2 wherein said means for filtering comprises a plurality of filters being selectable to filter the light at different wavelengths.

4. Apparatus according to claim 3 wherein said data processing means comprises means for controlling said filtering means to select any one of the plurality of filters.

5. Apparatus according to claim 1 wherein said means for forming an image comprises means for producing an image of the ocular fundus at the detecting plane having three-dimensional information.

6. Apparatus according to claim 5 wherein said means for producing comprises means for providing a stereo pair of images of the ocular fundus at the detecting plane.

7. Apparatus according to claim 6 wherein said means for providing a stereo pair comprises:

(a) means for imaging the pupil of the eye at an imaging plane;

(b) means for providing a pair of apertures at the imaging plane, each of the apertures transmitting light therethrough; and

(c) means for spatially separating the light being transmitted through one of the apertures relative to the light being transmitted through the other of the apertures.

8. Apparatus according to claim 7 wherein said means for spatially separating comprises a biprism.

9. Apparatus according to claim 5 wherein said means for producing the image comprises means for providing at least one aperture of predetermined shape, said one aperture being in the illuminating path of said means for illuminating, and said means for illuminating comprising means for forming an image of said one aperture on the ocular fundus.

0122961

10. Apparatus according to claim 9 wherein said aperture is a slit.

11. Apparatus according to claim 5 wherein said sensing and scanning means has distortion, and further comprising means for producing calibration data at the detecting plane to correct for the distortion.

12. Apparatus according to claim 11 wherein said means for producing calibration data comprises:

(a) means for providing at least one aperture in the optical path of said image forming means;

(b) means for illuminating said providing means; and

(c) means for imaging said one aperture at the detecting plane.

13. Apparatus according to claim 3 wherein said sensing and scanning means has different sensitivity to light at different wavelengths, and further comprising means for providing optical sensitivity data at the detecting plane to correct for the different sensitivity.

14. Apparatus according to claim 1 wherein said means for illuminating and said means for forming have an optical axis, and further comprising means for automatically aligning the eye relative to the optical axis.

0122961

15. Apparatus according to claim 1 wherein said data processing means. stores a plurality of images of the ocular fundus which are sensed and scanned by said sensing and scanning means.

16. Apparatus according to claim 15 wherein said data processing means includes means for registering two or more of the plurality of images with one another.

17. Apparatus according to claim 16 wherein said means for registering comprises:

(a) a display;

(b) means for displaying two of the plurality of images on said display; and

(c) means for moving one of the displayed images relative to the other.

18. Apparatus according to claim 17 wherein said means for registering further comprises means for determining and storing the amount and direction of movement of the one simultaneously displayed image relative to the other.

19. Apparatus according to claim 16 further comprising:

(a) means for determining the intensity of each two corresponding picture elements of the registered images;

(b) means for determining a difference in intensity between each two corresponding picture elements; and

(c) means for displaying a difference image representing the difference in intensity.

20. Apparatus according to claim 16 further comprising:

(a) means for determining the intensity of each two corresponding picture elements of the registered images;

(b) means for determining a ratio of intensities of each two corresponding picture elements; and

(c) means for displaying a ratio image representing the ratio.

21. Apparatus according to claim 6 wherein said data processing means comprises means for determining the distance between any corresponding features in the stereo pair of images.

22. Apparatus according to claim 21 wherein said data processing means further comprises means, connected to said determining means, for displaying three-dimensional information of a region of the ocular fundus.

23. Apparatus according to claim 22 wherein the three-dimensional information is a depth-profile map.

FIG _ 1

1/7

0122961

FIG _ 2

86
87
87a
87b
16
84
84a
84b
16
DEVIATED RAYS

0122961

FIG _ 3A

FIG _ 3B

FIG _ 3C

0122961

3/7

FIG_4

FIG _ 5

0122961

254

260

( BLACK )

( WHITE )

268

262

267

261

264

266

270

272

258

256

CORNEAL
REFLECTION

PUPIL

# FIG_6

FIG _ 7